(19) Europäisches Patentamt
European Patent Office
Office européen des brevets

(11) Veröffentlichungsnummer: **0 337 943**
**A2**

(12) # EUROPÄISCHE PATENTANMELDUNG

(21) Anmeldenummer: **89810251.2**

(22) Anmeldetag: **04.04.89**

(51) Int. Cl.[4]: **C 07 D 239/42**
C 07 D 239/47,
C 07 D 405/04,
C 07 D 409/04, C 07 F 9/65,
C 07 D 239/52,
C 07 D 239/56, A 01 N 47/36

(30) Priorität: **12.04.88 CH 1336/88**

(43) Veröffentlichungstag der Anmeldung:
**18.10.89 Patentblatt 89/42**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Rempfler, Hermann, Dr.**
**Brücklismattstrasse 16**
**CH-4107 Ettingen (CH)**

**Dürr, Dieter, Dr.**
**Brändelistalweg 16**
**CH-4103 Bottmingen (CH)**

**Thummel, Rudolf C.**
**Le Borbet**
**CH-2892 Courgenay (CH)**

Patentansprüche für folgenden Vertragsstaat: ES.
Die Bezeichnung der Erfindung wurde geändert (Richtlinien für die Prüfung im EPA, A-III, 7.3).

(54) **N-Phenyl-N-pyrimidin-2-yl-Harnstoffe mit herbiziden und pflanzenwuchsregulierender Wirkung.**

(57) Die vorliegende Erfindung betrifft neue N-Phenyl-N-pyrimidin-2-yl-harnstoffe mit herbizider und pflanzenwuchsregulierender Wirkung, agrochemische Mittel, welche diese Substanzen als Wirkstoffe enthalten, die Verwendung der neuen Harnstoffe zur Bekämpfung von Unkräutern oder zur Regulierung des Pflanzenwuchses sowie Verfahren zur Herstellung der neuen Verbindungen. Ferner betrifft die Erfindung auch neue Zwischenprodukte und Verfahren zu deren Herstellung.

Die neuen Verbindungen entsprechen der Formel I

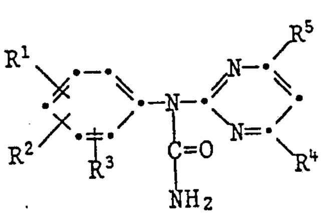

(I),

worin
$R^1$, $R^2$, und $R^3$ unabhängig voneinander Wasserstoff; Halogen; $C_1$-$C_4$-Alkyl; $C_1$-$C_4$-Alkoxy; $C_1$-$C_4$-Halogenalkyl; $C_1$-$C_4$-Halogenalkoxy; $C_1$-$C_4$-Alkyl-$S(O)_n$-; Nitro; Cyano; $C_1$-$C_4$-Alkoxycarbonyl; $C_1$-$C_4$-Alkylcarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-$S(O)_n$-; oder -PO[O-($C_1$-$C_4$)-Alkyl]$_2$;
$R^4$ $C_1$-$C_6$-Alkyl; $C_1$-$C_4$-Alkoxy; $C_1$-$C_4$-Alkyl-$S(O)_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituierten oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;
$R^5$ Wasserstoff; $C_1$-$C_3$-Alkyl; $C_1$-$C_3$-Halogenalkyl; Halogen; oder $C_1$-$C_3$-Halogenalkoxy; und
n 0, 1 oder 2
bedeutet, mit der Massgabe, dass, wenn einer der Reste $R^1$, $R^2$ oder $R^3$ Nitro bedeutet, dieser Substituent nicht in 2- oder 6-Stellung des Phenylringes gebunden sein darf.

EP 0 337 943 A2

**Beschreibung**

**Neue Harnstoffe**

Die vorliegende Erfindung betrifft neue N-Phenyl-N-pyrimidin-2-yl-harnstoffe mit herbizider und pflanzenwuchsregulierender Wirkung, agrochemische Mittel, welche diese Substanzen als Wirkstoffe enthalten, die Verwendung der neuen Harnstoffe zur Bekämpfung von Unkräutern oder zur Regulierung des Pflanzenwuchses sowie Verfahren zur Herstellung der neuen Verbindungen. Ferner betrifft die Erfindung auch neue Zwischenprodukte und Verfahren zu deren Herstellung.

Aus der Patentschrift DD-151 404 und der europäischen Patentanmeldung EP-A-0 172 786 sind (Pyrimidin-2-yl)-2-nitroaniline bekannt geworden. Diese Verbindungen sind fungizid wirksam. Demgegenüber wurde überraschenderweise gefunden, dass N-Phenyl-N-pyrimidin-2-yl-harnstoffe herbizide bzw. pflanzenwachstumsregulatorische Wirkung haben.

Die Erfindung betrifft Harnstoffe der Formel I

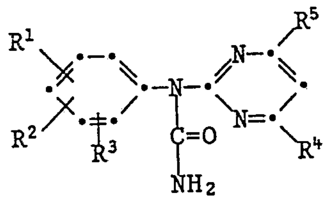

(I),

worin

$R^1$, $R^2$, und $R^3$ unabhängig voneinander Wasserstoff; Halogen; $C_1$-$C_4$-Alkyl; $C_1$-$C_4$-Alkoxy; $C_1$-$C_4$-Halogenalkyl; $C_1$-$C_4$-Halogenalkoxy; $C_1$-$C_4$-Alkyl-$S(O)_n$-; Nitro; Cyano; $C_1$-$C_4$-Alkoxycarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-$S(O)_n$-; $C_1$-$C_4$-Alkylcarbonyl; oder -PO[O-($C_1$-$C_4$)-Alkyl]$_2$;

$R^4$ $C_1$-$C_6$-Alkyl; $C_1$-$C_4$-Alkoxy; $C_1$-$C_4$-Alkyl-$S(O)_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituierten oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;

$R^5$ Wasserstoff; $C_1$-$C_3$-Alkyl; $C_1$-$C_3$-Halogenalkyl; Halogen; oder $C_1$-$C_3$-Halogenalkoxy; und

n 0, 1 oder 2

bedeutet, mit der Massgabe, dass, wenn einer der Reste $R^1$, $R^2$ oder $R^3$ Nitro bedeutet, dieser Substituent nicht in 2- oder 6-Stellung des Phenylringes gebunden sein darf, sowie Salze und Additionsverbindungen der Verbindungen der Formel I mit Säuren, Basen und Komplexbildnern.

Im Rahmen der hier offenbarten Erfindung umfassen die angegebenen generischen Begriffe beispielsweise die folgenden spezifischen Einzelsubstituenten, wobei diese Aufzählung keine Einschränkung der Erfindung darstellt:

Alkyl umfasst die geradkettigen oder verzweigten $C_1$-$C_6$-Alkyle, wie Methyl, Ethyl, n-Propyl, iso-Propyl, n-Butyl, sek-Butyl, tert-Butyl, iso-Butyl, die isomeren Pentyle- wie etwa n-Pentyl, tert-Pentyl (1,1-Dimethylpropyl), iso-Pentyl (1-Ethylpropyl), sowie die isomeren Hexylreste. Bevorzugt sind die $C_1$-$C_5$-Alkylreste.

Halogen ist Fluor, Chlor, Brom und Jod. Bevorzugt ist Fluor, Chlor und Brom.

Mit Halogenalkyl sind die ganz oder teilweise gleich oder unterschiedlich halogensubstituierten Alkyle, gemäss der jeweiligen Definitionsbreite gemeint, wie etwa Trifluormethyl, Difluormethyl, 1,1,2,2-Tetrafluorethyl, 2-Chlorethyl, Pentafluorethyl, Chloridifluormethyl, Dichlormethyl, Chlorfluormethyl, 1,1-Dichlor-2,2,2-trifluorethyl, 1,1-Dichlorethyl oder Heptafluorpropyl.

Als $C_1$-$C_4$-Alkoxycarbonylreste sind unter anderem Methoxycarbonyl, Ethoxy carbonyl sowie die isomeren Propyloxycarbonyle und Butyloxycarbonyle zu nennen.

Alkoxy sind im Rahmen der jeweiligen Definitionsbreite der isomeren Alkyloxyradikale, in erster Linie Methoxy, Ethoxy, (i)-Propyloxy, (n)-Propyloxy, (i)-Butyloxy, (t)-Butyloxy, (n)-Butyloxy und (sec)-Butyloxy.

Halogenalkoxy und Halogenalkylthio sind im Rahmen der jeweiligen Definitionsbreite die isomeren ein- oder mehrfach gleich oder verschieden halogensubstituierten Alkylreste, wie etwa Trifluormethoxy, 2,2,2-Trifluorethoxy, Trifluormethylthio, 2,2,3,3,3-Pentafluorpropyloxy oder 1,1,2,2-Tetrafluorethoxy, Difluormethoxy oder 2-Chlorethoxy.

Als Alkoxyalkylreste sind unter anderem zu nennen: 2-Ethoxyethyl, 2-Methoxyethyl, 3-Methoxypropyl, 2-Methoxy-1-methylethyl und Methoxymethyl.

Die Gruppe $C_1$-$C_4$-Alkyl-$S(O)_n$- steht für die jeweiligen Alkylthio-, Alkylsulfinyl- und Alkylsulfonylradikale. Als besonders bevorzugt sind Methylthio, Methylsulfinyl und Methylsulfonyl zu nennen.

Aus der Gruppe PO[O($C_1$-$C_4$)-Alkyl]$_2$ sind $PO(OCH_3)_2$, und $PO(OC_2H_5)_2$ bevorzugt.

di-($C_1$-$C_4$-Alkylamino)carbonyl umfasst sowohl die mit gleichen oder verschiedenen $C_1$-$C_4$-Alkylresten substituierten Radikale.

Halogenalkylthio ist insbesondere Fluormethylthio, Difluormethylthio, Trifluormethylthio, 1,1,2,2-Tetrafluorethylthio, Chlordifluormethylthio und Dichlorfluormethylthio.

In den weiteren Substituenten, welche aus mehreren Grundelementen zusammengesetzt sind, können die Teilelemente innerhalb der Definitionsbreite frei gewählt werden und obige Bedeutung haben.

Hervorzuheben sind in der 4-Position des Phenylringes unsubstituierte Verbindungen der Formel I

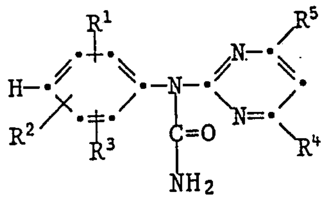

(I),

worin

$R^1$ Halogen; Cyano; $C_1$-$C_4$-Alkoxy; $C_1$-$C_4$-Halogenalkoxy; $C_1$-$C_4$-Alkyl-$S(O)_n$-; $C_1$-$C_4$-Alkyl; $C_1$-$C_4$-Halogenalkyl; $C_1$-$C_4$-Alkoxycarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-$S(O)_n$-; oder -PO[O-($C_1$-$C_4$)-Alkyl]$_2$;

$R^2$ Wasserstoff; Halogen; Cyano; Nitro; $C_1$-$C_4$-Alkyl; $C_1$-$C_4$-Alkoxy; $C_1$-$C_4$-Halogenalkyl; $C_1$-$C_4$-Alkoxycarbonyl; oder $C_1$-$C_4$-Alkylcarbonyl;

$R^3$ Wasserstoff; Halogen; oder $C_1$-$C_4$-Alkyl bedeutet und n und die Reste $R^4$ und $R^5$ wie zuvor definiert sind.

Bevorzugte Untergruppen bilden diejenigen Verbindungen der Formel I, in denen

a) der Rest $R^1$ an die 2-Stellung des Phenylringes gebunden ist,
b) der Rest $R^2$ an die 3-Stellung des Phenylringes gebunden ist,
c) der Rest $R^3$ an die 5-Stellung des Phenylringes gebunden ist,
d) der Rest $R^2$ an die 6-Stellung des Phenylringes gebunden ist,
e) der Rest $R^1$ an die 3-Stellung des Phenylringes gebunden ist,
f) der Rest $R^3$ an die 3-Stellung des Phenylringes gebunden ist,
g) der Rest $R^2$ an die 5-Stellung des Phenylringes gebunden ist.

Hervorzuheben sind folgende Kombinationen von Untergruppen: a+b, a+g, a+d, e+g, e+d, a+b+c, a+f+d, a+c+d und e+c+d, wobei bei der Kombination von nur zwei Untergruppen der Rest $R^3$ für Wasserstoff steht.

Besonders hervorzuheben sind Verbindungen der Formel I, worin

$R^1$ Halogen; Cyano; $C_1$-$C_3$-Alkoxy; $C_1$-$C_2$-Halogenalkoxy; Methyl-$S(O)_n$-; $C_1$-$C_3$-Alkyl; $C_1$-$C_2$-Halogenalkyl; $C_1$-$C_4$-Alkoxycarbonyl; Carbamoyl; Difluormethylthio oder -PO[O-($C_1$-$C_2$)-Alkyl]$_2$;

$R^2$ Wasserstoff; Fluor; Chlor; Brom; Cyano; Nitro; $C_1$-$C_3$-Alkyl; $C_1$-$C_2$-Halogenalkyl; oder $C_1$-$C_3$-Alkoxycarbonyl;

$R^3$ Wasserstoff; Chlor; Fluor; oder $C_1$-$C_3$-Alkyl;

$R^4$ $C_1$-$C_5$-Alkyl; $C_1$-$C_4$-Alkoxy; $C_1$-$C_4$-Alkylthio; Cyclopropyl; Phenyl; Furan-2-yl; Thiophen-2-yl; Cyano; $C_1$-$C_3$-Halogenalkoxy; $C_1$-$C_2$-Alkoxy-$C_1$-$C_2$-alkyl; $C_1$-$C_2$-Alkoxy-$C_1$-$C_2$-alkoxy; Methylsulfinyl; Methylsulfonyl; oder $C_2$-$C_3$-Halogenalkenyl;

$R^5$ $C_1$-$C_3$-Alkyl; Fluor; Chlor; Brom; $C_1$-$C_3$-Halogenalkyl; oder $C_1$-$C_3$-Halogenalkoxy; und

n 0, 1 oder 2

bedeutet.

Besonders bevorzugt sind Verbindungen der Formel I, worin

$R^1$ Halogen; Methyl; Trifluormethyl; Trifluormethoxy; Difluormethoxy; $C_1$-$C_3$-Alkoxy; Methylthio; Methylsulfinyl; Methylsulfonyl; Cyano; $C_1$-$C_4$-Alkoxycarbonyl; Carbamoyl; Difluormethylthio; oder -PO(O-$C_2H_5$)$_2$;

$R^2$ Wasserstoff; Fluor; Chlor; Brom; Nitro; Ethyl; Methyl; Trifluormethyl; Methoxy; oder $C_1$-$C_3$-Alkoxycarbonyl;

$R^3$ Wasserstoff; Chlor; oder Methyl;

$R^4$ $C_1$-$C_5$-Alkyl; $C_1$-$C_4$-Alkoxy; $C_1$-$C_4$-Alkylthio; Cyclopropyl; Phenyl; Furan-2-yl; Thiophen-2-yl; Cyano; 1,1,2,2-Tetrafluorethoxy; 2-Chlorethoxy; Methoxymethyl; 2-Methoxy-ethoxy; Methylsulfinyl; Methylsulfonyl; oder 2,2-Dichlorvinyl;

$R^5$ $C_1$-$C_3$-Alkyl; Fluor; Chlor; Brom; Difluormethoxy; Trifluormethyl; Pentafluorethyl; Chlordifluormethyl; Difluormethyl; Dichlormethyl; Chlorfluormethyl; 1,1-Dichlor-2,2,2-trifluorethyl; 1,1-Dichlorethyl; oder Heptafluorpropyl;

bedeutet.

Insbesondere betrifft die Erfindung Verbindungen der Formel I, worin

$R^1$ Fluor; Chlor; Brom; Jod; Trifluormethyl; Methyl; Difluormethoxy; Methoxy; Methylthio; PO($OC_2H_5$)$_2$; Methoxycarbonyl; Methylsulfonyl; oder Cyano;

$R^2$ Wasserstoff; Fluor; Chlor; Brom; Nitro; Methyl; Ethyl; Trifluormethyl; oder Methoxycarbonyl;

$R^3$ Wasserstoff; oder Methyl;

$R^4$ $C_1$-$C_5$-Alkyl; Butylthio; Thiophen-2-yl; Furan-2-yl; $C_1$-$C_4$-Alkoxy; Methylthio; Methylsulfinyl; Methylsulfonyl; oder Cyclopropyl; und

$R^5$ Methyl; Chlor; Dichlormethyl; Pentafluorethyl; Difluormethyl; Chlorfluormethyl; Trifluormethyl; oder Chlordifluormethyl

bedeutet.

Hervorzuheben sind ausserdem die Verbindungen der Formel I, worin

a) $R^1$ in Position 2 des Phenylringes gebundenes Fluor, Chlor, Brom, Trifluormethyl, Cyano, Difluormethyl, Trifluormethoxy, Methyl oder Methoxy,
$R^2$ und $R^3$ Wasserstoff,
$R^4$ Methyl, Furan-2-yl oder Cyclopropyl, und
$R^5$ Chlor, Methyl, Trifluormethyl oder Chlordifluormethyl, oder

b) $R^1$ in Position 2 des Phenylringes gebundenes Fluor, Chlor, Brom, Trifluormethyl, Difluormethoxy, Trifluormethoxy, Cyano oder Methoxy,
$R^2$ in Position 3, 5 oder 6 des Phenylringes gebundenes Fluor oder Chlor,
$R^4$ Methyl, Furan-2-yl oder Cyclopropyl, und
$R^5$ Chlor, Methyl, Trifluormethyl oder Chlordifluormethyl, oder

c) $R^1$ in Position 3 des Phenylringes gebundenes Chlor,
$R^2$ in Position 5 des Phenylringes gebundenes Chlor,
$R^4$ Methyl, Furan-2-yl oder Cyclopropyl, und
$R^5$ Chlor, Methyl Trifluormethyl oder Chlordifluormethyl bedeutet.

Aufgrund ihrer herbiziden Wirkung namentlich zu nennen sind:
N-(2-Bromphenyl)-N-(4-chlor-6-methyl-pyrimidin-2-yl)-harnstoff,
N-(4-Methyl-6-trifluormethyl-pyrimidin-2-yl)-N-(2-trifluormethyl-phenyl)-harnstoff,
N-(2,3-Dichlorphenyl)-N-(4-methyl-6-trifluormethyl-pyrimidin-2-yl)-harnstoff,
N-(2,5-Dichlorphenyl)-N-(4,6-dimethyl-pyrimidin-2-yl)-harnstoff,
N-(4-Chlor-6-methyl-pyrimidin-2-yl)-N-(2,5-dichlorphenyl)-harnstoff,
N-(2,5-Dichlorphenyl)-N-(4-methyl-6-trifluormethyl-pyrimidin-2-yl)-harnstoff,
N-[4-(Chlordifluormethyl)-6-methyl-pyrimidin-2-yl]-N-(2,5-dichlorphenyl)-harnstoff,
N-(4-Chlor-6-methyl-pyrimidin-2-yl)-N-(2,6-dichlorphenyl)-harnstoff,
N-(2,6-Dichlorphenyl)-N-(4-methyl-6-trifluormethyl-pyrimidin-2-yl)-harnstoff,
N-(4-Cyclopropyl-6-trifluormethyl-pyrimidin-2-yl)-N-(2,6-dichlorphenyl)-harnstoff,
N-(3,5-Dichlorphenyl)-N-(4-methyl-6-trifluormethyl-pyrimidin-2-yl)-harnstoff,
N-(5-Chlor-2-methyl-phenyl)-N-(4-chlor-6-methyl-pyrimidin-2-yl)-harnstoff,
N-(5-Chlor-2-methoxyphenyl)-N-(4-methyl-6-trifluormethyl-pyrimidin-2-yl)-harnstoff,
N-(2-Jodphenyl)-N-(4-methyl-6-trifluormethyl-pyrimidin-2-yl)-harnstoff,
N-(2-Chlor-6-methylphenyl)-N-(4-chlor-6-methylpyrimidin-2-yl)-harnstoff,
N-(3-Chlor-2-methoxyphenyl)-N-(4-methyl-6-trifluormethylpyrimidin-2-yl)-harnstoff,
N-(2,6-Difluorphenyl)-N-(4-methyl-6-trifluormethylpyrimidin-2-yl)-harnstoff,
N-(2,5-Difluorphenyl)-N-(4-methyl-6-trifluormethyl-pyrimidin-2-yl)-harnstoff,
N-(2-Cyanophenyl)-N-(4-methyl-6-trifluormethyl-pyrimidin-2-yl)-harnstoff,
N-(2-Bromphenyl)-N-(4-cyclopropyl-6-trifluormethyl-pyrimidin-2-yl)-harnstoff,
N-(4-Isopropyl-6-trifluormethyl-pyrimidin-2-yl)-N-2-(trifluormethylphenyl)-harnstoff,
N-(2-Fluorphenyl)-N-(4-methyl-6-trifluormethyl-pyrimidin-2-yl)-harnstoff,
N-(2-Fluorphenyl)-N-(4-isopropyl-6-trifluormethyl-pyrimidin-2-yl)-harnstoff,
N-(2,5-Dichlorphenyl)-N-(4-ethyl-6-trifluormethyl-pyrimidin-2-yl)-harnstoff,
N-(2,6-Dichlorphenyl)-N-(4-difluormethyl-6-methyl-pyrimidin-2-yl)-harnstoff,
N-(2,6-Dichlorphenyl)-N-(4-methylthio-6-trifluormethyl-pyrimidin-2-yl)-harnstoff,
N-(2,6-Dichlor-3-methyl)-N-(4-methyl-6-trifluor-methyl-pyrimidin-2-yl)-harnstoff,
N-(2,6-Dichlor-3-methylphenyl)-N-(4-isopropyl-6-trifluormethyl-pyrimidin-2-yl)-harnstoff,
N-(2-Chlor-6-trifluormethyl-phenyl)-N-(4-methyl-6-trifluormethyl-pyrimidin-2-yl)-harnstoff,
N-(2-Chlor-6-methyl-phenyl)-N-(4-methyl-6-trifluormethyl-pyrimidin-2-yl)-harnstoff,
N-(2-Methyl-6-trifluormethyl-phenyl)-N-(4-methyl-6-trifluormethyl-pyrimidin-2-yl)-harnstoff,
N-(5-Chlor-2-difluormethoxy-phenyl)-N-(4-methyl-6-trifluormethyl-pyrimidin-2-yl)-harnstoff,
N-(2-Methylthio-phenyl)-N-(4-methyl-6-trifluormethyl-pyrimidin-2-yl)-harnstoff,
N-(4-Cyclopropyl-6-trifluormethyl-pyrimidin-2-yl)-N-(2,5-difluorphenyl)-harnstoff,
N-(2,6-Difluorphenyl)-N-(4-ethyl-6-trifluormethyl-pyrimidin-2-yl)-harnstoff,
N-(6-Chlor-2-fluor-phenyl)-N-(4-methyl-6-trifluormethyl-pyrimidin-2-yl)-harnstoff,
N-(6-Chlor-2-fluor-phenyl)-N-(4-cyclopropyl-6-trifluormethyl-pyrimidin-2-yl)-harnstoff,
N-(2-Difluormethoxyphenyl)-N-(4-methyl-6-trifluormethyl-pyrimidin-2-yl)-harnstoff,
N-(6-Chlor-2-methoxycarbonyl-phenyl)-N-(4-methyl-6-trifluormethyl-pyrimidin-2-yl)-harnstoff,
N-(2-Bromphenyl)-N-[4-(furan-2-yl)-6-trifluormethyl-pyrimidin-2-yl]-harnstoff,
N-(2-Chlor-6-methyl-phenyl)-N-(4-methoxymethyl-6-trifluormethyl-pyrimidin-2-yl)-harnstoff,
und
N-(2-Difluormethoxy-6-methyl-phenyl)-N-(4-methyl-6-trifluormethyl-pyrimidin-2-yl)-harnstoff.

Die Verbindungen der Formel I können hergestellt werden, dadurch dass man

a) ein Anilin der Formel II mit Phosgen zu einem Carbaminchlorid der Formel III umsetzt und dieses in einer zweiten Stufe mit $NH_3$ zu

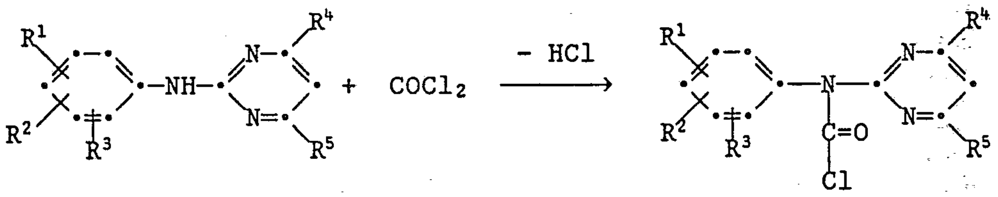


(II) (III)

$$III + NH_3 \xrightarrow{-HCl} I$$

einem Harnstoff der Formel I reagieren lässt oder

b) ein Anilin der Formel II mit Halogensulfonylisocyanat IX zu einem Halogensulfonylharnstoff der Formel IV umsetzt

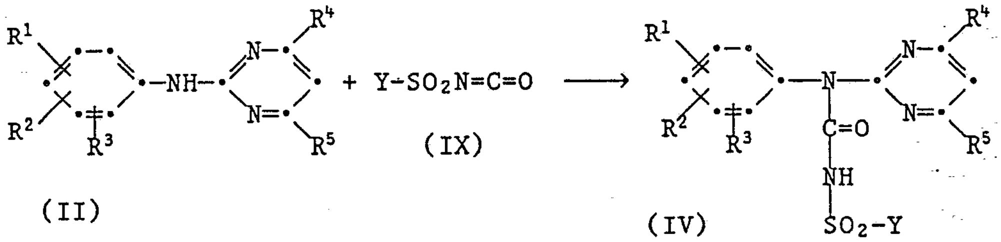


(II) (IV)

$$IV + 2H_2O \xrightarrow[-SO_4H_2]{-HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor, steht.

Die unter Halogenwasserstoffabspaltung bzw. HY-Eliminierung verlaufenden Umsetzungen II → III, III → I und IV → I werden vorzugsweise unter Verwendung säurebindender Mittel (Basen) durchgeführt.

Als solche kommen organische oder anorganische Basen in Betracht, z.B. tertiäre Amine wie Trialkylamine (Trimethylamin, Triethylamin, Tripropylamin usw.), Pyridine (Pyridin, 4-Dimethylaminopyridin, 4-Pyrrolidylaminopyridin usw.), Alkoholate wie z.B. Kalium-tert.-butylat, Natriummethylat oder -ethylat. Die zuvor genannten Reaktionen wie auch die - an späterer Stelle beschriebene - Umsetzung VII → II, können auch unter Phasentransferbedingungen mit Basen nach an sich bekannten Verfahren durchgeführt werden (Lit. Dehmlow & Dehmlow, Phase Transfer Catalysis Verlag Chemie, Weinheim, 1983).

Bei den Verfahrensvarianten a) und b) können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoffe, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Die Aniline der Formel II sind ebenso wie die Carbamoylchloride der Formel III und die Harnstoffe der Formel IV wertvolle Zwischenverbindungen. Die Carbamoylchloride III und die Harnstoffe IV sind neu, während aus DD-B-151 404 einige der Verbindungen der Formel II bekannt sind.

Die Erfindung betrifft somit auch die neuen Verbindungen der Formel II

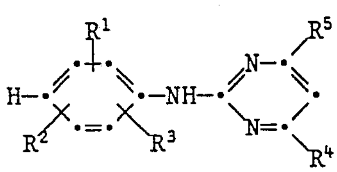

II

worin

$R^1$ Halogen; Cyano; $C_1$-$C_4$-Alkoxy; $C_1$-$C_4$-Halogenalkoxy; $C_1$-$C_4$-Alkyl-S(O)$_n$-; $C_1$-$C_4$-Alkyl; $C_1$-$C_4$-Halogenalkyl; $C_1$-$C_4$-Alkoxycarbonyl; di-($C_1$-$C_4$-Alkylamino)carbonyl; mono-($C_1$-$C_4$-Alkylamino)carbonyl; Carbamoyl;

$C_1$-$C_4$-Halogenalkyl-S(O)$_n$-; oder -PO[O-($C_1$-$C_4$)-Alkyl]$_2$;
$R^2$ Wasserstoff; Halogen; Cyano; Nitro; $C_1$-$C_4$-Alkyl; $C_1$-$C_4$-Alkoxy; $C_1$-$C_4$-Halogenalkyl; $C_1$-$C_4$-Alkoxycarbonyl; oder $C_1$-$C_4$-Alkylcarbonyl;
$R^3$ Wasserstoff; Halogen; oder $C_1$-$C_4$-Alkyl;
$R^4$ $C_1$-$C_6$-Alkyl; $C_1$-$C_4$-Alkoxy; $C_1$-$C_4$-Alkyl-S(O)$_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituiertes oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;
$R^5$ Wasserstoff; $C_1$-$C_3$-Alkyl; $C_1$-$C_3$-Halogenalkyl; Halogen; oder $C_1$-$C_3$-Halogenalkoxy; und
n 0, 1 oder 2
bedeutet, mit der Massgabe, dass a) wenn einer der Reste $R^2$ oder $R^3$ Nitro bedeutet, dieser Substituent nicht in 2- oder 6-Stellung des Phenylringes gebunden sein darf und, wenn die Reste $R^4$ und $R^5$ für Methyl stehen $R^1$ nicht für Chlor, Methoxy, Ethoxy, Fluor, Jod, Methyl oder Brom steht und dass ausserdem folgende Einzelverbindungen nicht mit umfasst sind:
N-[(4,6-Bis-trifluormethyl)-pyrimidin-2-yl]-2,6-dichloranilin und N-(4-Chlor-6-methylpyrimidin-2-yl)-3-chloranilin.

Ausserdem betrifft die Erfindung neue N,N-disubstituierte Carbamoylchloride der Formel III

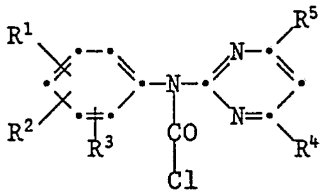

(III),

worin die Reste $R^1$ bis $R^5$ wie in Formel I definiert sind und neue Harnstoffe der Formel IV

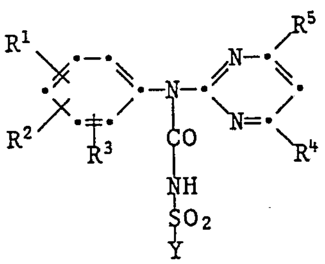

(IV),

worin $R^1$ bis $R^5$ wie unter Formel I definiert sind und Y Halogen, $C_1$-$C_4$-Alkoxy oder Phenoxy bedeutet.

Die Verbindungen der Formel III und IV sind Zwischenprodukte der Verfahren a) und b) und können wie dort beschrieben aus den entsprechenden Anilinen der Formel II hergestellt werden.

Die neuen Aniline der Formel II sind analog literaturbekannter Verfahren herstellbar z.B. durch:

aa) Umsetzung von Guanidinen der Formel V mit 1,3-Dicarbonylverbindungen der Formel VI

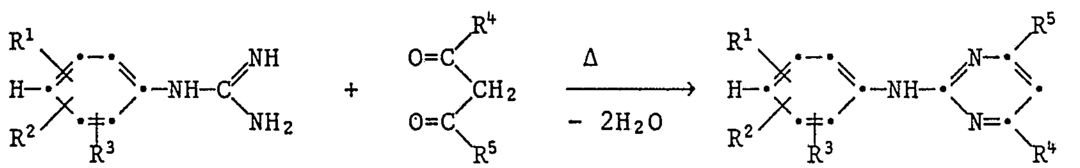


(V) (VI) (II)

wobei die Kondensationsreaktion gewünschtenfalls in Gegenwart wasserbindender Mittel durchgeführt wird (Lit: D.J. Brown in "The Chemistry of Heterocyclic Compounds" Bd. VI 1962, Interscience Publ. New York; J. Am. Chem. Soc. 69 1819 (1947); J. Am. Chem. Soc. 72 2948 (1950); J. Org. Chem. 29 1439 (1964) oder J. Org. Chem. 29 1883 (1964), Houben-Weyl "Methoden d. org. Chemie Bd. VIII S. 180ff) oder

bb) durch Umsetzung eines Anilins der Formel VII mit einem Pyrimidin der Formel VIII unter Baseneinwirkung

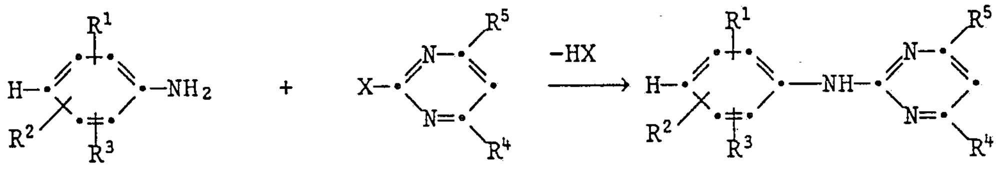

VII VIII II

worin die Reste $R^1$ bis $R^5$ zuvor definiert sind und X für eine nucleofuge Gruppe, wie Halogen, $C_1$-$C_4$-Alkylsulfonyl oder Phenylsulfonyl, steht.

Geeignete Basen zur Durchführung dieses Verfahrens sind u.a. Kalium-, tert.-Butylat, $Na_2CO_3$; $K_2CO_3$ oder NaH.

Diese Verfahren stehen generell zur Synthese von Verbindungen der Formel II zur Verfügung und sind auf alle Edukte der Formel V, VI, VII und VIII (in denen die Reste $R^1$ bis $R^5$ im Formelumfang von Verbindungen I definiert ist) anwendbar.

Des weiteren betrifft die Erfindung herbizide und pflanzenwuchsregulatorische Mittel enthaltend eine Verbindung der Formel I zusammen mit geeigneten Hilfs- und/oder Trägerstoffen.

Die Wirkstoffe der Formel I werden in der Regel mit Aufwandmengen von 0,005 bis 5 kg/ha insbesondere 0,1 bis 3 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Sonnenblumen, Raps, Mais und Reis befähigen.

Die Verbindungen der Formel I haben ausserdem pflanzenwuchsregulatorische Eigenschaften. Es werden sowohl Monokotyledonen als auch Dikotyledonen in ihrem Wachstum beeinflusst. Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann. Ein weiterer Mechanismus der Ertragssteigerung mit Wachstumsregulatoren beruht darauf, dass die Nährstoffe in stärkerem Masse der Blüten- und Fruchtbildung zugute kommen, während das vegetative Wachstum eingeschränkt wird. Bei grösseren Aufwandmengen werden Unkräuter und Gräser in ihrer Entwicklung so geschädigt, dass sie absterben.

In besonders vorteilhafter Weise können die wuchsregulatorischen Verbindungen der Formel I zur Wuchsregulation von Untersaaten in Maiskulturen verwendet werden. Als Untersaat in Maiskulturen sind prinzipiell diejenigen Pflanzen geeignet, die den Boden zwischen den einzelnen Maispflanzen bedecken und so in erster Linie der Bodenerosion in Maiskulturen entgegenwirken. Geeignete Pflanzen für die Untersaat sind unter anderem Raps, Klee, Gräser oder Leguminosen.

In geeigneten Aufwandmengen hemmen die Verbindungen der Formel I den Neuzuwachs von Gräsern. Dies erlaubt es in Rasenkulturen (Parks, Gärten, etc.) die Zahl der erforderlichen Schnitte zu vermindern, beziehungsweise die Zeiträume zwischen den einzelnen Schnitten zu verlängern. In besonders vorteilhafter Weise können hierzu Granulatformulierungen der Wirkstoffe der Formel I verwendet werden. Das Granulat kann entweder den Wirkstoff allein, neben den üblichen Hilfs- und Trägerstoffen enthalten, oder der Wirkstoff wird zusammen mit einem Mineraldünger, und/oder gegebenenfalls weiteren Wirkstoffen zur Bekämpfung von Moos oder anderen in Rasenkulturen unerwünschten Pflanzenwuchses, als Granulat formuliert. Die Anwendung als Streugranulat erlaubt es, mit Hilfe der bei Rasenkulturen üblichen Bearbeitungsgeräte, während längerer Zeit den Neuzuwachs der Gräser zu hemmen. Das Granulat kann dabei in an sich bekannter Weise hergestellt werden, es weist vorzugsweise eine Korngrösse von 0,1 bis 2,0 mm, insbesondere von 0,25 bis 1,0 mm auf.

Die Erfindung betrifft auch herbizide und pflanzenwachstumsregulierende Mittel, welche einen Wirkstoff der Formel I enthalten, sowie Verfahren zur pre- und post-emergenten Unkrautbekämpfung und zur Beinflussung des Pflanzenwuchses von monokotylen und dikotylen Pflanzen, insbesondere Gräsern, tropischen Bodenbedeckern und Geiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt. So können die Aktivsubstanzen der Formel I auch auf mineralische Dünger aufgebracht (aufgebeizt) werden. Das so erhältliche Mittel ist in vorteilhafter Weise als Wuchsregulator bei Gräsern geeignet.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylreste mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seinen Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich von allem um quaternäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedrige Hydroxyalkylreste aufweisen. Die Salze liegen vorzugsweise als Halogenide, Methylsulfate oder Ethylsulfate, vor, z.B. das Stearyltrimethylammoniumchlorid oder das Benzyldi(2-chlorethyl)-ethylammoniumbromid.

Klebstoffe sind insbesondere diejenigen Hilfsstoffe, welche beim Granulieren den Zusammenhalt des Trägermaterials, der Hilfsstoffe und der Wirkstoffe bewirken, wie Gummi arabicum oder Carboxymethylcellulose.

Die in der Formulierungstechnik gebräuchlichen Tenside sind u.a. in folgenden Publikationen beschrieben:
"1987 International Mc Cutcheon's Emulsifiers and Detergents",
Glen Rock, N.J., USA.
Dr. Helmut Stache "Tensid Taschenbuch"
Carl Hanser Verlag, München/Wien 1981.

Die Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

Stäube:

| | |
|---|---|
| Wirkstoff der Formel I: | 0,1 bis 10 %, vorzugsweise 0,1 bis 1 % |
| festes Trägermittel: | 99,9 bis 90 %, vorzugsweise 99,9 bis 99 %. |

Suspensions-Konzentrate:

| | |
|---|---|
| Wirkstoff der Formel I: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

Benetzbare Pulver:

| | |
|---|---|
| Wirkstoff der Formel I: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

Granulate:

| | |
|---|---|
| Wirkstoff der Formel I: | 0,5 bis 30 %, vorzugsweise 3 bis 15 % |
| festes Trägermittel: | 99,5 bis 70 %, vorzugsweise 97 bis 85 %. |

Streugranulat:

| | |
|---|---|
| Wirkstoff der Formel I: | 0,01 bis 30 %, vorzugsweise 0,05 bis 15 % |
| Klebstoff: | 0,05 bis 5 %, vorzugsweise 0,1 bis 2 % |
| oberflächenaktives Mittel: | 0,5 bis 20 %, vorzugsweise 1 bis 15 % |
| festes Trägermaterial: | 99,44 bis 45 %, vorzugsweise 95 bis 65 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

Die - unkorrigierten - Schmelzpunkte in den nachfolgenden Herstellungsbeispielen sind in °C angegeben.

H. 1. Verbindungen der Formel I

H. 1.1. N-(4-Methyl-6-trifluormethyl-pyrimidin-2-yl)-N-(2-trifluormethyl-phenyl)-harnstoff

Zu einer Lösung von 6,4 g (0.02 Mol) N-(4-Methyl-6-trifluormethyl-pyrimidin-2-yl)-2-trifluormethylanilin in 100 ml Essigsäureäthylester werden bei 3°C 3,6 g (0.026 Mol) Chlorsulfonylisocyanat gegeben. Nach 2-stündigem Rühren bei 3°C werden 100 ml Essigsäureäthylester und 50 ml Eiswasser zugegeben. Die Wasserphase wird abgetrennt, die organische Phase wird mit Kochsalzlösung gewaschen, mit Natriumsulfat getrocknet und am Rotationsverdampfer eingedampft. Die verbleibenden Kristalle werden mit Hexan verrieben, abfiltriert und im Vacuum bei 40°C getrocknet.

Man isoliert 6,1 g (83,8 %) der Titelverbindung der Formel

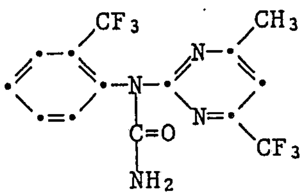

als Kristalle vom Smp. 150-153°C (Verb. No. 1.020).

H. 1.2. N-(2,5-Dichlorphenyl)-N-(4,6-dimethylpyrimidin-2-yl)-harnstoff

4,0 g (0.012 Mol) N-Chlorcarbonyl-N-(4,6-dimethylpyrimidin-2-yl)-2,5-dichloranilin werden in 50 ml Chloroform gelöst. Dazu wird ein Ueberschuss Ammoniak-Gas eingeleitet. Nach Abklingen der exothermen Reaktion wird das Reaktionsgemisch zwei Mal mit Wasser gewaschen, mit Magnesiumsulfat getrocknet und eingedampft.

Nach dem Umkristallisieren aus Ethanol erhält man 2,4 g (64,9 %) der Titelverbindung der Formel

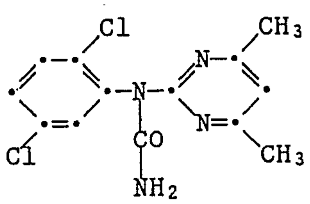

als Kristalle vom Smp. 153°C (Zers.) (Verb. No. 1.050).

Analog zu den Herstellungsbeispielen H.1 sind die Verbindungen der Tabelle 1 erhältlich:

Tabelle I

Verbindungen der Formel

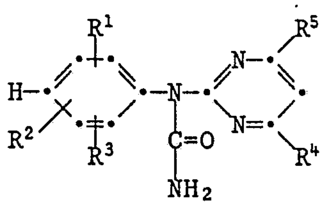

I

| Verb.Nr. | $R^1$ | $R^2$ | $R^3$ | $R^4$ | $R^5$ | physikalische Daten |
|---|---|---|---|---|---|---|
| 1.001 | 2-Cl | H | H | $CH_3$ | $CH_3$ | ab 147°C Zers. |
| 1.002 | 2-Cl | H | H | $CH_3$ | $OCHF_2$ | |
| 1.003 | 2-Cl | H | H | $CH_3$ | Cl | |
| 1.004 | 2-Cl | H | H | $CH_3$ | $CF_3$ | |
| 1.005 | 2-Cl | H | H | $CH_3$ | $CF_2CF_3$ | |
| 1.006 | 2-Cl | H | H | $CH_2CH_3$ | $CF_3$ | |
| 1.007 | 2-Cl | H | H | $CH_3$ | $CF_2Cl$ | |
| 1.008 | 2-Cl | H | H | $CH_3$ | $CHF_2$ | |
| 1.009 | 2-Br | H | H | $CH_3$ | Cl | Fp. 149-150°C |
| 1.010 | 2-Br | H | H | $OCH_3$ | $CF_3$ | |
| 1.011 | 2-Br | H | H | $CH_3$ | $CF_3$ | |
| 1.012 | 2-Br | H | H | $SCH_3$ | $CF_3$ | |
| 1.013 | 2-Br | H | H | $CH_2CH_3$ | $CF_3$ | |
| 1.014 | 2-Br | H | H | $CH_3$ | $CHCl_2$ | |
| 1.015 | 2-Br | H | H | $CH_3$ | $CHFCl$ | |
| 1.016 | 2-Br | H | H | Cyclo-propyl | $CF_3$ | Fp. 183-184°C |
| 1.017 | 2-$CF_3$ | H | H | Phenyl | $CF_3$ | |
| 1.018 | 2-$CF_3$ | H | H | 2-Furyl | $CF_3$ | |
| 1.019 | 2-$CF_3$ | H | H | $CH_3$ | Cl | |
| 1.020 | 2-$CF_3$ | H | H | $CH_3$ | $CF_3$ | Fp. 150-153°C |
| 1.021 | 2-$CF_3$ | H | H | $CH_3$ | $CH_3$ | Fp. 145-146°C. |
| 1.022 | 2-$CF_3$ | H | H | $CH_2CH_3$ | $CF_3$ | |
| 1.023 | 2-$CF_3$ | H | H | $CH(CH_3)_2$ | $CF_3$ | Fp. 137-138°C |
| 1.024 | 2-$CF_3$ | H | H | $(n)C_3H_7$ | $CF_3$ | Fp. 153-154°C |
| 1.025 | 2-$CF_3$ | H | H | $OCH_3$ | $CF_3$ | |
| 1.026 | 2-$CF_3$ | H | H | $OC_2H_5$ | $CF_3$ | |
| 1.027 | 2-$CF_3$ | H | H | $SCH_3$ | $CF_3$ | |
| 1.028 | 2-$CF_3$ | H | H | $SCH(CH_3)_2$ | $CF_3$ | |
| 1.029 | 2-$CF_3$ | H | H | $CH_3$ | $CF_2CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.030 | 2-$CF_3$ | H | H | $CH_3$ | CHFCl | |
| 1.031 | 2-J | H | H | $CH_3$ | Cl | |
| 1.032 | 2-J | H | H | $CH_3$ | $CF_3$ | Fp. 168-160°C |
| 1.033 | 2-J | H | H | $CH_2CH_3$ | $CF_3$ | |
| 1.034 | 2-J | H | H | $CH_3$ | $CF_2Cl$ | |
| 1.035 | 2-F | H | H | $CH_3$ | $CH_3$ | ab 159°C Zers. |
| 1.036 | 2-F | H | H | $CH_3$ | Cl | |
| 1.037 | 2-F | H | H | $CH_3$ | $CF_3$ | Fp. 169°C Zers. |
| 1.038 | 2-F | H | H | $CH(CH_3)_2$ | $CF_3$ | Fp. 144-146°C |
| 1.039 | 2-F | H | H | Cl | $CF_3$ | |
| 1.040 | 2-F | H | H | $OC_2H_5$ | $CF_3$ | |
| 1.041 | 2-Cl | 3-Cl | H | $CH_3$ | Cl | Fp. 143-144°C |
| 1.042 | 2-Cl | 3-Cl | H | $CH_3$ | $CF_3$ | Fp. 139-140°C |
| 1.043 | 2-Cl | 3-Cl | H | $OCH_3$ | $CF_3$ | |
| 1.044 | 2-Cl | 3-Cl | H | $OC_4H_9(n)$ | $CF_3$ | |
| 1.045 | 2-Cl | 3-Cl | H | $SC_2H_5$ | $CF_3$ | |
| 1.046 | 2-Cl | 3-Cl | H | Phenyl | $CF_3$ | |
| 1.047 | 2-Cl | 3-Cl | H | $CH_3$ | $CF_2Cl$ | |
| 1.048 | 2-Cl | 3-Cl | H | $CH_3$ | CHFCl | |
| 1.049 | 2-Cl | 3-Cl | H | $CH_3$ | $CH_3$ | ab 163°C Zers. |
| 1.050 | 2-Cl | 5-Cl | H | $CH_3$ | $CH_3$ | ab 153°C Zers. |
| 1.051 | 2-Cl | 5-Cl | H | $CH_3$ | $OCHF_2$ | |
| 1.052 | 2-Cl | 5-Cl | H | $CH_3$ | Cl | Fp. 123-125°C |
| 1.053 | 2-Cl | 5-Cl | H | $OCH_3$ | $CH_3$ | |
| 1.054 | 2-Cl | 5-Cl | H | $CH_3$ | Br | |
| 1.055 | 2-Cl | 5-Cl | H | $CH_3$ | $CF_3$ | Fp. 150-151°C |
| 1.056 | 2-Cl | 5-Cl | H | $CH_2CH_3$ | $CF_3$ | Fp. 149-150°C |
| 1.057 | 2-Cl | 5-Cl | H | $C_3H_7(n)$ | $CF_3$ | Fp. 148-149°C |
| 1.058 | 2-Cl | 5-Cl | H | $C_3H_7(i)$ | $CF_3$ | Fp. 156-157°C |
| 1.059 | 2-Cl | 5-Cl | H | Cyclo-propyl | $CF_3$ | Fp. 174-175°C |
| 1.060 | 2-Cl | 5-Cl | H | Phenyl | $CF_3$ | |
| 1.061 | 2-Cl | 5-Cl | H | 2-Thienyl | $CF_3$ | Fp. 177-178°C |
| 1.062 | 2-Cl | 5-Cl | H | 2-Furyl | $CF_3$ | Fp. 168-169°C |
| 1.063 | 2-Cl | 5-Cl | H | $CH_3$ | $CF_2CF_3$ | Fp. 157-158°C |
| 1.064 | 2-Cl | 5-Cl | H | $CH_3$ | $CF_2Cl$ | Fp. 178-179°C |
| 1.065 | 2-Cl | 5-Cl | H | $CH_3$ | $CHF_2$ | Fp. 159-160°C |
| 1.066 | 2-Cl | 5-Cl | H | $CH_3$ | CHClF | Fp. 153-154°C |
| 1.067 | 2-Cl | 5-Cl | H | $CH_2CH_3$ | $CF_2Cl$ | |
| 1.068 | 2-Cl | 5-Cl | H | $C_3H_7(i)$ | $CF_2Cl$ | Fp. 161-162°C |
| 1.069 | 2-Cl | 5-Cl | H | $OCH_3$ | $CF_3$ | |
| 1.070 | 2-Cl | 5-Cl | H | $OC_2H_5$ | $CF_3$ | |
| 1.071 | 2-Cl | 5-Cl | H | $OC_3H_7$ | $CF_3$ | |
| 1.072 | 2-Cl | 5-Cl | H | $OC_3H_7(i)$ | $CF_3$ | |
| 1.073 | 2-Cl | 5-Cl | H | $OC_4H_9(n)$ | $CF_3$ | |
| 1.074 | 2-Cl | 5-Cl | H | $OC_4H_9(i)$ | $CF_3$ | |
| 1.075 | 2-Cl | 5-Cl | H | $SCH_3$ | $CF_3$ | |
| 1.076 | 2-Cl | 5-Cl | H | $SC_2H_5$ | $CF_3$ | |
| 1.077 | 2-Cl | 5-Cl | H | $SC_3H_7(n)$ | $CF_3$ | |
| 1.078 | 2-Cl | 5-Cl | H | $SC_3H_7(i)$ | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.079 | 2-Cl | 5-Cl | H | $SC_4H_9(n)$ | $CF_3$ | |
| 1.080 | 2-Cl | 5-Cl | H | $C_4H_9(n)$ | $CF_3$ | Fp. 123-124°C |
| 1.081 | 2-Cl | 5-Cl | H | $C_4H_9(i)$ | $CF_3$ | |
| 1.082 | 2-Cl | 5-Cl | H | $C_4H_9(t)$ | $CF_3$ | Fp. 159-160°C |
| 1.083 | 2-Cl | 5-Cl | H | $C_2H_5$ | $CF_2CF_3$ | Fp. 157-158°C |
| 1.084 | 2-Cl | 5-Cl | H | $C_3H_7(i)$ | $CF_2CF_3$ | Fp. 161-162°C |
| 1.085 | 2-Cl | 5-Cl | H | $C_3H_7(n)$ | $CF_2CF_3$ | |
| 1.086 | 2-Cl | 5-Cl | H | $OCF_2CHF_2$ | $CF_3$ | |
| 1.087 | 2-Cl | 5-Cl | H | $CH_3$ | $CCl_2CF_3$ | |
| 1.088 | 2-Cl | 5-Cl | H | $C_2H_5$ | $CCl_2CF_3$ | |
| 1.089 | 2-Cl | 5-Cl | H | $CH_3$ | $CHCl_2$ | Fp. 157-159°C |
| 1.090 | 2-Cl | 5-Cl | H | $C_2H_5$ | $CHCl_2$ | |
| 1.091 | 2-Cl | 5-Cl | H | $CH_3$ | $CCl_2CH_3$ | |
| 1.092 | 2-Cl | 5-Cl | H | $C_2H_5$ | $CCl_2CH_3$ | |
| 1.093 | 2-Cl | 6-Cl | H | $CH_3$ | $CH_3$ | Fp. 173-174°C |
| 1.094 | 2-Cl | 6-Cl | H | $CH_3$ | $C_2H_5$ | |
| 1.095 | 2-Cl | 6-Cl | H | $CH_3$ | $C_3H_7(i)$ | |
| 1.096 | 2-Cl | 6-Cl | H | $CH_3$ | Cl | Fp. 184-185°C |
| 1.097 | 2-Cl | 6-Cl | H | $CH_3$ | $CCl_2CH_3$ | |
| 1.098 | 2-Cl | 6-Cl | H | $C_2H_5$ | $CCl_2CH_3$ | |
| 1.099 | 2-Cl | 6-Cl | H | $CH_3$ | $CHCl_2$ | |
| 1.100 | 2-Cl | 6-Cl | H | $C_2H_5$ | $CHCl_2$ | |
| 1.101 | 2-Cl | 6-Cl | H | $CH_3$ | $CCl_2CF_3$ | |
| 1.102 | 2-Cl | 6-Cl | H | $C_3H_7(i)$ | $CCl_2CF_3$ | |
| 1.103 | 2-Cl | 6-Cl | H | $CH_3$ | $CHClF$ | |
| 1.104 | 2-Cl | 6-Cl | H | $C_2H_5$ | $CHClF$ | |
| 1.105 | 2-Cl | 6-Cl | H | $CH_3$ | $CF_2Cl$ | |
| 1.106 | 2-Cl | 6-Cl | H | $C_2H_5$ | $CF_2Cl$ | |
| 1.107 | 2-Cl | 6-Cl | H | $C_3H_7(i)$ | $CF_2Cl$ | |
| 1.108 | 2-Cl | 6-Cl | H | $C_3H_7(n)$ | $CF_2Cl$ | |
| 1.109 | 2-Cl | 6-Cl | H | $CH_3$ | $CF_3$ | Fp. 156-157°C |
| 1.110 | 2-Cl | 6-Cl | H | $C_2H_5$ | $CF_3$ | |
| 1.111 | 2-Cl | 6-Cl | H | $C_3H_7(n)$ | $CF_3$ | |
| 1.112 | 2-Cl | 6-Cl | H | $C_3H_7(i)$ | $CF_3$ | |
| 1.113 | 2-Cl | 6-Cl | H | Cyclo-propyl | $CF_3$ | Fp. 187-188°C |
| 1.114 | 2-Cl | 6-Cl | H | Phenyl | $CF_3$ | |
| 1.115 | 2-Cl | 6-Cl | H | 2-Thienyl | $CF_3$ | Fp. 201-202°C |
| 1.116 | 2-Cl | 6-Cl | H | 2-Furyl | $CF_3$ | |
| 1.117 | 2-Cl | 6-Cl | H | $CH_3$ | $CF_2CF_3$ | |
| 1.118 | 2-Cl | 6-Cl | H | $C_2H_5$ | $CF_2CF_3$ | Fp. 168-169°C |
| 1.119 | 2-Cl | 6-Cl | H | $C_3H_7(i)$ | $CF_2CF_3$ | Fp. 154-155°C |
| 1.120 | 2-Cl | 6-Cl | H | $C_3H_7(n)$ | $CF_2CF_3$ | |
| 1.121 | 2-Cl | 6-Cl | H | Cyclo-propyl | $CF_2CF_3$ | |
| 1.122 | 2-Cl | 6-Cl | H | $CH_3$ | $OCHF_2$ | |
| 1.123 | 2-Cl | 6-Cl | H | $C_4H_9(n)$ | $CF_3$ | |
| 1.124 | 2-Cl | 6-Cl | H | $C_4H_9(i)$ | $CF_3$ | |
| 1.125 | 2-Cl | 6-Cl | H | $C_4H_9(t)$ | $CF_3$ | Fp. 174-175°C |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.126 | 2-Cl | 6-Cl | H | $OCH_3$ | $CF_3$ | |
| 1.127 | 2-Cl | 6-Cl | H | $OC_2H_5$ | $CF_3$ | |
| 1.128 | 2-Cl | 6-Cl | H | $OC_3H_7(n)$ | $CF_3$ | |
| 1.129 | 2-Cl | 6-Cl | H | $OC_3H_7(i)$ | $CF_3$ | |
| 1.130 | 2-Cl | 6-Cl | H | $OC_4H_9(n)$ | $CF_3$ | |
| 1.131 | 2-Cl | 6-Cl | H | $OC_4H_9(i)$ | $CF_3$ | |
| 1.132 | 2-Cl | 6-Cl | H | $SCH_3$ | $CF_3$ | Fp. 158-159°C |
| 1.133 | 2-Cl | 6-Cl | H | $SC_2H_5$ | $CF_3$ | |
| 1.134 | 2-Cl | 6-Cl | H | $SC_3H_7(n)$ | $CF_3$ | |
| 1.135 | 2-Cl | 6-Cl | H | $SC_3H_7(i)$ | $CF_3$ | |
| 1.136 | 2-Cl | 6-Cl | H | $SC_4H_9(n)$ | $CF_3$ | |
| 1.137 | 2-Cl | 6-Cl | H | $OCH_3$ | $CH_3$ | |
| 1.138 | 2-Cl | 6-Cl | H | $C_2H_5$ | Cl | |
| 1.139 | 2-Cl | 6-Cl | H | $OCH_3$ | $C_2H_5$ | |
| 1.140 | 2-Cl | 6-Cl | H | $C_2H_5$ | $OCHF_2$ | |
| 1.141 | 2-Cl | 6-Cl | $3-CH_3$ | $CH_3$ | $CF_3$ | Fp. 182°C u. Zers. |
| 1.142 | 2-Cl | 6-Cl | $3-CH_3$ | $C_2H_5$ | $CF_3$ | |
| 1.143 | 2-Cl | 6-Cl | $3-CH_3$ | $C_3H_7(n)$ | $CF_3$ | |
| 1.144 | 2-Cl | 6-Cl | $3-CH_3$ | $C_3H_7(i)$ | $CF_3$ | Fp. 160-162°C |
| 1.145 | 2-Cl | 6-Cl | $3-CH_3$ | $CH_3$ | $CF_2CF_3$ | Fp. 164-166°C |
| 1.146 | 2-Cl | 6-Cl | $3-CH_3$ | $C_2H_5$ | $CF_2CF_3$ | |
| 1.147 | 2-Cl | 6-Cl | $3-CH_3$ | Cl | $CF_3$ | |
| 1.148 | 2-Cl | 6-Cl | $3-CH_3$ | $OCH_3$ | $CF_3$ | |
| 1.149 | 2-Cl | 6-Cl | $3-CH_3$ | $OC_2H_5$ | $CF_3$ | |
| 1.150 | 2-Cl | 6-Cl | $3-CH_3$ | $SCH_3$ | $CF_3$ | |
| 1.151 | 2-Cl | 6-Cl | $3-CH_3$ | $SC_4H_9(n)$ | $CF_3$ | |
| 1.152 | 2-Cl | 6-Cl | $3-CH_3$ | $CH_3$ | $CF_2Cl$ | |
| 1.153 | 2-Cl | 6-Cl | $3-CH_3$ | $CH_3$ | $CHF_2$ | |
| 1.154 | 2-Cl | 6-Cl | $3-CH_3$ | $CH_3$ | $CHCl_2$ | |
| 1.155 | 2-Cl | $3-CF_3$ | H | $CH_3$ | Cl | |
| 1.156 | 2-Cl | $3-CF_3$ | H | $CH_3$ | $CH_3$ | |
| 1.157 | 2-Cl | $3-CF_3$ | H | $CH_3$ | $CF_3$ | |
| 1.158 | 2-Cl | $3-CF_3$ | H | $C_2H_5$ | $CF_3$ | |
| 1.159 | 2-Cl | $3-CF_3$ | H | $C_3H_7(n)$ | $CF_3$ | |
| 1.160 | 2-Cl | $3-CF_3$ | H | $CH_3$ | $CF_2CF_3$ | |
| 1.161 | 2-Cl | $3-CF_3$ | H | $CH_3$ | $CClF_2$ | |
| 1.162 | 2-Cl | $3-CF_3$ | H | $CH_3$ | $CHClF$ | |
| 1.163 | 2-Cl | $5-CF_3$ | H | $CH_3$ | Cl | Fp. 148-149°C |
| 1.164 | 2-Cl | $5-CF_3$ | H | $CH_3$ | $CF_3$ | Fp. 144-145°C |
| 1.165 | 2-Cl | $5-CF_3$ | H | $CH_3$ | $CClF_2$ | |
| 1.166 | 2-Cl | $5-CF_3$ | H | $C_2H_5$ | $CF_3$ | |
| 1.167 | 2-Cl | $5-CF_3$ | H | $OCH_3$ | $CF_3$ | |
| 1.168 | 2-Cl | $6-CF_3$ | H | $CH_3$ | Cl | |
| 1.169 | 2-Cl | $6-CF_3$ | H | $CH_3$ | $CH_3$ | |
| 1.170 | 2-Cl | $6-CF_3$ | H | $CH_3$ | $CF_3$ | Fp. 155-156°C |
| 1.171 | 2-Cl | $6-CF_3$ | H | $C_2H_5$ | $CF_3$ | |
| 1.172 | 2-Cl | $6-CF_3$ | H | $C_3H_7(n)$ | $CF_3$ | |
| 1.173 | 2-Cl | $6-CF_3$ | H | $C_3H_7(i)$ | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.174 | 2-Cl | 6-$CF_3$ | H | Cyclopropyl | $CF_3$ | |
| 1.175 | 2-Cl | 6-$CF_3$ | H | $C_4H_9$(n) | $CF_3$ | |
| 1.176 | 2-Cl | 6-$CF_3$ | H | $C_4H_9$(i) | $CF_3$ | |
| 1.177 | 2-Cl | 6-$CF_3$ | H | $C_4H_9$(t) | $CF_3$ | |
| 1.178 | 2-Cl | 6-$CF_3$ | H | $CH_3$ | $CF_2CF_3$ | |
| 1.179 | 2-Cl | 6-$CF_3$ | H | $C_2H_5$ | $CF_2CF_3$ | |
| 1.180 | 2-Cl | 6-$CF_3$ | H | $CH_3$ | $CHCl_2$ | |
| 1.181 | 2-Cl | 6-$CF_3$ | H | $CH_3$ | $CClF_2$ | |
| 1.182 | 2-Cl | 6-$CF_3$ | H | $C_2H_5$ | $CClF_2$ | |
| 1.183 | 2-Cl | 6-$CF_3$ | H | $CH_3$ | $CHF_2$ | |
| 1.184 | 2-Cl | 6-$CF_3$ | H | $CH_3$ | $OCHF_2$ | |
| 1.185 | 2-Cl | 6-$CF_3$ | H | $OCH_3$ | $CF_3$ | |
| 1.186 | 2-Cl | 6-$CF_3$ | H | $OC_2H_5$ | $CF_3$ | |
| 1.187 | 2-Cl | 6-$CF_3$ | H | $SC_2H_5$ | $CF_3$ | |
| 1.188 | 2-Cl | 6-$CF_3$ | H | $CH_3$ | $CCl_2CF_3$ | |
| 1.189 | 2-Cl | 6-$CF_3$ | H | $CH_3$ | $CCl_2CH_3$ | |
| 1.190 | 2-Cl | 6-$CF_3$ | H | Phenyl | $CF_3$ | |
| 1.191 | 2-Cl | 3-$CH_3$ | H | $CH_3$ | $CF_3$ | |
| 1.192 | 2-Cl | 3-$CH_3$ | H | $CH_3$ | $CF_2CF_3$ | |
| 1.193 | 2-Cl | 3-$CH_3$ | H | $C_2H_5$ | $CF_3$ | |
| 1.194 | 2-Cl | 3-$CH_3$ | H | $C_3H_7$(n) | $CF_3$ | |
| 1.195 | 2-Cl | 3-$CH_3$ | H | $C_3H_7$(i) | $CF_3$ | |
| 1.196 | 2-Cl | 3-$CH_3$ | H | $CH_3$ | Cl | |
| 1.197 | 2-Cl | 3-$CH_3$ | H | $CH_3$ | $CF_2Cl$ | |
| 1.198 | 2-Cl | 3-$CH_3$ | H | $CH_3$ | $CHCl_2$ | |
| 1.199 | 2-Cl | 3-$CH_3$ | H | $CH_3$ | CHClF | |
| 1.200 | 2-Cl | 3-$CH_3$ | H | $OCH_3$ | $CF_3$ | |
| 1.201 | 2-Cl | 5-$CH_3$ | H | $CH_3$ | Cl | Fp. 140-141°C |
| 1.202 | 2-Cl | 5-$CH_3$ | H | $CH_3$ | $CHCl_2$ | |
| 1.203 | 2-Cl | 5-$CH_3$ | H | $CH_3$ | $CCl_2CF_3$ | |
| 1.204 | 2-Cl | 5-$CH_3$ | H | $CH_3$ | CHClF | |
| 1.205 | 2-Cl | 5-$CH_3$ | H | $CH_3$ | $CF_2Cl$ | |
| 1.206 | 2-Cl | 5-$CH_3$ | H | $C_2H_5$ | $CF_2Cl$ | |
| 1.207 | 2-Cl | 5-$CH_3$ | H | $CH_3$ | $CF_3$ | |
| 1.208 | 2-Cl | 5-$CH_3$ | H | $C_2H_5$ | $CF_3$ | |
| 1.209 | 2-Cl | 5-$CH_3$ | H | $C_3H_7$(n) | $CF_3$ | |
| 1.210 | 2-Cl | 5-$CH_3$ | H | $C_3H_7$(i) | $CF_3$ | |
| 1.211 | 2-Cl | 5-$CH_3$ | H | $C_4H_9$(n) | $CF_3$ | |
| 1.212 | 2-Cl | 5-$CH_3$ | H | $C_4H_9$(t) | $CF_3$ | |
| 1.213 | 2-Cl | 5-$CH_3$ | H | 2-Furyl | $CF_3$ | |
| 1.214 | 2-Cl | 5-$CH_3$ | H | $CH_3$ | $CF_2CF_3$ | |
| 1.215 | 2-Cl | 5-$CH_3$ | H | $C_2H_5$ | $CF_2CF_3$ | |
| 1.216 | 2-Cl | 5-$CH_3$ | H | $OCH_3$ | $CF_3$ | |
| 1.217 | 2-Cl | 5-$CH_3$ | H | $OC_2H_5$ | $CF_3$ | |
| 1.218 | 2-Cl | 5-$CH_3$ | H | $SCH_3$ | $CF_3$ | |
| 1.219 | 2-Cl | 6-$CH_3$ | H | $CH_3$ | Cl | Fp. 167-168°C |
| 1.220 | 2-Cl | 6-$CH_3$ | H | CN | $CH_3$ | |
| 1.221 | 2-Cl | 6-$CH_3$ | H | $CH_3$ | $CF_3$ | Fp. 192-193°C |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.222 | 2-Cl | 6-$CH_3$ | H | $CH_3$ | $CF_2Cl$ | Fp. 185-187°C |
| 1.223 | 2-Cl | 6-$CH_3$ | H | $CH_3$ | $CF_2CF_3$ | |
| 1.224 | 2-Cl | 6-$CH_3$ | H | $CH_3$ | $CCl_2CF_3$ | |
| 1.225 | 2-Cl | 6-$CH_3$ | H | $CH_3$ | $CHF_2$ | |
| 1.226 | 2-Cl | 6-$CH_3$ | H | $CH_3$ | $CHCl_2$ | |
| 1.227 | 2-Cl | 6-$CH_3$ | H | $C_2H_5$ | $CF_3$ | Fp. 155-157°C |
| 1.228 | 2-Cl | 6-$CH_3$ | H | $C_3H_7$(i) | $CF_3$ | Fp. 146-148°C |
| 1.229 | 2-Cl | 6-$CH_3$ | H | Cyclo-propyl | $CF_3$ | Fp. 177-178°C |
| 1.230 | 2-Cl | 6-$CH_3$ | H | $C_4H_9$(i) | $CF_3$ | |
| 1.231 | 2-Cl | 6-$CH_3$ | H | $C_2H_5$ | $CF_2CF_3$ | |
| 1.232 | 2-Cl | 6-$CH_3$ | H | $OCH_3$ | $CF_3$ | |
| 1.233 | 2-Cl | 6-$CH_3$ | H | $OCH_2CH_2Cl$ | $CF_3$ | |
| 1.234 | 2-Cl | 6-$CH_3$ | H | $OCH_2CH_2OCH_3$ | $CF_3$ | |
| 1.235 | 2-Cl | 6-$CH_3$ | H | $OC_2H_5$ | $CF_3$ | |
| 1.236 | 2-Cl | 6-$CH_3$ | H | $SCH_3$ | $CF_3$ | |
| 1.237 | 2-Cl | 6-$CH_3$ | H | $SC_2H_5$ | $CF_3$ | |
| 1.238 | 2-Cl | 6-$CH_3$ | H | $SC_3H_7$(i) | $CF_3$ | |
| 1.239 | 2-Cl | 6-$CH_3$ | H | $SC_4H_9$(i) | $CF_3$ | |
| 1.240 | 2-Cl | 5-$OCH_3$ | H | $CH_3$ | $CF_3$ | |
| 1.241 | 2-Cl | 5-$OCH_3$ | H | $C_2H_5$ | $CF_3$ | |
| 1.242 | 2-Cl | 5-$OCH_3$ | H | $CH_3$ | Cl | |
| 1.243 | 2-Cl | 5-$OCH_3$ | H | $OCH_3$ | $CF_3$ | |
| 1.244 | 2-Cl | 5-$OCH_3$ | H | $CH_3$ | $CHF_2$ | |
| 1.245 | 2-Cl | 5-$OCH_3$ | H | $CH_3$ | $CF_2CF_3$ | |
| 1.246 | 2-Cl | 5-$OCH_3$ | H | $CH_3$ | $CF_2Cl$ | |
| 1.247 | 2-Cl | 5-$COOCH_3$ | H | $CH_3$ | $CF_3$ | Fp. 152-153°C |
| 1.248 | 2-Cl | 5-$COOCH_3$ | H | $C_2H_5$ | $CF_3$ | |
| 1.249 | 2-Cl | 5-$COOC_2H_5$ | H | $CH_3$ | $CF_3$ | |
| 1.250 | 2-Cl | 5-$COOC_2H_5$ | H | $CH_3$ | $CF_2CF_3$ | |
| 1.251 | 2-Cl | 5-$COOC_3H_7$(i) | H | $CH_3$ | $CF_3$ | |
| 1.252 | 2-Cl | 5-$COOC_3H_7$(i) | H | $C_3H_7$(i) | $CF_3$ | |
| 1.253 | 2-Cl | 5-$COOC_3H_7$(i) | H | $CH_3$ | $CF_2Cl$ | |
| 1.254 | 2-Cl | 5-$COOC_3H_7$(i) | H | Phenyl | $CF_3$ | |
| 1.255 | 2-Cl | 5-$COOC_3H_7$(i) | H | Cyclo-propyl | $CF_3$ | |
| 1.256 | 2-Cl | 5-$COOC_3H_7$(i) | H | $CH_3$ | CHFCl | |
| 1.257 | 3-Cl | 5-Cl | H | $CH_3$ | Cl | Fp. 117-118°C |
| 1.258 | 3-Cl | 5-Cl | H | $CH_3$ | $CF_3$ | Fp. 129-130°C |
| 1.259 | 3-Cl | 5-Cl | H | $C_2H_5$ | $CF_3$ | |
| 1.260 | 3-Cl | 5-Cl | H | $C_3H_7$(n) | $CF_3$ | |
| 1.261 | 3-Cl | 5-Cl | H | $C_3H_7$(i) | $CF_3$ | |
| 1.262 | 3-Cl | 5-Cl | H | Cyclo-propyl | $CF_3$ | |
| 1.263 | 3-Cl | 5-Cl | H | Phenyl | $CF_3$ | |
| 1.264 | 3-Cl | 5-Cl | H | $CH_3$ | $CHF_2$ | |
| 1.265 | 3-Cl | 5-Cl | H | $CH_3$ | $CF_2Cl$ | |
| 1.266 | 3-Cl | 5-Cl | H | $C_2H_5$ | $CF_2Cl$ | |
| 1.267 | 3-Cl | 5-Cl | H | $OCH_3$ | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.268 | 3-Cl | 5-Cl | H | $OC_2H_5$ | $CF_3$ | |
| 1.269 | 3-Cl | 5-Cl | H | $SCH_3$ | $CF_3$ | |
| 1.270 | 3-Cl | 5-Cl | H | $SC_3H_7$(i) | $CF_3$ | |
| 1.271 | 2-$CH_3$ | 5-Cl | H | $CH_3$ | Cl | Fp. 141-142°C |
| 1.272 | 2-$CH_3$ | 5-Cl | H | $CH_3$ | $CF_3$ | |
| 1.273 | 2-$CH_3$ | 5-Cl | H | CN | $CF_3$ | |
| 1.274 | 2-$CH_3$ | 5-Cl | H | $C_2H_5$ | Cl | |
| 1.275 | 2-$CH_3$ | 5-Cl | H | $CH_3$ | $OCHF_2$ | |
| 1.276 | 2-$CH_3$ | 5-Cl | H | $CH_3$ | Br | |
| 1.277 | 2-$CH_3$ | 5-Cl | H | $CH_3$ | F | |
| 1.278 | 2-$CH_3$ | 5-Cl | H | $CH_3$ | $CH_3$ | Fp. 150-151°C |
| 1.279 | 2-$CH_3$ | 5-Cl | H | $OCH_3$ | $CH_3$ | |
| 1.280 | 2-$CH_3$ | 5-Cl | H | $C_2H_5$ | $CF_3$ | |
| 1.281 | 2-$CH_3$ | 5-Cl | H | $C_3H_7$(n) | $CF_3$ | |
| 1.282 | 2-$CH_3$ | 5-Cl | H | $C_3H_7$(i) | $CF_3$ | |
| 1.283 | 2-$CH_3$ | 5-Cl | H | Cyclo-propyl | $CF_3$ | |
| 1.284 | 2-$CH_3$ | 5-Cl | H | Phenyl | $CF_3$ | |
| 1.285 | 2-$CH_3$ | 5-Cl | H | $C_4H_9$(n) | $CF_3$ | |
| 1.286 | 2-$CH_3$ | 5-Cl | H | $C_4H_9$(i) | $CF_3$ | |
| 1.287 | 2-$CH_3$ | 5-Cl | H | $C_4H_9$(t) | $CF_3$ | |
| 1.288 | 2-$CH_3$ | 5-Cl | H | $CH_3$ | $CF_2CF_3$ | |
| 1.289 | 2-$CH_3$ | 5-Cl | H | $C_2H_5$ | $CF_2CF_3$ | |
| 1.290 | 2-$CH_3$ | 5-Cl | H | $C_3H_7$(i) | $CF_2CF_3$ | |
| 1.291 | 2-$CH_3$ | 5-Cl | H | $CH_3$ | $CF_2Cl$ | |
| 1.292 | 2-$CH_3$ | 5-Cl | H | $C_2H_5$ | $CF_2Cl$ | |
| 1.293 | 2-$CH_3$ | 5-Cl | H | $CH_3$ | $CHF_2$ | |
| 1.294 | 2-$CH_3$ | 5-Cl | H | $C_2H_5$ | $CHF_2$ | |
| 1.295 | 2-$CH_3$ | 5-Cl | H | $CH_3$ | CHFCl | |
| 1.296 | 2-$CH_3$ | 5-Cl | H | $C_2H_5$ | CHFCl | |
| 1.297 | 2-$CH_3$ | 5-Cl | H | $CH_3$ | $CHCl_2$ | |
| 1.298 | 2-$CH_3$ | 5-Cl | H | $C_2H_5$ | $CHCl_2$ | |
| 1.299 | 2-$CH_3$ | 5-Cl | H | $CH_3$ | $CCl_2CF_3$ | |
| 1.300 | 2-$CH_3$ | 5-Cl | H | $CH_3$ | $CCl_2CH_3$ | |
| 1.301 | 2-$CH_3$ | 5-Cl | H | $OCH_3$ | $CF_3$ | |
| 1.302 | 2-$CH_3$ | 5-Cl | H | $OC_2H_5$ | $CF_3$ | |
| 1.303 | 2-$CH_3$ | 5-Cl | H | $SCH_3$ | $CF_3$ | |
| 1.304 | 2-$CH_3$ | 5-Cl | H | $SC_2H_5$ | $CF_3$ | |
| 1.305 | 2-$CH_3$ | 5-Cl | H | $SC_4H_9$(i) | $CF_3$ | |
| 1.306 | 2-$CH_3$ | 3-Cl | H | $CH_3$ | Cl | Fp. 155-156°C |
| 1.307 | 2-$CH_3$ | 3-Cl | H | $CH_3$ | $CF_3$ | |
| 1.308 | 2-$CH_3$ | 3-Cl | H | $C_2H_5$ | $CF_3$ | |
| 1.309 | 2-$CH_3$ | 3-Cl | H | $C_3H_7$(i) | $CF_3$ | |
| 1.310 | 2-$CH_3$ | 3-Cl | H | Cyclo-propyl | $CF_3$ | |
| 1.311 | 2-$CH_3$ | 3-Cl | H | $CH_3$ | $CF_2Cl$ | |
| 1.312 | 2-$CH_3$ | 3-Cl | H | $CH_3$ | $CF_2CF_3$ | |
| 1.313 | 2-$CH_3$ | 3-Cl | H | $C_2H_5$ | $CF_2CF_3$ | |
| 1.314 | 2-$CH_3$ | 3-Cl | H | $OCH_3$ | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.315 | 2-$CH_3$ | 3-Cl | H | $SCH_3$ | $CF_3$ | |
| 1.316 | 2-$CH_3$ | 5-$NO_2$ | H | $CH_3$ | Cl | Fp. 142-143°C |
| 1.317 | 2-$CH_3$ | 5-$NO_2$ | H | $CH_3$ | $CF_3$ | |
| 1.318 | 2-$CH_3$ | 5-$NO_2$ | H | $CH_3$ | $CF_2Cl$ | |
| 1.319 | 2-$CH_3$ | 5-$NO_2$ | H | $CH_3$ | $CHF_2$ | |
| 1.320 | 2-$CH_3$ | 5-$NO_2$ | H | $C_2H_5$ | $CF_3$ | |
| 1.321 | 2-$CH_3$ | 5-$NO_2$ | H | $C_3H_7$(n) | $CF_3$ | |
| 1.322 | 2-$CH_3$ | 5-$NO_2$ | H | $C_3H_7$(i) | $CF_3$ | |
| 1.323 | 2-$CH_3$ | 5-$NO_2$ | H | $C_4H_9$(t) | $CF_3$ | |
| 1.324 | 2-$CH_3$ | 3-$NO_2$ | H | $CH_3$ | Cl | Fp. 158-159°C |
| 1.325 | 2-$CH_3$ | 3-$NO_2$ | H | $CH_3$ | $CF_3$ | |
| 1.326 | 2-$CH_3$ | 3-$NO_2$ | H | $CH_3$ | $CF_2CF_3$ | |
| 1.327 | 2-$CH_3$ | 3-$NO_2$ | H | $C_2H_5$ | $CF_2CF_3$ | |
| 1.328 | 2-$CH_3$ | 3-$NO_2$ | H | Phenyl | $CF_3$ | |
| 1.329 | 2-$CH_3$ | 3-$NO_2$ | H | $C_2H_5$ | $CF_3$ | |
| 1.330 | 2-$CH_3$ | 3-$NO_2$ | H | $C_3H_7$(i) | $CF_3$ | |
| 1.331 | 2-$CH_3$ | 6-$CF_3$ | H | $CH_3$ | Cl | |
| 1.332 | 2-$CH_3$ | 6-$CF_3$ | H | $CH_3$ | $CF_3$ | Fp. 164-165°C |
| 1.333 | 2-$CH_3$ | 6-$CF_3$ | H | $CH_3$ | $CHF_2$ | |
| 1.334 | 2-$CH_3$ | 6-$CF_3$ | H | $CH_3$ | $CF_2Cl_2$ | |
| 1.335 | 2-$CH_3$ | 6-$CF_3$ | H | $CH_3$ | $CHCl_2$ | |
| 1.336 | 2-$CH_3$ | 6-$CF_3$ | H | $OCH_3$ | $CF_3$ | |
| 1.337 | 2-$CH_3$ | 6-$CF_3$ | H | $OC_2H_5$ | $CF_3$ | |
| 1.338 | 2-$CH_3$ | 6-$CF_3$ | H | $SCH_3$ | $CF_3$ | |
| 1.339 | 2-$CH_3$ | 6-$CF_3$ | H | $SC_2H_5$ | $CF_3$ | |
| 1.340 | 2-$CH_3$ | 6-$CF_3$ | H | $C_2H_5$ | $CF_3$ | |
| 1.341 | 2-$CH_3$ | 6-$CF_3$ | H | $CH_3$ | $CF_2CF_3$ | |
| 1.342 | 2-$CH_3$ | 6-$CF_3$ | H | $C_2H_5$ | $CF_2CF_3$ | |
| 1.343 | 2-$CH_3$ | 6-$CF_3$ | H | $C_3H_7$(i) | $CF_3$ | |
| 1.344 | 2-$CH_3$ | 6-$CF_3$ | H | $C_3H_7$(n) | $CF_3$ | |
| 1.345 | 2-$CH_3$ | 6-$CF_3$ | H | Cyclo-propyl | $CF_3$ | |
| 1.346 | 2-$OCHF_2$ | 5-Cl | H | $CH_3$ | Cl | Fp. 138-139°C |
| 1.347 | 2-$OCHF_2$ | 5-Cl | H | $CH_3$ | $CH_3$ | |
| 1.348 | 2-$OCHF_2$ | 5-Cl | H | $CH_3$ | $CHCl_2$ | |
| 1.349 | 2-$OCHF_2$ | 5-Cl | H | $CH_3$ | CHFCl | |
| 1.350 | 2-$OCHF_2$ | 5-Cl | H | $CH_3$ | $CHF_2$ | |
| 1.351 | 2-$OCHF_2$ | 5-Cl | H | $CH_3$ | $CF_2Cl$ | |
| 1.352 | 2-$OCHF_2$ | 5-Cl | H | $CH_3$ | $CF_3$ | Fp. 142-143°C |
| 1.353 | 2-$OCHF_2$ | 5-Cl | H | $CH_3$ | $CF_2CF_3$ | |
| 1.354 | 2-$OCHF_2$ | 5-Cl | H | $CH_3$ | $CCl_2CF_3$ | |
| 1.355 | 2-$OCHF_2$ | 5-Cl | H | $CH_3$ | $CCl_2CH_3$ | |
| 1.356 | 2-$OCHF_2$ | 5-Cl | H | $C_2H_5$ | $CF_3$ | |
| 1.357 | 2-$OCHF_2$ | 5-Cl | H | $C_3H_7$(n) | $CF_3$ | |
| 1.358 | 2-$OCHF_2$ | 5-Cl | H | $C_3H_7$(i) | $CF_3$ | |
| 1.359 | 2-$CH_3$ | 6-Cl | H | Cyclo-propyl | $CF_3$ | |
| 1.360 | 2-$CH_3$ | 6-Cl | H | $C_4H_9$(n) | $CF_3$ | |
| 1.361 | 2-$CH_3$ | 6-Cl | H | $C_4H_9$(i) | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.362 | 2-$CH_3$ | 6-Cl | H | $C_4H_9$(t) | $CF_3$ | |
| 1.363 | 2-$CH_3$ | 6-Cl | H | Phenyl | $CF_3$ | |
| 1.364 | 2-$CH_3$ | 6-Cl | H | 2-Furyl | $CF_3$ | |
| 1.365 | 2-$CH_3$ | 6-Cl | H | 2-Thienyl | $CF_3$ | |
| 1.366 | 2-$CH_3$ | 6-Cl | H | $C_2H_5$ | $CF_2CF_3$ | |
| 1.367 | 2-$CH_3$ | 6-Cl | H | $C_2H_5$ | $CCl_2CH_3$ | |
| 1.368 | 2-$CH_3$ | 6-Cl | H | $OCH_3$ | $CF_3$ | |
| 1.369 | 2-$CH_3$ | 6-Cl | H | $OC_2H_5$ | $CF_3$ | |
| 1.370 | 2-$CH_3$ | 6-Cl | H | $SCH_3$ | $CF_3$ | |
| 1.371 | 2-Cl | 6-Cl | 3-Cl | $CH_3$ | Cl | |
| 1.372 | 2-Cl | 6-Cl | 3-Cl | $CH_3$ | $CF_3$ | |
| 1.373 | 2-Cl | 6-Cl | 3-Cl | $C_2H_5$ | $CF_3$ | |
| 1.374 | 2-Cl | 6-Cl | 3-Cl | $C_3H_7$(i) | $CF_3$ | |
| 1.375 | 2-Cl | 6-Cl | 3-Cl | $CH_3$ | $CF_2CF_3$ | |
| 1.376 | 2-Cl | 6-Cl | 3-Cl | Phenyl | $CF_3$ | |
| 1.377 | 2-Cl | 6-Cl | 3-Cl | $OCH_3$ | $CF_3$ | |
| 1.378 | 2-Cl | 6-Cl | 3-Cl | $OC_2H_5$ | $CF_3$ | |
| 1.379 | 2-Cl | 6-Cl | 3-Cl | $SC_2H_5$ | $CF_3$ | |
| 1.380 | 2-$OCH_3$ | 3-Cl | H | $CH_3$ | $CF_3$ | Fp. 179-180°C |
| 1.381 | 2-$OCH_3$ | 3-Cl | H | $C_2H_5$ | $CF_3$ | |
| 1.382 | 2-$OCH_3$ | 3-Cl | H | $C_3H_7$(n) | $CF_3$ | |
| 1.383 | 2-$OCH_3$ | 3-Cl | H | Cyclo-propyl | $CF_3$ | |
| 1.384 | 2-$OCH_3$ | 3-Cl | H | $CH_3$ | Cl | |
| 1.385 | 2-$OCH_3$ | 3-Cl | H | $CH_3$ | $CF_2CF_3$ | |
| 1.386 | 2-$OCH_3$ | 3-Cl | H | $OCH_3$ | $CF_3$ | |
| 1.387 | 2-$OCH_3$ | 3-Cl | H | $SCH_3$ | $CF_3$ | |
| 1.388 | 2-$OCH_3$ | 3-Cl | H | $CH_3$ | $CF_2Cl$ | |
| 1.389 | 2-$OCH_3$ | 3-Cl | H | $CH_3$ | $CHF_2$ | |
| 1.390 | 2-$OCH_3$ | 5-Cl | H | $CH_3$ | Cl | |
| 1.391 | 2-$OCH_3$ | 5-Cl | H | $CH_3$ | $CH_3$ | Fp. 157-158°C |
| 1.392 | 2-$OCH_3$ | 5-Cl | H | $CH_3$ | $CF_3$ | Fp. 158-159°C |
| 1.393 | 2-$OCH_3$ | 5-Cl | H | $CH_3$ | Br | |
| 1.394 | 2-$OCH_3$ | 5-Cl | H | CN | $CH_3$ | |
| 1.395 | 2-$OCH_3$ | 5-Cl | H | $CH_3$ | $CF_2Cl$ | |
| 1.396 | 2-$OCH_3$ | 5-Cl | H | $CH_3$ | $CHF_2$ | |
| 1.397 | 2-$OCH_3$ | 5-Cl | H | $CH_3$ | $CHCl_2$ | |
| 1.398 | 2-$OCH_3$ | 5-Cl | H | $CH_3$ | $CF_2CF_3$ | Fp. 125-126°C |
| 1.399 | 2-$OCH_3$ | 5-Cl | H | $CH_3$ | $CCl_2CF_3$ | |
| 1.400 | 2-$OCH_3$ | 5-Cl | H | $CH_3$ | CHClF | |
| 1.401 | 2-$OCH_3$ | 5-Cl | H | $CH_3$ | $CCl_2CH_3$ | |
| 1.402 | 2-$OCH_3$ | 5-Cl | H | $C_2H_5$ | $CF_3$ | |
| 1.403 | 2-$OCH_3$ | 5-Cl | H | $C_2H_5$ | $CF_2CF_3$ | Fp. 148-150°C |
| 1.404 | 2-$OCH_3$ | 5-Cl | H | $C_2H_5$ | $CF_2Cl$ | |
| 1.405 | 2-$OCH_3$ | 5-Cl | H | $C_3H_7$(n) | $CF_3$ | Fp. 168-169°C |
| 1.406 | 2-$OCH_3$ | 5-Cl | H | $C_3H_7$(i) | $CF_3$ | Fp. 167-168°C |
| 1.407 | 2-$OCH_3$ | 5-Cl | H | $C_3H_7$(i) | $CF_2CF_3$ | |
| 1.408 | 2-$OCH_3$ | 5-Cl | H | Cyclo-propyl | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.409 | 2-$OCH_3$ | 5-Cl | H | $C_4H_9$(n) | $CF_3$ | |
| 1.410 | 2-$OCH_3$ | 5-Cl | H | $C_2H_5$(i) | $CF_3$ | |
| 1.411 | 2-$OCH_3$ | 5-Cl | H | $C_4H_9$(t) | $CF_3$ | Fp. 156-157°C |
| 1.412 | 2-$OCH_3$ | 5-Cl | H | $OCH_3$ | $CF_3$ | |
| 1.413 | 2-$OCH_3$ | 5-Cl | H | $OC_4H_9$(n) | $CF_3$ | |
| 1.414 | 2-$OCH_3$ | 5-Cl | H | $SCH_3$ | $CF_3$ | |
| 1.415 | 2-$OCH_3$ | 5-Cl | H | $SC_2H_5$ | $CF_3$ | |
| 1.416 | 2-$OCH_3$ | 5-Cl | H | $SC_3H_7$(n) | $CF_3$ | |
| 1.417 | 2-$OCH_3$ | 5-Cl | H | $SC_3H_7$(i) | $CF_3$ | |
| 1.418 | 2-$OC_2H_5$ | 5-Cl | H | $CH_3$ | Cl | |
| 1.419 | 2-$OC_2H_5$ | 5-Cl | H | $CH_3$ | $CF_3$ | |
| 1.420 | 2-$OC_2H_5$ | 5-Cl | H | $C_2H_5$ | $CF_3$ | |
| 1.421 | 2-$OC_2H_5$ | 5-Cl | H | $C_3H_7$(i) | $CF_3$ | |
| 1.422 | 2-$OC_2H_5$ | 5-Cl | H | Cyclo-propyl | $CF_3$ | |
| 1.423 | 2-$OC_2H_5$ | 5-Cl | H | Phenyl | $CF_3$ | |
| 1.424 | 2-$OC_2H_5$ | 5-Cl | H | $OCH_3$ | $CF_3$ | |
| 1.425 | 2-$OC_2H_5$ | 5-Cl | H | $CH_3$ | $CF_2CF_3$ | |
| 1.426 | 2-$OC_3H_7$(i) | 5-Cl | H | $CH_3$ | Cl | |
| 1.427 | 2-$OC_3H_7$(i) | 5-Cl | H | $CH_3$ | $CF_3$ | |
| 1.428 | 2-$OC_3H_7$(i) | 5-Cl | H | $CH_3$ | $CF_2Cl$ | |
| 1.429 | 2-$OC_3H_7$(i) | 5-Cl | H | $C_2H_5$ | $CF_3$ | |
| 1.430 | 2-$OC_3H_7$(i) | 5-Cl | H | $C_3H_7$(n) | $CF_3$ | |
| 1.431 | 2-$OC_3H_7$(i) | 5-Cl | H | $CH_3$ | $CHF_3$ | |
| 1.432 | 2-$OC_3H_7$(i) | 5-Cl | H | $OCH_3$ | $CF_3$ | |
| 1.433 | 2-$OC_3H_7$(i) | 5-Cl | H | $SCH_3$ | $CF_3$ | |
| 1.434 | 2-$OCF_3$ | H | H | $CH_3$ | Cl | |
| 1.435 | 2-$OCF_3$ | H | H | $CH_3$ | $CF_3$ | |
| 1.436 | 2-$OCF_3$ | H | H | $CH_3$ | $CF_2Cl$ | |
| 1.437 | 2-$OCF_3$ | H | H | $CH_3$ | $CHF_2$ | |
| 1.438 | 2-$OCF_3$ | H | H | $CH_3$ | CHFCl | |
| 1.439 | 2-$OCF_3$ | H | H | $C_2H_5$ | $CF_3$ | |
| 1.440 | 2-$OCF_3$ | H | H | $C_3H_7$(i) | $CF_3$ | |
| 1.441 | 2-$OCF_3$ | H | H | $C_4H_9$(n) | $CF_3$ | |
| 1.442 | 2-$OCF_3$ | H | H | $C_4H_9$(t) | $CF_3$ | |
| 1.443 | 2-$OCF_3$ | 5-Cl | H | $CH_3$ | $CF_3$ | |
| 1.444 | 2-$OCF_3$ | 5-Cl | H | $CH_3$ | Cl | |
| 1.445 | 2-$OCF_3$ | 5-Cl | H | $CH_3$ | $CF_3$ | |
| 1.446 | 2-$OCF_3$ | 5-Cl | H | $CH_3$ | $CF_2Cl$ | |
| 1.447 | 2-$OCF_3$ | 5-Cl | H | $CH_3$ | $CHF_2$ | |
| 1.448 | 2-$OCF_3$ | 5-Cl | H | $CH_3$ | $CHCl_2$ | |
| 1.449 | 2-$OCF_3$ | 5-Cl | H | $CH_3$ | CHFCl | |
| 1.450 | 2-$OCF_3$ | 5-Cl | H | $CH_3$ | $CF_2CF_3$ | |
| 1.451 | 2-$OCF_3$ | 5-Cl | H | $C_2H_5$ | $CF_2CF_3$ | |
| 1.452 | 2-$OCF_3$ | 5-Cl | H | $C_2H_5$ | $CF_3$ | |
| 1.453 | 2-$OCF_3$ | 5-Cl | H | $C_3H_7$(i) | $CF_3$ | |
| 1.454 | 2-$OCF_3$ | 5-Cl | H | Cyclo-propyl | $CF_3$ | |
| 1.455 | 2-$OCF_3$ | 5-Cl | H | Phenyl | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.456 | 2-$OCF_3$ | 5-Cl | H | $OC_2H_5$ | $CF_3$ | |
| 1.457 | 2-$OCF_3$ | 5-Cl | H | $SC_2H_5$ | $CF_3$ | |
| 1.458 | 2-$SCH_3$ | H | H | $CH_3$ | Cl | |
| 1.459 | 2-$SCH_3$ | H | H | $CH_3$ | $CF_3$ | Fp. 156-157°C |
| 1.460 | 2-$SCH_3$ | H | H | $CH_3$ | $CHF_2$ | |
| 1.461 | 2-$SCH_3$ | H | H | $CH_3$ | $CF_2CF_3$ | |
| 1.462 | 2-$SCH_3$ | H | H | $C_2H_5$ | $CF_3$ | |
| 1.463 | 2-$SCH_3$ | H | H | $C_3H_7$(i) | $CF_3$ | Fp. 170-171°C |
| 1.464 | 2-$SCH_3$ | H | H | $C_4H_9$(n) | $CF_3$ | |
| 1.465 | 2-$SCH_3$ | H | H | $C_4H_9$(t) | $CF_3$ | |
| 1.466 | 2-$SCH_3$ | 5-Cl | H | $CH_3$ | Cl | |
| 1.467 | 2-$SCH_3$ | 5-Cl | H | $CH_3$ | $CF_3$ | Fp. 162-163°C |
| 1.468 | 2-$SCH_3$ | 5-Cl | H | $CH_3$ | $CF_2CF_3$ | |
| 1.469 | 2-$SCH_3$ | 5-Cl | H | $CH_3$ | $CF_2Cl$ | |
| 1.470 | 2-$SCH_3$ | 5-Cl | H | $C_2H_5$ | $CF_3$ | |
| 1.471 | 2-$SOCH_3$ | H | H | $CH_3$ | Cl | |
| 1.472 | 2-$SOCH_3$ | H | H | $CH_3$ | $CF_3$ | Fp. 80-82°C |
| 1.473 | 2-$SOCH_3$ | H | H | $CH_3$ | $CHF_2$ | |
| 1.474 | 2-$SOCH_3$ | H | H | $CH_3$ | $CF_2CF_3$ | |
| 1.475 | 2-$SOCH_3$ | H | H | $C_2H_5$ | $CF_3$ | |
| 1.476 | 2-$SOCH_3$ | H | H | $C_3H_7$(i) | $CF_3$ | |
| 1.477 | 2-$SOCH_3$ | H | H | $C_4H_9$(n) | $CF_3$ | |
| 1.478 | 2-$SOCH_3$ | H | H | $C_4H_9$(t) | $CF_3$ | |
| 1.479 | 2-$SO_2CH_3$ | H | H | $CH_3$ | Cl | Fp. 164-165°C |
| 1.480 | 2-$SO_2CH_3$ | H | H | $CH_3$ | $CF_3$ | Fp. 166-167°C |
| 1.481 | 2-$SO_2CH_3$ | H | H | $C_2H_5$ | $CF_3$ | |
| 1.482 | 2-$SO_2CH_3$ | H | H | $C_3H_7$(n) | $CF_3$ | |
| 1.483 | 2-$SO_2CH_3$ | H | H | $C_3H_7$(i) | $CF_3$ | |
| 1.484 | 2-$SO_2CH_3$ | H | H | Cyclo-propyl | $CF_3$ | |
| 1.485 | 2-$SO_2CH_3$ | H | H | $C_4H_9$(i) | $CF_3$ | |
| 1.486 | 2-$SO_2CH_3$ | H | H | $CH_3$ | $CF_2CF_3$ | |
| 1.487 | 2-$SO_2CH_3$ | H | H | $C_2H_5$ | $CF_2CF_3$ | |
| 1.488 | 2-$SO_2CH_3$ | H | H | $C_3H_7$(i) | $CF_2CF_3$ | |
| 1.489 | 2-$SOCH_3$ | 5-Cl | H | $CH_3$ | Cl | |
| 1.490 | 2-$SOCH_3$ | 5-Cl | H | $CH_3$ | $CF_3$ | Fp. 132-133°C |
| 1.491 | 2-$SOCH_3$ | 5-Cl | H | $C_2H_5$ | $CF_3$ | |
| 1.492 | 2-$SOCH_3$ | 5-Cl | H | $CH_3$ | $CF_2CF_3$ | |
| 1.493 | 2-$SOCH_3$ | 5-Cl | H | $C_3H_7$(i) | $CF_3$ | |
| 1.494 | 2-$SO_2CH_3$ | 5-Cl | H | $CH_3$ | Cl | |
| 1.495 | 2-$SO_2CH_3$ | 5-Cl | H | $CH_3$ | $CF_3$ | Fp. 154-155°C |
| 1.496 | 2-$SO_2CH_3$ | 5-Cl | H | $C_2H_5$ | $CF_3$ | |
| 1.497 | 2-$SO_2CH_3$ | 5-Cl | H | $CH_3$ | $CF_2CF_3$ | |
| 1.498 | 2-$SO_2CH_3$ | 5-Cl | H | $C_3H_7$(i) | $CF_3$ | |
| 1.499 | 2-F | 5-F | H | $CH_3$ | $OCHF_2$ | |
| 1.500 | 2-F | 5-F | H | $CH_3$ | Cl | |
| 1.501 | 2-F | 5-F | H | $CH_3$ | $CF_3$ | Fp. 152-153°C |
| 1.502 | 2-F | 5-F | H | $C_2H_5$ | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.503 | 2-F | 5-F | H | $C_3H_7(n)$ | $CF_3$ | |
| 1.504 | 2-F | 5-F | H | $C_3H_7(i)$ | $CF_3$ | |
| 1.505 | 2-F | 5-F | H | Cyclo-propyl | $CF_3$ | Fp. 174-175°C |
| 1.506 | 2-F | 5-F | H | $CH_3$ | $CF_2CF_3$ | |
| 1.507 | 2-F | 5-F | H | $CH_3$ | $CF_2CF_2CF_3$ | |
| 1.508 | 2-F | 5-F | H | $CH_3$ | $CF_2Cl$ | |
| 1.509 | 2-F | 5-F | H | $CH_3$ | $CHF_2$ | |
| 1.510 | 2-F | 5-F | H | $CH_3$ | CHClF | |
| 1.511 | 2-F | 5-F | H | $C_2H_5$ | $CF_2Cl$ | |
| 1.512 | 2-F | 5-F | H | $C_3H_7(i)$ | $CF_2Cl$ | Fp. 161-162°C |
| 1.513 | 2-F | 5-F | H | $C_4H_9(n)$ | $CF_3$ | |
| 1.514 | 2-F | 5-F | H | $C_4H_9(t)$ | $CF_3$ | Fp. 156-157°C |
| 1.515 | 2-F | 5-F | H | $C_2H_5$ | $CF_2CF_3$ | |
| 1.516 | 2-F | 5-F | H | $C_3H_7(i)$ | $CF_2CF_3$ | Fp. 150-151°C |
| 1.517 | 2-F | 5-F | H | $C_3H_7(n)$ | $CF_2CF_3$ | |
| 1.518 | 2-F | 5-F | H | $CH_3$ | $CCl_2CF_3$ | |
| 1.519 | 2-F | 5-F | H | $CH_3$ | $CHCl_2$ | |
| 1.520 | 2-F | 5-F | H | $C_2H_5$ | $CHCl_2$ | Fp. 144-145°C |
| 1.521 | 2-F | 5-F | H | $CH_3$ | $CCl_2CH_3$ | |
| 1.522 | 2-F | 5-F | H | $OCH_3$ | $CF_3$ | |
| 1.523 | 2-F | 5-F | H | $OC_3H_7(i)$ | $CF_3$ | |
| 1.524 | 2-F | 5-F | H | $SCH_3$ | $CF_3$ | |
| 1.525 | 2-F | 5-F | H | $SC_3H_7(i)$ | $CF_3$ | |
| 1.526 | 2-F | 6-F | H | $CH_3$ | Cl | |
| 1.527 | 2-F | 6-F | H | $CH_3$ | $CF_3$ | Fp. 177-178°C |
| 1.528 | 2-F | 6-F | H | $C_2H_5$ | $CF_3$ | Fp. 151-153°C |
| 1.529 | 2-F | 6-F | H | $C_3H_7(n)$ | $CF_3$ | |
| 1.530 | 2-F | 6-F | H | $C_3H_7(i)$ | $CF_3$ | Fp. 158-159°C |
| 1.531 | 2-F | 6-F | H | $CH_3$ | $CF_2CF_3$ | |
| 1.532 | 2-F | 6-F | H | $C_2H_5$ | $CF_2CF_3$ | Fp. 137-139°C |
| 1.533 | 2-F | 6-F | H | $CH_3$ | $CF_2Cl$ | Fp. 178-179°C |
| 1.534 | 2-F | 6-F | H | $CH_3$ | $CHF_2$ | |
| 1.535 | 2-F | 6-F | H | $CH_3$ | CHClF | |
| 1.536 | 2-F | 6-F | H | $OCH_3$ | $CF_3$ | |
| 1.537 | 2-F | 6-F | H | $SCH_3$ | $CF_3$ | |
| 1.538 | 2-F | 6-Cl | H | $CH_3$ | Cl | |
| 1.539 | 2-F | 6-Cl | H | $CH_3$ | $CH_3$ | |
| 1.540 | 2-F | 6-Cl | H | $CH_3$ | $CF_3$ | Fp. 184-185°C |
| 1.541 | 2-F | 6-Cl | H | $C_2H_5$ | $CF_3$ | |
| 1.542 | 2-F | 6-Cl | H | $C_3H_7(i)$ | $CF_3$ | Fp. 146-147°C |
| 1.543 | 2-F | 6-Cl | H | Cyclo-propyl | $CF_3$ | Fp. 172-173°C |
| 1.544 | 2-F | 6-Cl | H | $CH_3$ | $CF_2CF_3$ | |
| 1.545 | 2-F | 6-Cl | H | $C_3H_7(i)$ | $CF_2CF_3$ | |
| 1.546 | 2-F | 6-Cl | H | $CH_3$ | $CCl_2CF_3$ | |
| 1.547 | 2-F | 6-Cl | H | $CH_3$ | $CHCl_2$ | |
| 1.548 | 2-F | 6-Cl | H | $CH_3$ | $CHF_2$ | |
| 1.549 | 2-F | 6-Cl | H | $CH_3$ | CHFCl | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.550 | 2-F | 6-Cl | H | $CH_3$ | $CF_2Cl$ | |
| 1.551 | 2-F | 6-Cl | H | $C_3H_7(i)$ | $CF_2Cl$ | |
| 1.552 | 2-F | 6-Cl | H | $OCH_3$ | $CF_3$ | |
| 1.553 | 2-CN | H | H | $CH_3$ | $CF_3$ | Fp. 142-143°C |
| 1.554 | 2-CN | H | H | $C_2H_5$ | $CF_3$ | |
| 1.555 | 2-CN | H | H | $C_3H_7(i)$ | $CF_3$ | |
| 1.556 | 2-CN | H | H | $CH_3$ | $CF_2CF_3$ | |
| 1.557 | 2-CN | H | H | $C_2H_5$ | $CF_2CF_3$ | |
| 1.558 | 2-CN | H | H | $C_3H_7(i)$ | $CF_2CF_3$ | |
| 1.559 | 2-CN | H | H | $CH_3$ | $CHF_2$ | |
| 1.560 | 2-CN | H | H | $CH_3$ | $CF_2Cl$ | |
| 1.561 | 2-CN | H | H | $OCH_3$ | $CF_3$ | |
| 1.562 | 2-CN | H | H | $OC_2H_5$ | $CF_3$ | |
| 1.563 | $2-PO(OC_2H_5)_2$ | H | H | $CH_3$ | $CF_3$ | Fp. 144-145°C |
| 1.564 | $2-PO(OC_2H_5)_2$ | H | H | $C_2H_5$ | $CF_3$ | |
| 1.565 | $2-PO(OC_2H_5)_2$ | H | H | $C_3H_7(i)$ | $CF_3$ | |
| 1.566 | 2-Cl | 5-Cl | H | $SOCH_3$ | $CF_3$ | |
| 1.567 | 2-Cl | 5-Cl | H | $SO_2CH_3$ | $CF_3$ | |
| 1.568 | 2-Cl | 6-Cl | H | $SOCH_3$ | $CF_3$ | |
| 1.569 | 2-Cl | 6-Cl | H | $SO_2CH_3$ | $CF_3$ | |
| 1.570 | 2-Cl | 6-Cl | H | $SOCH_3$ | $CH_3$ | |
| 1.571 | 2-Cl | 6-Cl | H | $SO_2CH_3$ | $CH_3$ | |
| 1.572 | 2-Cl | 6-Cl | H | CN | $CH_3$ | |
| 1.573 | 2-Cl | 6-Cl | H | CN | $CF_3$ | |
| 1.574 | 2-Cl | 6-Cl | H | $CH=CCl_2$ | Cl | |
| 1.575 | 2-Cl | 5-Cl | H | $CH=CCl_2$ | Cl | |
| 1.576 | $2-CF_3$ | H | H | $CH=CCl_2$ | Cl | |
| 1.577 | 2-Cl | 6-Cl | H | $CH_2OCH_3$ | $CF_3$ | |
| 1.578 | 2-Cl | 6-Cl | H | $CH_2OCH_3$ | $C_2F_5$ | |
| 1.579 | 2-Cl | 5-Cl | H | $CH_2OCH_3$ | $CF_3$ | |
| 1.580 | 2-Cl | 5-Cl | H | $CH_2OCH_3$ | $CF_2Cl$ | |
| 1.581 | $2-OCH_3$ | 5-Cl | H | $CH_2OCH_3$ | $CF_3$ | |
| 1.582 | $2-OCH_3$ | 5-Cl | H | $CH_2OCH_3$ | $CF_2CF_3$ | |
| 1.583 | 2-F | 5-F | H | $CH_2OCH_3$ | $CF_3$ | |
| 1.584 | $2-OCHF_2$ | 5-Cl | H | $CH_2OCH_3$ | $CF_3$ | |
| 1.585 | $2-OCHF_2$ | 5-Cl | H | $CH_2OCH_3$ | $CF_2Cl$ | |
| 1.586 | 3-Br | H | H | $CH_3$ | Cl | Fp. 133-134°C |
| 1.587 | 3-Br | H | H | $CH_3$ | $CF_3$ | |
| 1.588 | 2-Br | 5-Br | H | $CH_3$ | Cl | |
| 1.589 | 2-Br | 5-Br | H | $CH_3$ | $CF_3$ | Fp. 155-156°C |
| 1.590 | 2-Br | 5-Br | H | $OCH_3$ | $CF_3$ | |
| 1.591 | 2-Br | 5-Br | H | $C_2H_5$ | $CF_3$ | |
| 1.592 | 2-Br | 5-Br | H | $CH_3$ | $CF_2Cl$ | |
| 1.593 | 2-Br | 5-Br | H | $CH_3$ | $CHF_2$ | |
| 1.594 | $2-CF_3$ | 5-Cl | H | $CH_3$ | $CF_3$ | Fp. 152-153°C |
| 1.595 | $2-CF_3$ | 5-Cl | H | $CH_3$ | Cl | Fp. 142-143°C |
| 1.596 | $2-CF_3$ | 5-Cl | H | $C_2H_5$ | $CF_3$ | |
| 1.597 | $2-CF_3$ | 5-Cl | H | $C_3H_7(i)$ | $CF_3$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.598 | 2-$CF_3$ | 5-Cl | H | Cyclo-propyl | $CF_3$ | |
| 1.599 | 2-$CF_3$ | 5-Cl | H | $CH_3$ | $CH_2Cl$ | |
| 1.600 | 2-$CF_3$ | 5-Cl | H | $CH_3$ | $CHF_2$ | |
| 1.601 | 2-$CF_3$ | 5-Cl | H | $C_2H_5$ | $CF_2Cl$ | |
| 1.602 | 2-$CF_3$ | 5-Cl | H | $C_3H_7$(i) | $CF_2Cl$ | |
| 1.603 | 2-$CF_3$ | 5-Cl | H | $CH_3$ | $CF_2CF_3$ | |
| 1.604 | 2-$CF_3$ | 5-Cl | H | $OCH_3$ | $CF_3$ | |
| 1.605 | 2-$CF_3$ | 5-Cl | H | $SCH_3$ | $CF_3$ | |
| 1.606 | 2-$CF_3$ | 5-Cl | H | $SOCH_3$ | $CF_3$ | |
| 1.607 | 2-$CF_3$ | 5-Cl | H | $SO_2CH_3$ | $CH_3$ | |
| 1.608 | 2-$CH_3$ | 3-$CH_3$ | H | $CH_3$ | Cl | |
| 1.609 | 2-$CH_3$ | 3-$CH_3$ | H | $CH_3$ | $CF_3$ | |
| 1.610 | 2-$CH_3$ | 5-$CH_3$ | H | $CH_3$ | Cl | |
| 1.611 | 2-$CH_3$ | 5-$CH_3$ | H | $CH_3$ | $CF_3$ | |
| 1.612 | 2-$CH_3$ | 6-$CH_3$ | H | $CH_3$ | Cl | |
| 1.613 | 2-$CH_3$ | 6-$CH_3$ | H | $CH_3$ | $CF_3$ | Fp. 160-161°C |
| 1.614 | 2-$CH_3$ | 6-$CH_3$ | H | $CH_3$ | $CF_2Cl$ | Fp. 169-170°C |
| 1.615 | 2-$CH_3$ | 6-$CH_3$ | H | $CH_3$ | $CH_2CF_3$ | |
| 1.616 | 2-$CH_3$ | 6-$CH_3$ | H | $OCH_3$ | $CF_3$ | |
| 1.617 | 2-$CH_3$ | 6-$CH_3$ | H | $SCH_3$ | $CF_3$ | |
| 1.618 | 2-$CH_3$ | 6-$C_2H_5$ | H | $CH_3$ | Cl | |
| 1.619 | 2-$CH_3$ | 6-$C_2H_5$ | H | $CH_3$ | $CF_3$ | Fp. 140-144°C |
| 1.620 | 2-$CH_3$ | 6-$C_2H_5$ | H | $OCH_3$ | $CF_3$ | |
| 1.621 | 2-$CH_3$ | 5-F | H | $CH_3$ | Cl | |
| 1.622 | 2-$CH_3$ | 5-F | H | $CH_3$ | $CF_3$ | Fp. 170-172°C |
| 1.623 | 2-$CH_3$ | 5-F | H | $CH_3$ | $CHF_2$ | |
| 1.624 | 2-$CH_3$ | 5-F | H | $CH_3$ | $CF_2Cl$ | |
| 1.625 | 2-$CH_3$ | 5-F | H | $C_2H_5$ | $CF_3$ | |
| 1.626 | 2-$CH_3$ | 5-F | H | $C_3H_7$(i) | $CF_3$ | |
| 1.627 | 2-$CH_3$ | 5-F | H | $OCH_3$ | $CF_3$ | |
| 1.628 | 2-$CH_3$ | 5-F | H | $OC_2H_5$ | $CF_3$ | |
| 1.629 | 2-Br | 6-Br | H | $CH_3$ | Cl | |
| 1.630 | 2-Br | 6-Br | H | $CH_3$ | $CF_3$ | |
| 1.631 | 2-Br | 6-Br | H | $C_2H_5$ | $CF_3$ | Fp. 157-158°C |
| 1.632 | 2-Br | 6-Br | H | $C_3H_7$(i) | $CF_3$ | |
| 1.633 | 2-Br | 6-Br | H | Cyclo-propyl | $CF_3$ | |
| 1.634 | 2-Br | 6-Br | H | $CH_3$ | $CHCl_2$ | |
| 1.635 | 2-Br | 6-Br | H | $CH_3$ | $CF_2CF_3$ | |
| 1.636 | 2-Br | H | H | $C_3H_7$(i) | $CF_2CF_3$ | Fp. 149-150°C |
| 1.637 | 2-$CF_3$ | H | H | $CH_3$ | $CHCl_2$ | Fp. 161-162°C |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.638 | 2-$OCHF_2$ | H | H | $CH_3$ | $CF_3$ | Fp. 145-146°C |
| 1.639 | 2-$OCHF_2$ | H | H | $C_3H_7$(i) | $CF_3$ | |
| 1.640 | 2-$OCHF_2$ | H | H | Cyclo-propyl | $CF_3$ | Fp. 137-138°C |
| 1.641 | 2-$OCHF_2$ | H | H | $CH_3$ | $CHF_2$ | |
| 1.642 | 2-$OCHF_2$ | H | H | $CH_3$ | CHFCl | |
| 1.643 | 2-$CF_3$ | H | H | $CH_3$ | $CHF_2$ | |
| 1.644 | 2-$CF_3$ | H | H | $CH_3$ | $CF_2Cl$ | |
| 1.645 | 2-$CF_3$ | H | H | $C_3H_7$(i) | $CF_2Cl$ | Smp. 140-141°C |
| 1.646 | 2-$CF_3$ | H | H | Cyclo-propyl | $CF_3$ | Smp. 169-170°C |
| 1.647 | 2-$CF_3$ | H | H | 2-Thienyl | $CF_3$ | Smp. 170-171°C |
| 1.648 | 2-Br | H | H | $C_4H_9$(n) | $CF_3$ | Smp. 144-145°C |
| 1.649 | 2-Br | H | H | $C_3H_7$(i) | $CF_3$ | Smp. 169-170°C |
| 1.650 | 2-Br | H | H | $CH_3$ | $CHF_2$ | |
| 1.651 | 2-Br | H | H | $CH_3$ | $CF_2Cl$ | |
| 1.652 | 2-Br | H | H | Cyclo-propyl | $CF_2Cl$ | |
| 1.653 | 2-Br | H | H | $C_2H_5$ | $CHF_2$ | |
| 1.654 | 2-Cl | 6-$CH_3$ | H | $CH_3$ | $CH_3$ | Smp. 165-167°C |
| 1.655 | 2-Cl | 6-Cl | H | $CH_3$ | $CHF_2$ | Smp. 182-183°C |
| 1.656 | 2-Cl | 6-Cl | H | $C_2H_5$ | $CHF_2$ | |
| 1.657 | 2-Cl | 5-Cl | H | $C_5H_{11}$(n) | $CF_3$ | Smp. 118-119°C |
| 1.658 | 2-$COOCH_3$ | H | H | $CH_3$ | $CF_3$ | Smp. 138-139°C |
| 1.659 | 2-$COOCH_3$ | H | H | $C_2H_5$ | $CF_3$ | |
| 1.660 | 2-$COOCH_3$ | H | H | $C_3H_7$(i) | $CF_3$ | |
| 1.661 | 2-$COOCH_3$ | H | H | Cyclo-propyl | $CF_3$ | |
| 1.662 | 2-$COOCH_3$ | H | H | $CH_3$ | $CF_2Cl$ | |
| 1.663 | 2-$COOCH_3$ | H | H | $CH_3$ | $CHF_2$ | |
| 1.664 | 2-$COOC_2H_5$ | H | H | $CH_3$ | $CF_3$ | |
| 1.665 | 2-$COOC_3H_7$(n) | H | H | $CH_3$ | $CF_3$ | |
| 1.666 | 2-$COOC_3H_7$(i) | H | H | $CH_3$ | $CF_3$ | |
| 1.667 | 2-$COOC_4H_9$(n) | H | H | $CH_3$ | $CF_3$ | |
| 1.668 | 2-$OCH_3$ | 5-F | H | $CH_3$ | $CF_3$ | Smp. 141-142°C |
| 1.669 | 2-$OCH_3$ | 5-F | H | $CH_3$ | $CHF_2$ | |
| 1.670 | 2-$OCH_3$ | 5-F | H | $C_2H_5$ | $CHF_2$ | |
| 1.671 | 2-$OCH_3$ | 5-F | H | $C_3H_7$(i) | $CF_3$ | |
| 1.672 | 2-$OCH_3$ | 5-F | H | Cyclo-propyl | $CF_3$ | |
| 1.673 | 2-$OCH_3$ | 5-F | H | Cyclo-propyl | $CF_3Cl$ | |

Tabelle 1 (Fortsetzung)

| Verb.Nr. | $R^1$ | $R^2$ | $R^3$ | $R^4$ | $R^5$ | physikalische Daten |
|---|---|---|---|---|---|---|
| 1.674 | 2-$OCHF_2$ | 5-F | H | $CH_3$ | $CF_3$ | |
| 1.675 | 2-$OCHF_2$ | 5-F | H | $CH_3$ | $CHF_2$ | |
| 1.676 | 2-$OCHF_2$ | 5-F | H | $CH_3$ | $CF_2Cl$ | |
| 1.677 | 2-$COOCH_3$ | 6-$CH_3$ | H | $CH_3$ | $CF_3$ | Fp. 161-162°C |
| 1.678 | 2-$COOCH_3$ | 6-$CH_3$ | H | $CH_3$ | $CHF_2$ | |
| 1.679 | 2-$COOCH_3$ | 6-$CH_3$ | H | $C_3H_7$(i) | $CF_3$ | |
| 1.680 | 2-$COOCH_3$ | 6-$CH_3$ | H | Cyclo-propyl | $CF_3$ | |
| 1.681 | 2-$COOCH_3$ | 6-Cl | H | $CH_3$ | $CF_3$ | Fp. 144-145°C |
| 1.682 | 2-$COOCH_3$ | 6-Cl | H | $CH_3$ | $CHF_2$ | |
| 1.683 | 2-Br | H | H | 2-Furyl | $CF_3$ | Smp. 176-177°C |
| 1.684 | 2-Cl | 6-Cl | 3-$CH_3$ | $CH_3$ | Cl | |
| 1.685 | 2-$CF_3$ | H | H | $C_2H_5$ | $CHCl_2$ | Fp. 155-156°C |
| 1.686 | 2-Cl | 5-Cl | H | Cl | $CF_3$ | |
| 1.687 | 2-$CONH_2$ | H | H | $CH_3$ | $CF_3$ | |
| 1.688 | 2-Cl | 6-Cl | H | $CH_3$<br>$CHCH_2CH_3$ | $CF_3$ | Fp. 124-125°C |
| 1.689 | 2-F | H | H | $C_2H_5$ | $CF_3$ | Fp. 136-137°C |
| 1.690 | 2-F | H | H | $CH_2OCH_3$ | $CF_3$ | Fp. 122-124°C |
| 1.691 | 2-F | H | H | $CH_2OCH_3$ | $CF_2Cl$ | Fp. 145-147°C |
| 1.692 | 2-$OCH_3$ | 6-Cl | H | $CH_3$ | $CF_3$ | Fp. 191-192°C |
| 1.693 | 2-$OCH_3$ | 6-Cl | H | $C_2H_5$ | $CF_3$ | Fp. 147-149°C |
| 1.694 | 2-$OCH_3$ | 6-Cl | H | $C_3H_7$(i) | $CF_3$ | Fp. 143-146°C |
| 1.695 | 2-$CH_3$ | 6-$CH_3$ | H | $C_2H_5$ | $CF_3$ | Fp. 158-160°C |
| 1.696 | 2-$CH_3$ | 6-$CH_3$ | H | $C_3H_7$(i) | $CF_3$ | Fp. 143-146°C |
| 1.697 | 2-F | 6-F | H | $C_4H_9$(i) | $CF_3$ | Fp. 135-137°C |
| 1.698 | 2-F | 6-F | H | 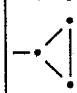 | $CF_3$ | Fp. 158-159°C |
| 1.699 | 2-F | 6-F | H | $CH_2OCH_3$ | $CF_3$ | Fp. 138-139°C |
| 1.700 | 2-F | 6-F | H | $C_2H_5$ | $CHCl_2$ | Fp. 169-171°C |
| 1.701 | 2-F | 6-F | H | 2-Furyl | $CF_3$ | Fp. 152-153°C |
| 1.702 | 2-$CH_3$ | 5-F | H | $C_2H_5$ | $CHCl_2$ | |
| 1.703 | 2-Cl | 6-$CH_3$ | H | $CH_2OCH_3$ | $CF_3$ | Fp. 137-138°C |
| 1.704 | 2-Cl | 6-$CH_3$ | H | $CH_2OCH_3$ | $CF_2Cl$ | Fp. 133-134°C |
| 1.705 | 2-Cl | 6-$CH_3$ | H | $C_3H_7$(i) | $CF_2Cl$ | Fp. 142-144°C |
| 1.706 | 2-Cl | 6-$CH_3$ | H | Cl | $CF_3$ | |
| 1.707 | 2-Cl | 6-$CH_3$ | H | $CH_2OCH_3$ | $CHF_2$ | Fp. 142-144°C |
| 1.708 | 2-F | H | H | $CH_2OCH_3$ | $CHF_2$ | Fp. 116-118°C |
| 1.709 | 2-$OCH_3$ | 6-$CH_3$ | H | $CH_3$ | $CF_3$ | Fp. 180-181°C |
| 1.710 | 2-Cl | 6-Cl | H | Cl | $CF_3$ | Fp. 189-190°C |
| 1.711 | 2-$SCHF_2$ | H | H | $CH_3$ | $CF_3$ | |
| 1.712 | 2-$OCHF_2$ | 6-$CH_3$ | H | $CH_3$ | $CF_3$ | |
| 1.713 | 2-$OCHF_2$ | 6-$CH_3$ | H | $C_2H_5$ | $CF_3$ | |
| 1.714 | 2-F | 6-Cl | H | 2-Furyl | $CF_3$ | |
| 1.715 | 2-$CH_3$ | 6-F | H | $C_2H_5$ | $CHCl_2$ | Fp. 141-142°C |

H. 2. Verbindungen der Formel II

H. 2.1. N-(4-Methyl-6-trifluormethyl-pyrimidin-2-yl)-2-trifluormethylanilin

Zu einer Lösung von 12 g (0.1 Mol) Kalium-tert.Butylat in 100 ml Tetrahydrofuran wird eine Suspension von 24 g (0.1 Mol) 2-Trifluormethylphenyl-guanidiniumhydrochlorid in 80 ml Tetrahydrofuran gegeben. Nach dem Abklingen der exothermen Reaktion werden 15,4 g (0.1 Mol) α,α,α-Trifluoracetylaceton in 20 ml Tetrahydrofuran zugegeben. Das Reaktionsgemisch wird 2 Stunden bei 60°C gerührt, dann am Rotationsverdampfer eingedampft. Der Rückstand wird an Kieselgel mit einem Gemisch von Essigsäureäthylester und Hexan (Verhältnis 1:2) chromatographiert. Man erhält 18,7 g (58.2 % d.Th.) der Titelverbindung der Formel

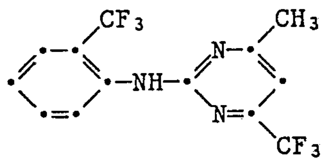

als Kristalle vom Smp. 65-66°C (Verb. No. 2.020).

Analog zu Beispiel H 2 sind die Verbindungen der Tabelle II erhältlich:

Tabelle II

Verbindungen der Formel

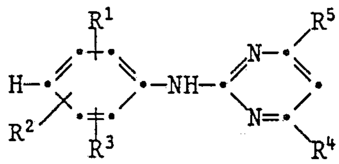

II

| Verb.Nr. | $R^1$ | $R^2$ | $R^3$ | $R^4$ | $R^5$ | physikalische Daten |
|---|---|---|---|---|---|---|
| 2.001 | 2-Cl | H | H | $CH_3$ | $CH_3$ | |
| 2.002 | 2-Cl | H | H | $CH_3$ | $OCHF_2$ | |
| 2.003 | 2-Cl | H | H | $CH_3$ | Cl | |
| 2.004 | 2-Cl | H | H | $CH_3$ | $CF_3$ | |
| 2.005 | 2-Cl | H | H | $CH_3$ | $CF_2CF_3$ | |
| 2.006 | 2-Cl | H | H | $CH_2CH_3$ | $CF_3$ | |
| 2.007 | 2-Cl | H | H | $CH_3$ | $CF_2Cl$ | |
| 2.008 | 2-Cl | H | H | $CH_3$ | $CHF_2$ | |
| 2.009 | 2-Br | H | H | $CH_3$ | Cl | Fp. 109-110°C |
| 2.010 | 2-Br | H | H | $OCH_3$ | $CF_3$ | |
| 2.011 | 2-Br | H | H | $CH_3$ | $CF_3$ | |
| 2.012 | 2-Br | H | H | $SCH_3$ | $CF_3$ | |
| 2.013 | 2-Br | H | H | $CH_2CH_3$ | $CF_3$ | |
| 2.014 | 2-Br | H | H | $CH_3$ | $CHCl_2$ | |
| 2.015 | 2-Br | H | H | $CH_3$ | CHFCl | |
| 2.016 | 2-Br | H | H | Cyclo-propyl | $CF_3$ | Fp. 85-86°C |
| 2.017 | 2-$CF_3$ | H | H | Phenyl | $CF_3$ | |
| 2.018 | 2-$CF_3$ | H | H | 2-Furyl | $CF_3$ | |
| 2.019 | 2-$CF_3$ | H | H | $CH_3$ | Cl | |
| 2.020 | 2-$CF_3$ | H | H | $CH_3$ | $CF_3$ | Fp. 65-66°C |
| 2.021 | 2-$CF_3$ | H | H | $CH_3$ | $CH_3$ | Fp. 70-72°C |
| 2.022 | 2-$CF_3$ | H | H | $CH_2CH_3$ | $CF_3$ | |
| 2.023 | 2-$CF_3$ | H | H | $CH(CH_3)_2$ | $CF_3$ | $n_D^{25}$: 1,4960 |
| 2.024 | 2-$CF_3$ | H | H | (n)$C_3H_7$ | $CF_3$ | $n_D^{25}$: 1,4970 |
| 2.025 | 2-$CF_3$ | H | H | $OCH_3$ | $CF_3$ | |
| 2.026 | 2-$CF_3$ | H | H | $OC_2H_5$ | $CF_3$ | |
| 2.027 | 2-$CF_3$ | H | H | $SCH_3$ | $CF_3$ | |
| 2.028 | 2-$CF_3$ | H | H | $SCH(CH_3)_2$ | $CF_3$ | |
| 2.029 | 2-$CF_3$ | H | H | $CH_3$ | $CF_2CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.030 | 2-$CF_3$ | H | H | $CH_3$ | CHFCl | |
| 2.031 | 2-J | H | H | $CH_3$ | Cl | |
| 2.032 | 2-J | H | H | $CH_3$ | $CF_3$ | Fp. 109-110°C |
| 2.033 | 2-J | H | H | $CH_2CH_3$ | $CF_3$ | |
| 2.034 | 2-J | H | H | $CH_3$ | $CF_2Cl$ | |
| 2.035 | 2-F | H | H | $CH_3$ | $CH_3$ | |
| 2.036 | 2-F | H | H | $CH_3$ | Cl | |
| 2.037 | 2-F | H | H | $CH_3$ | $CF_3$ | Fp. 64-66°C |
| 2.038 | 2-F | H | H | $CH(CH_3)_2$ | $CF_3$ | $n_D^{25}$: 1.5240 |
| 2.039 | 2-F | H | H | Cl | $CF_3$ | |
| 2.040 | 2-F | H | H | $OC_2H_5$ | $CF_3$ | |
| 2.041 | 2-Cl | 3-Cl | H | $CH_3$ | Cl | Fp. 123-124°C |
| 2.042 | 2-Cl | 3-Cl | H | $CH_3$ | $CF_3$ | Fp. 109-110°C |
| 2.043 | 2-Cl | 3-Cl | H | $OCH_3$ | $CF_3$ | |
| 2.044 | 2-Cl | 3-Cl | H | $OC_4H_9(n)$ | $CF_3$ | |
| 2.045 | 2-Cl | 3-Cl | H | $SC_2H_5$ | $CF_3$ | |
| 2.046 | 2-Cl | 3-Cl | H | Phenyl | $CF_3$ | |
| 2.047 | 2-Cl | 3-Cl | H | $CH_3$ | $CF_2Cl$ | |
| 2.048 | 2-Cl | 3-Cl | H | $CH_3$ | CHFCl | |
| 2.049 | 2-Cl | 3-Cl | H | $CH_3$ | $CH_3$ | |
| 2.050 | 2-Cl | 5-Cl | H | $CH_3$ | $CH_3$ | |
| 2.051 | 2-Cl | 5-Cl | H | $CH_3$ | $OCHF_2$ | |
| 2.052 | 2-Cl | 5-Cl | H | $CH_3$ | Cl | Fp. 99-100°C |
| 2.053 | 2-Cl | 5-Cl | H | $OCH_3$ | $CH_3$ | |
| 2.054 | 2-Cl | 5-Cl | H | $CH_3$ | Br | |
| 2.055 | 2-Cl | 5-Cl | H | $CH_3$ | $CF_3$ | Fp. 79-80°C |
| 2.056 | 2-Cl | 5-Cl | H | $CH_2CH_3$ | $CF_3$ | Fp. 62-63°C |
| 2.057 | 2-Cl | 5-Cl | H | $C_3H_7(n)$ | $CF_3$ | $n_D^{25}$: 1,5490 |
| 2.058 | 2-Cl | 5-Cl | H | $C_3H_7(i)$ | $CF_3$ | Fp. 60-61°C |
| 2.059 | 2-Cl | 5-Cl | H | Cyclo-propyl | $CF_3$ | Fp. 106-107°C |
| 2.060 | 2-Cl | 5-Cl | H | Phenyl | $CF_3$ | |
| 2.061 | 2-Cl | 5-Cl | H | 2-Thienyl | $CF_3$ | Fp. 112-113°C |
| 2.062 | 2-Cl | 5-Cl | H | 2-Furyl | $CF_3$ | Fp. 102-103°C |
| 2.063 | 2-Cl | 5-Cl | H | $CH_3$ | $CF_2CF_3$ | Fp. 99-100°C |
| 2.064 | 2-Cl | 5-Cl | H | $CH_3$ | $CF_2Cl$ | Fp. 67°C |
| 2.065 | 2-Cl | 5-Cl | H | $CH_3$ | $CHF_2$ | Fp. 72-74°C |
| 2.066 | 2-Cl | 5-Cl | H | $CH_3$ | CHClF | Fp. 66-67°C |
| 2.067 | 2-Cl | 5-Cl | H | $CH_2CH_3$ | $CF_2Cl$ | |
| 2.068 | 2-Cl | 5-Cl | H | $C_3H_7(i)$ | $CF_2Cl$ | $n_D^{25}$: 1,5682 |
| 2.069 | 2-Cl | 5-Cl | H | $OCH_3$ | $CF_3$ | |
| 2.070 | 2-Cl | 5-Cl | H | $OC_2H_5$ | $CF_3$ | |
| 2.071 | 2-Cl | 5-Cl | H | $OC_3H_7$ | $CF_3$ | |
| 2.072 | 2-Cl | 5-Cl | H | $OC_3H_7(i)$ | $CF_3$ | |
| 2.073 | 2-Cl | 5-Cl | H | $OC_4H_9(n)$ | $CF_3$ | |
| 2.074 | 2-Cl | 5-Cl | H | $OC_4H_9(i)$ | $CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.075 | 2-Cl | 5-Cl | H | $SCH_3$ | $CF_3$ | |
| 2.076 | 2-Cl | 5-Cl | H | $SC_2H_5$ | $CF_3$ | |
| 2.077 | 2-Cl | 5-Cl | H | $SC_3H_7$(n) | $CF_3$ | |
| 2.078 | 2-Cl | 5-Cl | H | $SC_3H_7$(i) | $CF_3$ | |
| 2.079 | 2-Cl | 5-Cl | H | $SC_4H_9$(n) | $CF_3$ | |
| 2.080 | 2-Cl | 5-Cl | H | $C_4H_9$(n) | $CF_3$ | $n_D^{25}$: 1,5475 |
| 2.081 | 2-Cl | 5-Cl | H | $C_4H_9$(i) | $CF_3$ | |
| 2.082 | 2-Cl | 5-Cl | H | $C_4H_9$(t) | $CF_3$ | Fp. 106-107°C |
| 2.083 | 2-Cl | 5-Cl | H | $C_2H_5$ | $CF_2CF_3$ | Fp. 70-72°C |
| 2.084 | 2-Cl | 5-Cl | H | $C_3H_7$(i) | $CF_2CF_3$ | Fp. 39-41°C |
| 2.085 | 2-Cl | 5-Cl | H | $C_3H_7$(n) | $CF_2CF_3$ | |
| 2.086 | 2-Cl | 5-Cl | H | $OCF_2CHF_2$ | $CF_3$ | |
| 2.087 | 2-Cl | 5-Cl | H | $CH_3$ | $CCl_2CF_3$ | |
| 2.088 | 2-Cl | 5-Cl | H | $C_2H_5$ | $CCl_2CF_3$ | |
| 2.089 | 2-Cl | 5-Cl | H | $CH_3$ | $CHCl_2$ | Fp. 80-82°C |
| 2.090 | 2-Cl | 5-Cl | H | $C_2H_5$ | $CHCl_2$ | $n_D^{25}$: 1,6115 |
| 2.091 | 2-Cl | 5-Cl | H | $CH_3$ | $CCl_2CH_3$ | |
| 2.092 | 2-Cl | 5-Cl | H | $C_2H_5$ | $CCl_2CH_3$ | |
| 2.093 | 2-Cl | 6-Cl | H | $CH_3$ | $CH_3$ | Fp. 185-186°C |
| 2.094 | 2-Cl | 6-Cl | H | $CH_3$ | $C_2H_5$ | |
| 2.095 | 2-Cl | 6-Cl | H | $CH_3$ | $C_3H_7$(i) | |
| 2.096 | 2-Cl | 6-Cl | H | $CH_3$ | Cl | Fp. 190-191°C |
| 2.097 | 2-Cl | 6-Cl | H | $CH_3$ | $CCl_2CH_3$ | |
| 2.098 | 2-Cl | 6-Cl | H | $C_2H_5$ | $CCl_2CH_3$ | |
| 2.099 | 2-Cl | 6-Cl | H | $CH_3$ | $CHCl_2$ | |
| 2.100 | 2-Cl | 6-Cl | H | $C_2H_5$ | $CHCl_2$ | |
| 2.101 | 2-Cl | 6-Cl | H | $CH_3$ | $CCl_2CF_3$ | |
| 2.102 | 2-Cl | 6-Cl | H | $C_3H_7$(i) | $CCl_2CF_3$ | |
| 2.103 | 2-Cl | 6-Cl | H | $CH_3$ | CHClF | |
| 2.104 | 2-Cl | 6-Cl | H | $C_2H_5$ | CHClF | |
| 2.105 | 2-Cl | 6-Cl | H | $CH_3$ | $CF_2Cl$ | |
| 2.106 | 2-Cl | 6-Cl | H | $C_2H_5$ | $CF_2Cl$ | |
| 2.107 | 2-Cl | 6-Cl | H | $C_3H_7$(i) | $CF_2Cl$ | |
| 2.108 | 2-Cl | 6-Cl | H | $C_3H_7$(n) | $CF_2Cl$ | |
| 2.109 | 2-Cl | 6-Cl | H | $CH_3$ | $CF_3$ | Fp. 138-139°C |
| 2.110 | 2-Cl | 6-Cl | H | $C_2H_5$ | $CF_3$ | |
| 2.111 | 2-Cl | 6-Cl | H | $C_3H_7$(n) | $CF_3$ | |
| 2.112 | 2-Cl | 6-Cl | H | $C_3H_7$(i) | $CF_3$ | |
| 2.113 | 2-Cl | 6-Cl | H | Cyclo-propyl | $CF_3$ | Fp. 70-73°C |
| 2.114 | 2-Cl | 6-Cl | H | Phenyl | $CF_3$ | |
| 2.115 | 2-Cl | 6-Cl | H | 2-Thienyl | $CF_3$ | Fp. 119-120°C |
| 2.116 | 2-Cl | 6-Cl | H | 2-Furyl | $CF_3$ | |
| 2.117 | 2-Cl | 6-Cl | H | $CH_3$ | $CF_2CF_3$ | |
| 2.118 | 2-Cl | 6-Cl | H | $C_2H_5$ | $CF_2CF_3$ | Fp. 96-98°C |
| 2.119 | 2-Cl | 6-Cl | H | $C_3H_7$(i) | $CF_2CF_3$ | Fp. 88-90°C |
| 2.120 | 2-Cl | 6-Cl | H | $C_3H_7$(n) | $CF_2CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.121 | 2-Cl | 6-Cl | H | Cyclo-propyl | $CF_2CF_3$ | |
| 2.122 | 2-Cl | 6-Cl | H | $CH_3$ | $OCHF_2$ | |
| 2.123 | 2-Cl | 6-Cl | H | $C_4H_9(n)$ | $CF_3$ | |
| 2.124 | 2-Cl | 6-Cl | H | $C_4H_9(i)$ | $CF_3$ | |
| 2.125 | 2-Cl | 6-Cl | H | $C_4H_9(t)$ | $CF_3$ | Fp. 79-81°C |
| 2.126 | 2-Cl | 6-Cl | H | $OCH_3$ | $CF_3$ | |
| 2.127 | 2-Cl | 6-Cl | H | $OC_2H_5$ | $CF_3$ | |
| 2.128 | 2-Cl | 6-Cl | H | $OC_3H_7(n)$ | $CF_3$ | |
| 2.129 | 2-Cl | 6-Cl | H | $OC_3H_7(i)$ | $CF_3$ | |
| 2.130 | 2-Cl | 6-Cl | H | $OC_4H_9(n)$ | $CF_3$ | |
| 2.131 | 2-Cl | 6-Cl | H | $OC_4H_9(i)$ | $CF_3$ | |
| 2.132 | 2-Cl | 6-Cl | H | $SCH_3$ | $CF_3$ | Fp. 70-71°C |
| 2.133 | 2-Cl | 6-Cl | H | $SC_2H_5$ | $CF_3$ | |
| 2.134 | 2-Cl | 6-Cl | H | $SC_3H_7(n)$ | $CF_3$ | |
| 2.135 | 2-Cl | 6-Cl | H | $SC_3H_7(i)$ | $CF_3$ | |
| 2.136 | 2-Cl | 6-Cl | H | $SC_4H_9(n)$ | $CF_3$ | |
| 2.137 | 2-Cl | 6-Cl | H | $OCH_3$ | $CH_3$ | |
| 2.138 | 2-Cl | 6-Cl | H | $C_2H_5$ | Cl | |
| 2.139 | 2-Cl | 6-Cl | H | $OCH_3$ | $C_2H_5$ | |
| 2.140 | 2-Cl | 6-Cl | H | $C_2H_5$ | $OCHF_2$ | |
| 2.141 | 2-Cl | 6-Cl | $3-CH_3$ | $CH_3$ | $CF_3$ | Fp. 124-125°C |
| 2.142 | 2-Cl | 6-Cl | $3-CH_3$ | $C_2H_5$ | $CF_3$ | Fp. 90-91°C |
| 2.143 | 2-Cl | 6-Cl | $3-CH_3$ | $C_3H_7(n)$ | $CF_3$ | |
| 2.144 | 2-Cl | 6-Cl | $3-CH_3$ | $C_3H_7(i)$ | $CF_3$ | Fp. 74-75°C |
| 2.145 | 2-Cl | 6-Cl | $3-CH_3$ | $CH_3$ | $CF_2CF_3$ | Fp. 119-121°C |
| 2.146 | 2-Cl | 6-Cl | $3-CH_3$ | $C_2H_5$ | $CF_2CF_3$ | |
| 2.147 | 2-Cl | 6-Cl | $3-CH_3$ | Cl | $CF_3$ | |
| 2.148 | 2-Cl | 6-Cl | $3-CH_3$ | $OCH_3$ | $CF_3$ | |
| 2.149 | 2-Cl | 6-Cl | $3-CH_3$ | $OC_2H_5$ | $CF_3$ | |
| 2.150 | 2-Cl | 6-Cl | $3-CH_3$ | $SCH_3$ | $CF_3$ | |
| 2.151 | 2-Cl | 6-Cl | $3-CH_3$ | $SC_4H_9(n)$ | $CF_3$ | |
| 2.152 | 2-Cl | 6-Cl | $3-CH_3$ | $CH_3$ | $CF_2Cl$ | |
| 2.153 | 2-Cl | 6-Cl | $3-CH_3$ | $CH_3$ | $CHF_2$ | |
| 2.154 | 2-Cl | 6-Cl | $3-CH_3$ | $CH_3$ | $CHCl_2$ | |
| 2.155 | 2-Cl | $3-CF_3$ | H | $CH_3$ | Cl | |
| 2.156 | 2-Cl | $3-CF_3$ | H | $CH_3$ | $CH_3$ | |
| 2.157 | 2-Cl | $3-CF_3$ | H | $CH_3$ | $CF_3$ | |
| 2.158 | 2-Cl | $3-CF_3$ | H | $C_2H_5$ | $CF_3$ | |
| 2.159 | 2-Cl | $3-CF_3$ | H | $C_3H_7(n)$ | $CF_3$ | |
| 2.160 | 2-Cl | $3-CF_3$ | H | $CH_3$ | $CF_2CF_3$ | |
| 2.161 | 2-Cl | $3-CF_3$ | H | $CH_3$ | $CClF_2$ | |
| 2.162 | 2-Cl | $3-CF_3$ | H | $CH_3$ | CHClF | |
| 2.163 | 2-Cl | $5-CF_3$ | H | $CH_3$ | Cl | Fp. 101-103°C |
| 2.164 | 2-Cl | $5-CF_3$ | H | $CH_3$ | $CF_3$ | Fp. 84-85°C |
| 2.165 | 2-Cl | $5-CF_3$ | H | $CH_3$ | $CClF_2$ | |
| 2.166 | 2-Cl | $5-CF_3$ | H | $C_2H_5$ | $CF_3$ | |
| 2.167 | 2-Cl | $5-CF_3$ | H | $OCH_3$ | $CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.168 | 2-Cl | 6-$CF_3$ | H | $CH_3$ | Cl | |
| 2.169 | 2-Cl | 6-$CF_3$ | H | $CH_3$ | $CH_3$ | |
| 2.170 | 2-Cl | 6-$CF_3$ | H | $CH_3$ | $CF_3$ | Fp. 117-119°C |
| 2.171 | 2-Cl | 6-$CF_3$ | H | $C_2H_5$ | $CF_3$ | |
| 2.172 | 2-Cl | 6-$CF_3$ | H | $C_3H_7$(n) | $CF_3$ | |
| 2.173 | 2-Cl | 6-$CF_3$ | H | $C_3H_7$(i) | $CF_3$ | |
| 2.174 | 2-Cl | 6-$CF_3$ | H | Cyclo-propyl | $CF_3$ | |
| 2.175 | 2-Cl | 6-$CF_3$ | H | $C_4H_9$(n) | $CF_3$ | |
| 2.176 | 2-Cl | 6-$CF_3$ | H | $C_4H_9$(i) | $CF_3$ | |
| 2.177 | 2-Cl | 6-$CF_3$ | H | $C_4H_9$(t) | $CF_3$ | |
| 2.178 | 2-Cl | 6-$CF_3$ | H | $CH_3$ | $CF_2CF_3$ | |
| 2.179 | 2-Cl | 6-$CF_3$ | H | $C_2H_5$ | $CF_2CF_3$ | |
| 2.180 | 2-Cl | 6-$CF_3$ | H | $CH_3$ | $CHCl_2$ | |
| 2.181 | 2-Cl | 6-$CF_3$ | H | $CH_3$ | $CClF_2$ | |
| 2.182 | 2-Cl | 6-$CF_3$ | H | $C_2H_5$ | $CClF_2$ | |
| 2.183 | 2-Cl | 6-$CF_3$ | H | $CH_3$ | $CHF_2$ | |
| 2.184 | 2-Cl | 6-$CF_3$ | H | $CH_3$ | $OCHF_2$ | |
| 2.185 | 2-Cl | 6-$CF_3$ | H | $OCH_3$ | $CF_3$ | |
| 2.186 | 2-Cl | 6-$CF_3$ | H | $OC_2H_5$ | $CF_3$ | |
| 2.187 | 2-Cl | 6-$CF_3$ | H | $SC_2H_5$ | $CF_3$ | |
| 2.188 | 2-Cl | 6-$CF_3$ | H | $CH_3$ | $CCl_2CF_3$ | |
| 2.189 | 2-Cl | 6-$CF_3$ | H | $CH_3$ | $CCl_2CH_3$ | |
| 2.190 | 2-Cl | 6-$CF_3$ | H | Phenyl | $CF_3$ | |
| 2.191 | 2-Cl | 3-$CH_3$ | H | $CH_3$ | $CF_3$ | |
| 2.192 | 2-Cl | 3-$CH_3$ | H | $CH_3$ | $CF_2CF_3$ | |
| 2.193 | 2-Cl | 3-$CH_3$ | H | $C_2H_5$ | $CF_3$ | |
| 2.194 | 2-Cl | 3-$CH_3$ | H | $C_3H_7$(n) | $CF_3$ | |
| 2.195 | 2-Cl | 3-$CH_3$ | H | $C_3H_7$(i) | $CF_3$ | |
| 2.196 | 2-Cl | 3-$CH_3$ | H | $CH_3$ | Cl | |
| 2.197 | 2-Cl | 3-$CH_3$ | H | $CH_3$ | $CF_2Cl$ | |
| 2.198 | 2-Cl | 3-$CH_3$ | H | $CH_3$ | $CHCl_2$ | |
| 2.199 | 2-Cl | 3-$CH_3$ | H | $CH_3$ | $CHClF$ | |
| 2.200 | 2-Cl | 3-$CH_3$ | H | $OCH_3$ | $CF_3$ | |
| 2.201 | 2-Cl | 5-$CH_3$ | H | $CH_3$ | Cl | Fp. 77-78°C |
| 2.202 | 2-Cl | 5-$CH_3$ | H | $CH_3$ | $CHCl_2$ | |
| 2.203 | 2-Cl | 5-$CH_3$ | H | $CH_3$ | $CCl_2CF_3$ | |
| 2.204 | 2-Cl | 5-$CH_3$ | H | $CH_3$ | $CHClF$ | |
| 2.205 | 2-Cl | 5-$CH_3$ | H | $CH_3$ | $CF_2Cl$ | |
| 2.206 | 2-Cl | 5-$CH_3$ | H | $C_2H_5$ | $CF_2Cl$ | |
| 2.207 | 2-Cl | 5-$CH_3$ | H | $CH_3$ | $CF_3$ | |
| 2.208 | 2-Cl | 5-$CH_3$ | H | $C_2H_5$ | $CF_3$ | |
| 2.209 | 2-Cl | 5-$CH_3$ | H | $C_3H_7$(n) | $CF_3$ | |
| 2.210 | 2-Cl | 5-$CH_3$ | H | $C_3H_7$(i) | $CF_3$ | |
| 2.211 | 2-Cl | 5-$CH_3$ | H | $C_4H_9$(n) | $CF_3$ | |
| 2.212 | 2-Cl | 5-$CH_3$ | H | $C_4H_9$(t) | $CF_3$ | |
| 2.213 | 2-Cl | 5-$CH_3$ | H | 2-Furyl | $CF_3$ | |
| 2.214 | 2-Cl | 5-$CH_3$ | H | $CH_3$ | $CF_2CF_3$ | |
| 2.215 | 2-Cl | 5-$CH_3$ | H | $C_2H_5$ | $CF_2CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.216 | 2-Cl | 5-$CH_3$ | H | $OCH_3$ | $CF_3$ | |
| 2.217 | 2-Cl | 5-$CH_3$ | H | $OC_2H_5$ | $CF_3$ | |
| 2.218 | 2-Cl | 5-$CH_3$ | H | $SCH_3$ | $CF_3$ | |
| 2.219 | 2-Cl | 6-$CH_3$ | H | $CH_3$ | Cl | Fp. 142-143°C |
| 2.220 | 2-Cl | 6-$CH_3$ | H | CN | $CH_3$ | |
| 2.221 | 2-Cl | 6-$CH_3$ | H | $CH_3$ | $CF_3$ | Fp. 143-144°C |
| 2.222 | 2-Cl | 6-$CH_3$ | H | $CH_3$ | $CF_2Cl$ | Fp. 158-162°C |
| 2.223 | 2-Cl | 6-$CH_3$ | H | $CH_3$ | $CF_2CF_3$ | |
| 2.224 | 2-Cl | 6-$CH_3$ | H | $CH_3$ | $CCl_2CF_3$ | |
| 2.225 | 2-Cl | 6-$CH_3$ | H | $CH_3$ | $CHF_2$ | |
| 2.226 | 2-Cl | 6-$CH_3$ | H | $CH_3$ | $CHCl_2$ | |
| 2.227 | 2-Cl | 6-$CH_3$ | H | $C_2H_5$ | $CF_3$ | Fp. 99-102°C |
| 2.228 | 2-Cl | 6-$CH_3$ | H | $C_3H_7$(i) | $CF_3$ | Fp. 56-59°C |
| 2.229 | 2-Cl | 6-$CH_3$ | H | Cyclo-propyl | $CF_3$ | Fp. 75-77°C |
| 2.230 | 2-Cl | 6-$CH_3$ | H | $C_4H_9$(i) | $CF_3$ | |
| 2.231 | 2-Cl | 6-$CH_3$ | H | $C_2H_5$ | $CF_2CF_3$ | Fp. 89-91°C |
| 2.232 | 2-Cl | 6-$CH_3$ | H | $OCH_3$ | $CF_3$ | |
| 2.233 | 2-Cl | 6-$CH_3$ | H | $OCH_2CH_2Cl$ | $CF_3$ | |
| 2.234 | 2-Cl | 6-$CH_3$ | H | $OCH_2CH_2OCH_3$ | $CF_3$ | |
| 2.235 | 2-Cl | 6-$CH_3$ | H | $OC_2H_5$ | $CF_3$ | |
| 2.236 | 2-Cl | 6-$CH_3$ | H | $SCH_3$ | $CF_3$ | |
| 2.237 | 2-Cl | 6-$CH_3$ | H | $SC_2H_5$ | $CF_3$ | |
| 2.238 | 2-Cl | 6-$CH_3$ | H | $SC_3H_7$(i) | $CF_3$ | |
| 2.239 | 2-Cl | 6-$CH_3$ | H | $SC_4H_9$(i) | $CF_3$ | |
| 2.240 | 2-Cl | 5-$OCH_3$ | H | $CH_3$ | $CF_3$ | |
| 2.241 | 2-Cl | 5-$OCH_3$ | H | $C_2H_5$ | $CF_3$ | |
| 2.242 | 2-Cl | 5-$OCH_3$ | H | $CH_3$ | Cl | |
| 2.243 | 2-Cl | 5-$OCH_3$ | H | $OCH_3$ | $CF_3$ | |
| 2.244 | 2-Cl | 5-$OCH_3$ | H | $CH_3$ | $CHF_2$ | |
| 2.245 | 2-Cl | 5-$OCH_3$ | H | $CH_3$ | $CF_2CF_3$ | |
| 2.246 | 2-Cl | 5-$OCH_3$ | H | $CH_3$ | $CF_2Cl$ | |
| 2.247 | 2-Cl | 5-$COOCH_3$ | H | $CH_3$ | $CF_3$ | Fp. 88-89°C |
| 2.248 | 2-Cl | 5-$COOCH_3$ | H | $C_2H_5$ | $CF_3$ | |
| 2.249 | 2-Cl | 5-$COOC_2H_5$ | H | $CH_3$ | $CF_3$ | |
| 2.250 | 2-Cl | 5-$COOC_2H_5$ | H | $CH_3$ | $CF_2CF_3$ | |
| 2.251 | 2-Cl | 5-$COOC_3H_7$(i) | H | $CH_3$ | $CF_3$ | |
| 2.252 | 2-Cl | 5-$COOC_3H_7$(i) | H | $C_3H_7$(i) | $CF_3$ | |
| 2.253 | 2-Cl | 5-$COOC_3H_7$(i) | H | $CH_3$ | $CF_2Cl$ | |
| 2.254 | 2-Cl | 5-$COOC_3H_7$(i) | H | Phenyl | $CF_3$ | |
| 2.255 | 2-Cl | 5-$COOC_3H_7$(i) | H | Cyclo-propyl | $CF_3$ | |
| 2.256 | 2-Cl | 5-$COOC_3H_7$(i) | H | $CH_3$ | CHFCl | |
| 2.257 | 3-Cl | 5-Cl | H | $CH_3$ | Cl | Fp. 152-153°C |
| 2.258 | 3-Cl | 5-Cl | H | $CH_3$ | $CF_3$ | Fp. 93-94°C |
| 2.259 | 3-Cl | 5-Cl | H | $C_2H_5$ | $CF_3$ | |
| 2.260 | 3-Cl | 5-Cl | H | $C_3H_7$(n) | $CF_3$ | |
| 2.261 | 3-Cl | 5-Cl | H | $C_3H_7$(i) | $CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.262 | 3-Cl | 5-Cl | H | Cyclo-propyl | $CF_3$ | |
| 2.263 | 3-Cl | 5-Cl | H | Phenyl | $CF_3$ | |
| 2.264 | 3-Cl | 5-Cl | H | $CH_3$ | $CHF_2$ | |
| 2.265 | 3-Cl | 5-Cl | H | $CH_3$ | $CF_2Cl$ | |
| 2.266 | 3-Cl | 5-Cl | H | $C_2H_5$ | $CF_2Cl$ | |
| 2.267 | 3-Cl | 5-Cl | H | $OCH_3$ | $CF_3$ | |
| 2.268 | 3-Cl | 5-Cl | H | $OC_2H_5$ | $CF_3$ | |
| 2.269 | 3-Cl | 5-Cl | H | $SCH_3$ | $CF_3$ | |
| 2.270 | 3-Cl | 5-Cl | H | $SC_3H_7$(i) | $CF_3$ | |
| 2.271 | 2-$CH_3$ | 5-Cl | H | $CH_3$ | Cl | Fp. 118-119°C |
| 2.272 | 2-$CH_3$ | 5-Cl | H | $CH_3$ | $CF_3$ | |
| 2.273 | 2-$CH_3$ | 5-Cl | H | CN | $CF_3$ | |
| 2.274 | 2-$CH_3$ | 5-Cl | H | $C_2H_5$ | Cl | |
| 2.275 | 2-$CH_3$ | 5-Cl | H | $CH_3$ | $OCHF_2$ | |
| 2.276 | 2-$CH_3$ | 5-Cl | H | $CH_3$ | Br | |
| 2.277 | 2-$CH_3$ | 5-Cl | H | $CH_3$ | F | |
| 2.278 | 2-$CH_3$ | 5-Cl | H | $CH_3$ | $CH_3$ | Fp. 121-122°C |
| 2.279 | 2-$CH_3$ | 5-Cl | H | $OCH_3$ | $CH_3$ | |
| 2.280 | 2-$CH_3$ | 5-Cl | H | $C_2H_5$ | $CF_3$ | |
| 2.281 | 2-$CH_3$ | 5-Cl | H | $C_3H_7$(n) | $CF_3$ | |
| 2.282 | 2-$CH_3$ | 5-Cl | H | $C_3H_7$(i) | $CF_3$ | |
| 2.283 | 2-$CH_3$ | 5-Cl | H | Cyclo-propyl | $CF_3$ | |
| 2.284 | 2-$CH_3$ | 5-Cl | H | Phenyl | $CF_3$ | |
| 2.285 | 2-$CH_3$ | 5-Cl | H | $C_4H_9$(n) | $CF_3$ | |
| 2.286 | 2-$CH_3$ | 5-Cl | H | $C_4H_9$(i) | $CF_3$ | |
| 2.287 | 2-$CH_3$ | 5-Cl | H | $C_4H_9$(t) | $CF_3$ | |
| 2.288 | 2-$CH_3$ | 5-Cl | H | $CH_3$ | $CF_2CF_3$ | |
| 2.289 | 2-$CH_3$ | 5-Cl | H | $C_2H_5$ | $CF_2CF_3$ | |
| 2.290 | 2-$CH_3$ | 5-Cl | H | $C_3H_7$(i) | $CF_2CF_3$ | |
| 2.291 | 2-$CH_3$ | 5-Cl | H | $CH_3$ | $CF_2Cl$ | |
| 2.292 | 2-$CH_3$ | 5-Cl | H | $C_2H_5$ | $CF_2Cl$ | |
| 2.293 | 2-$CH_3$ | 5-Cl | H | $CH_3$ | $CHF_2$ | |
| 2.294 | 2-$CH_3$ | 5-Cl | H | $C_2H_5$ | $CHF_2$ | |
| 2.295 | 2-$CH_3$ | 5-Cl | H | $CH_3$ | $CHFCl$ | |
| 2.296 | 2-$CH_3$ | 5-Cl | H | $C_2H_5$ | $CHFCl$ | |
| 2.297 | 2-$CH_3$ | 5-Cl | H | $CH_3$ | $CHCl_2$ | |
| 2.298 | 2-$CH_3$ | 5-Cl | H | $C_2H_5$ | $CHCl_2$ | |
| 2.299 | 2-$CH_3$ | 5-Cl | H | $CH_3$ | $CCl_2CF_3$ | |
| 2.300 | 2-$CH_3$ | 5-Cl | H | $CH_3$ | $CCl_2CH_3$ | |
| 2.301 | 2-$CH_3$ | 5-Cl | H | $OCH_3$ | $CF_3$ | |
| 2.302 | 2-$CH_3$ | 5-Cl | H | $OC_2H_5$ | $CF_3$ | |
| 2.303 | 2-$CH_3$ | 5-Cl | H | $SCH_3$ | $CF_3$ | |
| 2.304 | 2-$CH_3$ | 5-Cl | H | $SC_2H_5$ | $CF_3$ | |
| 2.305 | 2-$CH_3$ | 5-Cl | H | $SC_4H_9$(i) | $CF_3$ | |
| 2.306 | 2-$CH_3$ | 3-Cl | H | $CH_3$ | Cl | Fp. 116-117°C |
| 2.307 | 2-$CH_3$ | 3-Cl | H | $CH_3$ | $CF_3$ | |
| 2.308 | 2-$CH_3$ | 3-Cl | H | $C_2H_5$ | $CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.309 | 2-$CH_3$ | 3-Cl | H | $C_3H_7$(i) | $CF_3$ | |
| 2.310 | 2-$CH_3$ | 3-Cl | H | Cyclo-propyl | $CF_3$ | |
| 2.311 | 2-$CH_3$ | 3-Cl | H | $CH_3$ | $CF_2Cl$ | |
| 2.312 | 2-$CH_3$ | 3-Cl | H | $CH_3$ | $CF_2CF_3$ | |
| 2.313 | 2-$CH_3$ | 3-Cl | H | $C_2H_5$ | $CF_2CF_3$ | |
| 2.314 | 2-$CH_3$ | 3-Cl | H | $OCH_3$ | $CF_3$ | |
| 2.315 | 2-$CH_3$ | 3-Cl | H | $SCH_3$ | $CF_3$ | |
| 2.316 | 2-$CH_3$ | 5-$NO_2$ | H | $CH_3$ | Cl | Fp. 178-179°C |
| 2.317 | 2-$CH_3$ | 5-$NO_2$ | H | $CH_3$ | $CF_3$ | |
| 2.318 | 2-$CH_3$ | 5-$NO_2$ | H | $CH_3$ | $CF_2Cl$ | |
| 2.319 | 2-$CH_3$ | 5-$NO_2$ | H | $CH_3$ | $CHF_2$ | |
| 2.320 | 2-$CH_3$ | 5-$NO_2$ | H | $C_2H_5$ | $CF_3$ | |
| 2.321 | 2-$CH_3$ | 5-$NO_2$ | H | $C_3H_7$(n) | $CF_3$ | |
| 2.322 | 2-$CH_3$ | 5-$NO_2$ | H | $C_3H_7$(i) | $CF_3$ | |
| 2.323 | 2-$CH_3$ | 5-$NO_2$ | H | $C_4H_9$(t) | $CF_3$ | |
| 2.324 | 2-$CH_3$ | 3-$NO_2$ | H | $CH_3$ | Cl | Fp. 160-161°C |
| 2.325 | 2-$CH_3$ | 3-$NO_2$ | H | $CH_3$ | $CF_3$ | |
| 2.326 | 2-$CH_3$ | 3-$NO_2$ | H | $CH_3$ | $CF_2CF_3$ | |
| 2.327 | 2-$CH_3$ | 3-$NO_2$ | H | $C_2H_5$ | $CF_2CF_3$ | |
| 2.328 | 2-$CH_3$ | 3-$NO_2$ | H | Phenyl | $CF_3$ | |
| 2.329 | 2-$CH_3$ | 3-$NO_2$ | H | $C_2H_5$ | $CF_3$ | |
| 2.330 | 2-$CH_3$ | 3-$NO_2$ | H | $C_3H_7$(i) | $CF_3$ | |
| 2.331 | 2-$CH_3$ | 6-$CF_3$ | H | $CH_3$ | Cl | |
| 2.332 | 2-$CH_3$ | 6-$CF_3$ | H | $CH_3$ | $CF_3$ | Fp. 100-101°C |
| 2.333 | 2-$CH_3$ | 6-$CF_3$ | H | $CH_3$ | $CHF_2$ | |
| 2.334 | 2-$CH_3$ | 6-$CF_3$ | H | $CH_3$ | $CF_2Cl_2$ | |
| 2.335 | 2-$CH_3$ | 6-$CF_3$ | H | $CH_3$ | $CHCl_2$ | |
| 2.336 | 2-$CH_3$ | 6-$CF_3$ | H | $OCH_3$ | $CF_3$ | |
| 2.337 | 2-$CH_3$ | 6-$CF_3$ | H | $OC_2H_5$ | $CF_3$ | |
| 2.338 | 2-$CH_3$ | 6-$CF_3$ | H | $SCH_3$ | $CF_3$ | |
| 2.339 | 2-$CH_3$ | 6-$CF_3$ | H | $SC_2H_5$ | $CF_3$ | |
| 2.340 | 2-$CH_3$ | 6-$CF_3$ | H | $C_2H_5$ | $CF_3$ | |
| 2.341 | 2-$CH_3$ | 6-$CF_3$ | H | $CH_3$ | $CF_2CF_3$ | |
| 2.342 | 2-$CH_3$ | 6-$CF_3$ | H | $C_2H_5$ | $CF_2CF_3$ | |
| 2.343 | 2-$CH_3$ | 6-$CF_3$ | H | $C_3H_7$(i) | $CF_3$ | |
| 2.344 | 2-$CH_3$ | 6-$CF_3$ | H | $C_3H_7$(n) | $CF_3$ | |
| 2.345 | 2-$CH_3$ | 6-$CF_3$ | H | Cyclo-propyl | $CF_3$ | |
| 2.346 | 2-$OCHF_2$ | 5-Cl | H | $CH_3$ | Cl | Fp. 61-62°C |
| 2.347 | 2-$OCHF_2$ | 5-Cl | H | $CH_3$ | $CH_3$ | |
| 2.348 | 2-$OCHF_2$ | 5-Cl | H | $CH_3$ | $CHCl_2$ | |
| 2.349 | 2-$OCHF_2$ | 5-Cl | H | $CH_3$ | $CHFCl$ | |
| 2.350 | 2-$OCHF_2$ | 5-Cl | H | $CH_3$ | $CHF_2$ | |
| 2.351 | 2-$OCHF_2$ | 5-Cl | H | $CH_3$ | $CF_2Cl$ | |
| 2.352 | 2-$OCHF_2$ | 5-Cl | H | $CH_3$ | $CF_3$ | Fp. 66-67°C |
| 2.353 | 2-$OCHF_2$ | 5-Cl | H | $CH_3$ | $CF_2CF_3$ | |
| 2.354 | 2-$OCHF_2$ | 5-Cl | H | $CH_3$ | $CCl_2CF_3$ | |
| 2.355 | 2-$OCHF_2$ | 5-Cl | H | $CH_3$ | $CCl_2CH_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.356 | 2-$OCHF_2$ | 5-Cl | H | $C_2H_5$ | $CF_3$ | |
| 2.357 | 2-$OCHF_2$ | 5-Cl | H | $C_3H_7$(n) | $CF_3$ | |
| 2.358 | 2-$OCHF_2$ | 5-Cl | H | $C_3H_7$(i) | $CF_3$ | |
| 2.359 | 2-$CH_3$ | 6-Cl | H | Cyclo-propyl | $CF_3$ | |
| 2.360 | 2-$CH_3$ | 6-Cl | H | $C_4H_9$(n) | $CF_3$ | |
| 2.361 | 2-$CH_3$ | 6-Cl | H | $C_4H_9$(i) | $CF_3$ | |
| 2.362 | 2-$CH_3$ | 6-Cl | H | $C_4H_9$(t) | $CF_3$ | |
| 2.363 | 2-$CH_3$ | 6-Cl | H | Phenyl | $CF_3$ | |
| 2.364 | 2-$CH_3$ | 6-Cl | H | 2-Furyl | $CF_3$ | |
| 2.365 | 2-$CH_3$ | 6-Cl | H | 2-Thienyl | $CF_3$ | |
| 2.366 | 2-$CH_3$ | 6-Cl | H | $C_2H_5$ | $CF_2CF_3$ | |
| 2.367 | 2-$CH_3$ | 6-Cl | H | $C_2H_5$ | $CCl_2CH_3$ | |
| 2.368 | 2-$CH_3$ | 6-Cl | H | $OCH_3$ | $CF_3$ | |
| 2.369 | 2-$CH_3$ | 6-Cl | H | $OC_2H_5$ | $CF_3$ | |
| 2.370 | 2-$CH_3$ | 6-Cl | H | $SCH_3$ | $CF_3$ | |
| 2.371 | 2-Cl | 6-Cl | 3-Cl | $CH_3$ | Cl | |
| 2.372 | 2-Cl | 6-Cl | 3-Cl | $CH_3$ | $CF_3$ | |
| 2.373 | 2-Cl | 6-Cl | 3-Cl | $C_2H_5$ | $CF_3$ | |
| 2.374 | 2-Cl | 6-Cl | 3-Cl | $C_3H_7$(i) | $CF_3$ | |
| 2.375 | 2-Cl | 6-Cl | 3-Cl | $CH_3$ | $CF_2CF_3$ | |
| 2.376 | 2-Cl | 6-Cl | 3-Cl | Phenyl | $CF_3$ | |
| 2.377 | 2-Cl | 6-Cl | 3-Cl | $OCH_3$ | $CF_3$ | |
| 2.378 | 2-Cl | 6-Cl | 3-Cl | $OC_2H_5$ | $CF_3$ | |
| 2.379 | 2-Cl | 6-Cl | 3-Cl | $SC_2H_5$ | $CF_3$ | |
| 2.380 | 2-$OCH_3$ | 3-Cl | H | $CH_3$ | $CF_3$ | Fp. 41-42°C |
| 2.381 | 2-$OCH_3$ | 3-Cl | H | $C_2H_5$ | $CF_3$ | |
| 2.382 | 2-$OCH_3$ | 3-Cl | H | $C_3H_7$(n) | $CF_3$ | |
| 2.383 | 2-$OCH_3$ | 3-Cl | H | Cyclo-propyl | $CF_3$ | |
| 2.384 | 2-$OCH_3$ | 3-Cl | H | $CH_3$ | Cl | |
| 2.385 | 2-$OCH_3$ | 3-Cl | H | $CH_3$ | $CF_2CF_3$ | |
| 2.386 | 2-$OCH_3$ | 3-Cl | H | $OCH_3$ | $CF_3$ | |
| 2.387 | 2-$OCH_3$ | 3-Cl | H | $SCH_3$ | $CF_3$ | |
| 2.388 | 2-$OCH_3$ | 3-Cl | H | $CH_3$ | $CF_2Cl$ | |
| 2.389 | 2-$OCH_3$ | 3-Cl | H | $CH_3$ | $CHF_2$ | |
| 2.390 | 2-$OCH_3$ | 5-Cl | H | $CH_3$ | Cl | |
| 2.391 | 2-$OCH_3$ | 5-Cl | H | $CH_3$ | $CH_3$ | Fp. 106-107°C |
| 2.392 | 2-$OCH_3$ | 5-Cl | H | $CH_3$ | $CF_3$ | Fp. 65-66°C |
| 2.393 | 2-$OCH_3$ | 5-Cl | H | $CH_3$ | Br | |
| 2.394 | 2-$OCH_3$ | 5-Cl | H | CN | $CH_3$ | |
| 2.395 | 2-$OCH_3$ | 5-Cl | H | $CH_3$ | $CF_2Cl$ | |
| 2.396 | 2-$OCH_3$ | 5-Cl | H | $CH_3$ | $CHF_2$ | |
| 2.397 | 2-$OCH_3$ | 5-Cl | H | $CH_3$ | $CHCl_2$ | |
| 2.398 | 2-$OCH_3$ | 5-Cl | H | $CH_3$ | $CF_2CF_3$ | Fp. 75-76°C |
| 2.399 | 2-$OCH_3$ | 5-Cl | H | $CH_3$ | $CCl_2CF_3$ | |
| 2.400 | 2-$OCH_3$ | 5-Cl | H | $CH_3$ | CHClF | |
| 2.401 | 2-$OCH_3$ | 5-Cl | H | $CH_3$ | $CCl_2CH_3$ | |
| 2.402 | 2-$OCH_3$ | 5-Cl | H | $C_2H_5$ | $CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.403 | 2-$OCH_3$ | 5-Cl | H | $C_2H_5$ | $CF_2CF_3$ | Fp. 64-65°C |
| 2.404 | 2-$OCH_3$ | 5-Cl | H | $C_2H_5$ | $CF_2Cl$ | |
| 2.405 | 2-$OCH_3$ | 5-Cl | H | $C_3H_7$(n) | $CF_3$ | $n_D^{25}$: 1,5463 |
| 2.406 | 2-$OCH_3$ | 5-Cl | H | $C_3H_7$(i) | $CF_3$ | $n_D^{25}$: 1,5455 |
| 2.407 | 2-$OCH_3$ | 5-Cl | H | $C_3H_7$(i) | $CF_2CF_3$ | |
| 2.408 | 2-$OCH_3$ | 5-Cl | H | Cyclo-propyl | $CF_3$ | |
| 2.409 | 2-$OCH_3$ | 5-Cl | H | $C_4H_9$(n) | $CF_3$ | |
| 2.410 | 2-$OCH_3$ | 5-Cl | H | $C_2H_5$(i) | $CF_3$ | |
| 2.411 | 2-$OCH_3$ | 5-Cl | H | $C_4H_9$(t) | $CF_3$ | Fp. 55-56°C |
| 2.412 | 2-$OCH_3$ | 5-Cl | H | $OCH_3$ | $CF_3$ | |
| 2.413 | 2-$OCH_3$ | 5-Cl | H | $OC_4H_9$(n) | $CF_3$ | |
| 2.414 | 2-$OCH_3$ | 5-Cl | H | $SCH_3$ | $CF_3$ | |
| 2.415 | 2-$OCH_3$ | 5-Cl | H | $SC_2H_5$ | $CF_3$ | |
| 2.416 | 2-$OCH_3$ | 5-Cl | H | $SC_3H_7$(n) | $CF_3$ | |
| 2.417 | 2-$OCH_3$ | 5-Cl | H | $SC_3H_7$(i) | $CF_3$ | |
| 2.418 | 2-$OC_2H_5$ | 5-Cl | H | $CH_3$ | Cl | |
| 2.419 | 2-$OC_2H_5$ | 5-Cl | H | $CH_3$ | $CF_3$ | |
| 2.420 | 2-$OC_2H_5$ | 5-Cl | H | $C_2H_5$ | $CF_3$ | |
| 2.421 | 2-$OC_2H_5$ | 5-Cl | H | $C_3H_7$(i) | $CF_3$ | |
| 2.422 | 2-$OC_2H_5$ | 5-Cl | H | Cyclo-propyl | $CF_3$ | |
| 2.423 | 2-$OC_2H_5$ | 5-Cl | H | Phenyl | $CF_3$ | |
| 2.424 | 2-$OC_2H_5$ | 5-Cl | H | $OCH_3$ | $CF_3$ | |
| 2.425 | 2-$OC_2H_5$ | 5-Cl | H | $CH_3$ | $CF_2CF_3$ | |
| 2.426 | 2-$OC_3H_7$(i) | 5-Cl | H | $CH_3$ | Cl | |
| 2.427 | 2-$OC_3H_7$(i) | 5-Cl | H | $CH_3$ | $CF_3$ | |
| 2.428 | 2-$OC_3H_7$(i) | 5-Cl | H | $CH_3$ | $CF_2Cl$ | |
| 2.429 | 2-$OC_3H_7$(i) | 5-Cl | H | $C_2H_5$ | $CF_3$ | |
| 2.430 | 2-$OC_3H_7$(i) | 5-Cl | H | $C_3H_7$(n) | $CF_3$ | |
| 2.431 | 2-$OC_3H_7$(i) | 5-Cl | H | $CH_3$ | $CHF_3$ | |
| 2.432 | 2-$OC_3H_7$(i) | 5-Cl | H | $OCH_3$ | $CF_3$ | |
| 2.433 | 2-$OC_3H_7$(i) | 5-Cl | H | $SCH_3$ | $CF_3$ | |
| 2.434 | 2-$OCF_3$ | H | H | $CH_3$ | Cl | |
| 2.435 | 2-$OCF_3$ | H | H | $CH_3$ | $CF_3$ | $n_D^{23}$: 1,4943 |
| 2.436 | 2-$OCF_3$ | H | H | $CH_3$ | $CF_2Cl$ | |
| 2.437 | 2-$OCF_3$ | H | H | $CH_3$ | $CHF_2$ | |
| 2.438 | 2-$OCF_3$ | H | H | $CH_3$ | $CHFCl$ | |
| 2.439 | 2-$OCF_3$ | H | H | $C_2H_5$ | $CF_3$ | |
| 2.440 | 2-$OCF_3$ | H | H | $C_3H_7$(i) | $CF_3$ | |
| 2.441 | 2-$OCF_3$ | H | H | $C_4H_9$(n) | $CF_3$ | |
| 2.442 | 2-$OCF_3$ | H | H | $C_4H_9$(t) | $CF_3$ | |
| 2.443 | 2-$OCF_3$ | 5-Cl | H | $CH_3$ | $CF_3$ | |
| 2.444 | 2-$OCF_3$ | 5-Cl | H | $CH_3$ | Cl | |
| 2.445 | 2-$OCF_3$ | 5-Cl | H | $CH_3$ | $CF_3$ | |
| 2.446 | 2-$OCF_3$ | 5-Cl | H | $CH_3$ | $CF_2Cl$ | |
| 2.447 | 2-$OCF_3$ | 5-Cl | H | $CH_3$ | $CHF_2$ | |
| 2.448 | 2-$OCF_3$ | 5-Cl | H | $CH_3$ | $CHCl_2$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.449 | 2-$OCF_3$ | 5-Cl | H | $CH_3$ | CHFCl | |
| 2.450 | 2-$OCF_3$ | 5-Cl | H | $CH_3$ | $CF_2CF_3$ | |
| 2.451 | 2-$OCF_3$ | 5-Cl | H | $C_2H_5$ | $CF_2CF_3$ | |
| 2.452 | 2-$OCF_3$ | 5-Cl | H | $C_2H_5$ | $CF_3$ | |
| 2.453 | 2-$OCF_3$ | 5-Cl | H | $C_3H_7$(i) | $CF_3$ | |
| 2.454 | 2-$OCF_3$ | 5-Cl | H | Cyclo-propyl | $CF_3$ | |
| 2.455 | 2-$OCF_3$ | 5-Cl | H | Phenyl | $CF_3$ | |
| 2.456 | 2-$OCF_3$ | 5-Cl | H | $OC_2H_5$ | $CF_3$ | |
| 2.457 | 2-$OCF_3$ | 5-Cl | H | $SC_2H_5$ | $CF_3$ | |
| 2.458 | 2-$SCH_3$ | H | H | $CH_3$ | Cl | |
| 2.459 | 2-$SCH_3$ | H | H | $CH_3$ | $CF_3$ | Fp. 74-75°C |
| 2.460 | 2-$SCH_3$ | H | H | $CH_3$ | $CHF_2$ | |
| 2.461 | 2-$SCH_3$ | H | H | $CH_3$ | $CF_2CF_3$ | |
| 2.462 | 2-$SCH_3$ | H | H | $C_2H_5$ | $CF_3$ | |
| 2.463 | 2-$SCH_3$ | H | H | $C_3H_7$(i) | $CF_3$ | $n_D^{25}$: 1,5575 |
| 2.464 | 2-$SCH_3$ | H | H | $C_4H_9$(n) | $CF_3$ | |
| 2.465 | 2-$SCH_3$ | H | H | $C_4H_9$(t) | $CF_3$ | |
| 2.466 | 2-$SCH_3$ | 5-Cl | H | $CH_3$ | Cl | |
| 2.467 | 2-$SCH_3$ | 5-Cl | H | $CH_3$ | $CF_3$ | Fp. 144-145°C |
| 2.468 | 2-$SCH_3$ | 5-Cl | H | $CH_3$ | $CF_2CF_3$ | |
| 2.469 | 2-$SCH_3$ | 5-Cl | H | $CH_3$ | $CF_2Cl$ | |
| 2.470 | 2-$SCH_3$ | 5-Cl | H | $C_2H_5$ | $CF_3$ | |
| 2.471 | 2-$SOCH_3$ | H | H | $CH_3$ | Cl | |
| 2.472 | 2-$SOCH_3$ | H | H | $CH_3$ | $CF_3$ | |
| 2.473 | 2-$SOCH_3$ | H | H | $CH_3$ | $CHF_2$ | |
| 2.474 | 2-$SOCH_3$ | H | H | $CH_3$ | $CF_2CF_3$ | |
| 2.475 | 2-$SOCH_3$ | H | H | $C_2H_5$ | $CF_3$ | |
| 2.476 | 2-$SOCH_3$ | H | H | $C_3H_7$(i) | $CF_3$ | |
| 2.477 | 2-$SOCH_3$ | H | H | $C_4H_9$(n) | $CF_3$ | |
| 2.478 | 2-$SOCH_3$ | H | H | $C_4H_9$(t) | $CF_3$ | |
| 2.479 | 2-$SO_2CH_3$ | H | H | $CH_3$ | Cl | Fp. 168°C |
| 2.480 | 2-$SO_2CH_3$ | H | H | $CH_3$ | $CF_3$ | Fp. 149°C |
| 2.481 | 2-$SO_2CH_3$ | H | H | $C_2H_5$ | $CF_3$ | |
| 2.482 | 2-$SO_2CH_3$ | H | H | $C_3H_7$(n) | $CF_3$ | |
| 2.483 | 2-$SO_2CH_3$ | H | H | $C_3H_7$(i) | $CF_3$ | |
| 2.484 | 2-$SO_2CH_3$ | H | H | Cyclo-propyl | $CF_3$ | |
| 2.485 | 2-$SO_2CH_3$ | H | H | $C_4H_9$(i) | $CF_3$ | |
| 2.486 | 2-$SO_2CH_3$ | H | H | $CH_3$ | $CF_2CF_3$ | |
| 2.487 | 2-$SO_2CH_3$ | H | H | $C_2H_5$ | $CF_2CF_3$ | |
| 2.488 | 2-$SO_2CH_3$ | H | H | $C_3H_7$(i) | $CF_2CF_3$ | |
| 2.489 | 2-$SOCH_3$ | 5-Cl | H | $CH_3$ | Cl | |
| 2.490 | 2-$SOCH_3$ | 5-Cl | H | $CH_3$ | $CF_3$ | Fp. 194-195°C |
| 2.491 | 2-$SOCH_3$ | 5-Cl | H | $C_2H_5$ | $CF_3$ | |
| 2.492 | 2-$SOCH_3$ | 5-Cl | H | $CH_3$ | $CF_2CF_3$ | |
| 2.493 | 2-$SOCH_3$ | 5-Cl | H | $C_3H_7$(i) | $CF_3$ | |
| 2.494 | 2-$SO_2CH_3$ | 5-Cl | H | $CH_3$ | Cl | |

Tabelle II (Fortsetzung)

| Verb.Nr. | R[1] | R[2] | R[3] | R[4] | R[5] | physikalische Daten |
|---|---|---|---|---|---|---|
| 2.495 | 2-$SO_2CH_3$ | 5-Cl | H | $CH_3$ | $CF_3$ | Fp. 183-184°C |
| 2.496 | 2-$SO_2CH_3$ | 5-Cl | H | $C_2H_5$ | $CF_3$ | |
| 2.497 | 2-$SO_2CH_3$ | 5-Cl | H | $CH_3$ | $CF_2CF_3$ | |
| 2.498 | 2-$SO_2CH_3$ | 5-Cl | H | $C_3H_7$(i) | $CF_3$ | |
| 2.499 | 2-F | 5-F | H | $CH_3$ | $OCHF_2$ | |
| 2.500 | 2-F | 5-F | H | $CH_3$ | Cl | |
| 2.501 | 2-F | 5-F | H | $CH_3$ | $CF_3$ | Fp. 68-69°C |
| 2.502 | 2-F | 5-F | H | $C_2H_5$ | $CF_3$ | |
| 2.503 | 2-F | 5-F | H | $C_3H_7$(n) | $CF_3$ | |
| 2.504 | 2-F | 5-F | H | $C_3H_7$(i) | $CF_3$ | |
| 2.505 | 2-F | 5-F | H | Cyclo-propyl | $CF_3$ | Fp. 103-104°C |
| 2.506 | 2-F | 5-F | H | $CH_3$ | $CF_2CF_3$ | |
| 2.507 | 2-F | 5-F | H | $CH_3$ | $CF_2CF_2CF_3$ | |
| 2.508 | 2-F | 5-F | H | $CH_3$ | $CF_2Cl$ | |
| 2.509 | 2-F | 5-F | H | $CH_3$ | $CHF_2$ | |
| 2.510 | 2-F | 5-F | H | $CH_3$ | CHClF | |
| 2.511 | 2-F | 5-F | H | $C_2H_5$ | $CF_2Cl$ | |
| 2.512 | 2-F | 5-F | H | $C_3H_7$(i) | $CF_2Cl$ | $n_D^{25}$: 1,5358 |
| 2.513 | 2-F | 5-F | H | $C_4H_9$(n) | $CF_3$ | |
| 2.514 | 2-F | 5-F | H | $C_4H_9$(t) | $CF_3$ | Fp. 37-38°C |
| 2.515 | 2-F | 5-F | H | $C_2H_5$ | $CF_2CF_3$ | |
| 2.516 | 2-F | 5-F | H | $C_3H_7$(i) | $CF_2CF_3$ | Fp. 53-54°C |
| 2.517 | 2-F | 5-F | H | $C_3H_7$(n) | $CF_2CF_3$ | |
| 2.518 | 2-F | 5-F | H | $CH_3$ | $CCl_2CF_3$ | |
| 2.519 | 2-F | 5-F | H | $CH_3$ | $CHCl_2$ | |
| 2.520 | 2-F | 5-F | H | $C_2H_5$ | $CHCl_2$ | $n_D^{25}$: 1,5824 |
| 2.521 | 2-F | 5-F | H | $CH_3$ | $CCl_2CH_3$ | |
| 2.522 | 2-F | 5-F | H | $OCH_3$ | $CF_3$ | |
| 2.523 | 2-F | 5-F | H | $OC_3H_7$(i) | $CF_3$ | |
| 2.524 | 2-F | 5-F | H | $SCH_3$ | $CF_3$ | |
| 2.525 | 2-F | 5-F | H | $SC_3H_7$(i) | $CF_3$ | |
| 2.526 | 2-F | 6-F | H | $CH_3$ | Cl | |
| 2.527 | 2-F | 6-F | H | $CH_3$ | $CF_3$ | Fp. 164°C |
| 2.528 | 2-F | 6-F | H | $C_2H_5$ | $CF_3$ | Fp. 120-121°C |
| 2.529 | 2-F | 6-F | H | $C_3H_7$(n) | $CF_3$ | |
| 2.530 | 2-F | 6-F | H | $C_3H_7$(i) | $CF_3$ | Fp. 92-94°C |
| 2.531 | 2-F | 6-F | H | $CH_3$ | $CF_2CF_3$ | |
| 2.532 | 2-F | 6-F | H | $C_2H_5$ | $CF_2CF_3$ | Fp. 85-87°C |
| 2.533 | 2-F | 6-F | H | $CH_3$ | $CF_2Cl$ | Fp. 92-95°C |
| 2.534 | 2-F | 6-F | H | $CH_3$ | $CHF_2$ | |
| 2.535 | 2-F | 6-F | H | $CH_3$ | CHClF | |
| 2.536 | 2-F | 6-F | H | $OCH_3$ | $CF_3$ | |
| 2.537 | 2-F | 6-F | H | $SCH_3$ | $CF_3$ | |
| 2.538 | 2-F | 6-Cl | H | $CH_3$ | Cl | |
| 2.539 | 2-F | 6-Cl | H | $CH_3$ | $CH_3$ | |
| 2.540 | 2-F | 6-Cl | H | $CH_3$ | $CF_3$ | Fp. 160-161°C |
| 2.541 | 2-F | 6-Cl | H | $C_2H_5$ | $CF_3$ | |
| 2.542 | 2-F | 6-Cl | H | $C_3H_7$(i) | $CF_3$ | Fp. 108-109°C |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.543 | 2-F | 6-Cl | H | Cyclo-propyl | $CF_3$ | Fp. 95-96°C |
| 2.544 | 2-F | 6-Cl | H | $CH_3$ | $CF_2CF_3$ | |
| 2.545 | 2-F | 6-Cl | H | $C_3H_7(i)$ | $CF_2CF_3$ | |
| 2.546 | 2-F | 6-Cl | H | $CH_3$ | $CCl_2CF_3$ | |
| 2.547 | 2-F | 6-Cl | H | $CH_3$ | $CHCl_2$ | |
| 2.548 | 2-F | 6-Cl | H | $CH_3$ | $CHF_2$ | |
| 2.549 | 2-F | 6-Cl | H | $CH_3$ | $CHFCl$ | |
| 2.550 | 2-F | 6-Cl | H | $CH_3$ | $CF_2Cl$ | |
| 2.551 | 2-F | 6-Cl | H | $C_3H_7(i)$ | $CF_2Cl$ | |
| 2.552 | 2-F | 6-Cl | H | $OCH_3$ | $CF_3$ | |
| 2.553 | 2-CN | H | H | $CH_3$ | $CF_3$ | Fp. 117-118°C |
| 2.554 | 2-CN | H | H | $C_2H_5$ | $CF_3$ | |
| 2.555 | 2-CN | H | H | $C_3H_7(i)$ | $CF_3$ | |
| 2.556 | 2-CN | H | H | $CH_3$ | $CF_2CF_3$ | |
| 2.557 | 2-CN | H | H | $C_2H_5$ | $CF_2CF_3$ | |
| 2.558 | 2-CN | H | H | $C_3H_7(i)$ | $CF_2CF_3$ | |
| 2.559 | 2-CN | H | H | $CH_3$ | $CHF_2$ | |
| 2.560 | 2-CN | H | H | $CH_3$ | $CF_2Cl$ | |
| 2.561 | 2-CN | H | H | $OCH_3$ | $CF_3$ | |
| 2.562 | 2-CN | H | H | $OC_2H_5$ | $CF_3$ | |
| 2.563 | $2-PO(OC_2H_5)_2$ | H | H | $CH_3$ | $CF_3$ | Fp. 60-61°C |
| 2.564 | $2-PO(OC_2H_5)_2$ | H | H | $C_2H_5$ | $CF_3$ | |
| 2.565 | $2-PO(OC_2H_5)_2$ | H | H | $C_3H_7(i)$ | $CF_3$ | |
| 2.566 | 2-Cl | 5-Cl | H | $SOCH_3$ | $CF_3$ | |
| 2.567 | 2-Cl | 5-Cl | H | $SO_2CH_3$ | $CF_3$ | |
| 2.568 | 2-Cl | 6-Cl | H | $SOCH_3$ | $CF_3$ | Fp. 144-148°C |
| 2.569 | 2-Cl | 6-Cl | H | $SO_2CH_3$ | $CF_3$ | Fp. 144-146°C |
| 2.570 | 2-Cl | 6-Cl | H | $SOCH_3$ | $CH_3$ | |
| 2.571 | 2-Cl | 6-Cl | H | $SO_2CH_3$ | $CH_3$ | |
| 2.572 | 2-Cl | 6-Cl | H | CN | $CH_3$ | |
| 2.573 | 2-Cl | 6-Cl | H | CN | $CF_3$ | |
| 2.574 | 2-Cl | 6-Cl | H | $CH=CCl_2$ | Cl | |
| 2.575 | 2-Cl | 5-Cl | H | $CH=CCl_2$ | Cl | |
| 2.576 | $2-CF_3$ | H | H | $CH=CCl_2$ | Cl | |
| 2.577 | 2-Cl | 6-Cl | H | $CH_2OCH_3$ | $CF_3$ | |
| 2.578 | 2-Cl | 6-Cl | H | $CH_2OCH_3$ | $C_2F_5$ | |
| 2.579 | 2-Cl | 5-Cl | H | $CH_2OCH_3$ | $CF_3$ | |
| 2.580 | 2-Cl | 5-Cl | H | $CH_2OCH_3$ | $CF_2Cl$ | |
| 2.581 | $2-OCH_3$ | 5-Cl | H | $CH_2OCH_3$ | $CF_3$ | |
| 2.582 | $2-OCH_3$ | 5-Cl | H | $CH_2OCH_3$ | $CF_2CF_3$ | |
| 2.583 | 2-F | 5-F | H | $CH_2OCH_3$ | $CF_3$ | |
| 2.584 | $2-OCHF_2$ | 5-Cl | H | $CH_2OCH_3$ | $CF_3$ | |
| 2.585 | $2-OCHF_2$ | 5-Cl | H | $CH_2OCH_3$ | $CF_2Cl$ | |
| 2.586 | 3-Br | H | H | $CH_3$ | Cl | Fp. 101-102°C |
| 2.587 | 3-Br | H | H | $CH_3$ | $CF_3$ | |
| 2.588 | 2-Br | 5-Br | H | $CH_3$ | Cl | |
| 2.589 | 2-Br | 5-Br | H | $CH_3$ | $CF_3$ | Fp. 95-97°C |
| 2.590 | 2-Br | 5-Br | H | $OCH_3$ | $CF_3$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.591 | 2-Br | 5-Br | H | $C_2H_5$ | $CF_3$ | |
| 2.592 | 2-Br | 5-Br | H | $CH_3$ | $CF_2Cl$ | |
| 2.593 | 2-Br | 5-Br | H | $CH_3$ | $CHF_2$ | |
| 2.594 | 2-$CF_3$ | 5-Cl | H | $CH_3$ | $CF_3$ | Fp. 65-67°C |
| 2.595 | 2-$CF_3$ | 5-Cl | H | $CH_3$ | Cl | Fp. 117-118°C |
| 2.596 | 2-$CF_3$ | 5-Cl | H | $C_2H_5$ | $CF_3$ | |
| 2.597 | 2-$CF_3$ | 5-Cl | H | $C_3H_7$(i) | $CF_3$ | |
| 2.598 | 2-$CF_3$ | 5-Cl | H | Cyclo-propyl | $CF_3$ | |
| 2.599 | 2-$CF_3$ | 5-Cl | H | $CH_3$ | $CH_2Cl$ | |
| 2.600 | 2-$CF_3$ | 5-Cl | H | $CH_3$ | $CHF_2$ | |
| 2.601 | 2-$CF_3$ | 5-Cl | H | $C_2H_5$ | $CF_2Cl$ | |
| 2.602 | 2-$CF_3$ | 5-Cl | H | $C_3H_7$(i) | $CF_2Cl$ | |
| 2.603 | 2-$CF_3$ | 5-Cl | H | $CH_3$ | $CF_2CF_3$ | |
| 2.604 | 2-$CF_3$ | 5-Cl | H | $OCH_3$ | $CF_3$ | |
| 2.605 | 2-$CF_3$ | 5-Cl | H | $SCH_3$ | $CF_3$ | |
| 2.606 | 2-$CF_3$ | 5-Cl | H | $SOCH_3$ | $CF_3$ | |
| 2.607 | 2-$CF_3$ | 5-Cl | H | $SO_2CH_3$ | $CH_3$ | |
| 2.608 | 2-$CH_3$ | 3-$CH_3$ | H | $CH_3$ | Cl | |
| 2.609 | 2-$CH_3$ | 3-$CH_3$ | H | $CH_3$ | $CF_3$ | |
| 2.610 | 2-$CH_3$ | 5-$CH_3$ | H | $CH_3$ | Cl | |
| 2.611 | 2-$CH_3$ | 5-$CH_3$ | H | $CH_3$ | $CF_3$ | |
| 2.612 | 2-$CH_3$ | 6-$CH_3$ | H | $CH_3$ | Cl | |
| 2.613 | 2-$CH_3$ | 6-$CH_3$ | H | $CH_3$ | $CF_3$ | Fp. 145-146°C |
| 2.614 | 2-$CH_3$ | 6-$CH_3$ | H | $CH_3$ | $CF_2Cl$ | Fp. 147-148°C |
| 2.615 | 2-$CH_3$ | 6-$CH_3$ | H | $CH_3$ | $CH_2CF_3$ | |
| 2.616 | 2-$CH_3$ | 6-$CH_3$ | H | $OCH_3$ | $CF_3$ | |
| 2.617 | 2-$CH_3$ | 6-$CH_3$ | H | $SCH_3$ | $CF_3$ | |
| 2.618 | 2-$CH_3$ | 6-$C_2H_5$ | H | $CH_3$ | Cl | |
| 2.619 | 2-$CH_3$ | 6-$C_2H_5$ | H | $CH_3$ | $CF_3$ | Fp. 90-93°C |
| 2.620 | 2-$CH_3$ | 6-$C_2H_5$ | H | $OCH_3$ | $CF_3$ | |
| 2.621 | 2-$CH_3$ | 5-F | H | $CH_3$ | Cl | |
| 2.622 | 2-$CH_3$ | 5-F | H | $CH_3$ | $CF_3$ | Fp. 78-79°C |
| 2.623 | 2-$CH_3$ | 5-F | H | $CH_3$ | $CHF_2$ | |
| 2.624 | 2-$CH_3$ | 5-F | H | $CH_3$ | $CF_2Cl$ | |
| 2.625 | 2-$CH_3$ | 5-F | H | $C_2H_5$ | $CF_3$ | |
| 2.626 | 2-$CH_3$ | 5-F | H | $C_3H_7$(i) | $CF_3$ | |
| 2.627 | 2-$CH_3$ | 5-F | H | $OCH_3$ | $CF_3$ | |
| 2.628 | 2-$CH_3$ | 5-F | H | $OC_2H_5$ | $CF_3$ | |
| 2.629 | 2-Br | 6-Br | H | $CH_3$ | Cl | |
| 2.630 | 2-Br | 6-Br | H | $CH_3$ | $CF_3$ | |
| 2.631 | 2-Br | 6-Br | H | $C_2H_5$ | $CF_3$ | Fp. 104-105°C |
| 2.632 | 2-Br | 6-Br | H | $C_3H_7$(i) | $CF_3$ | |
| 2.633 | 2-Br | 6-Br | H | Cyclo-propyl | $CF_3$ | |
| 2.634 | 2-Br | 6-Br | H | $CH_3$ | $CHCl_2$ | |
| 2.635 | 2-Br | 6-Br | H | $CH_3$ | $CF_2CF_3$ | |
| 2.636 | 2-Br | H | H | $C_3H_7$(i) | $CF_2CF_3$ | $n_D^{25}$: 1,5307 |
| 2.637 | 2-$CF_3$ | H | H | $CH_3$ | $CHCl_2$ | Fp. 72-74°C |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.638 | 2-$OCHF_2$ | H | H | $CH_3$ | $CF_3$ | Fp. 71-72°C |
| 2.639 | 2-$OCHF_2$ | H | H | $C_3H_7$(i) | $CF_3$ | |
| 2.640 | 2-$OCHF_2$ | H | H | Cyclo-propyl | $CF_3$ | $n_D^{25}$: 1,5242 |
| 2.641 | 2-$OCHF_2$ | H | H | $CH_3$ | $CHF_2$ | |
| 2.642 | 2-$OCHF_2$ | H | H | $CH_3$ | CHFCl | |
| 2.643 | 2-$CF_3$ | H | H | $CH_3$ | $CHF_2$ | |
| 2.644 | 2-$CF_3$ | H | H | $CH_3$ | $CF_2Cl$ | |
| 2.645 | 2-$CF_3$ | H | H | $C_3H_7$(i) | $CF_2Cl$ | $n_D^{25}$: 1,5184 |
| 2.646 | 2-$CF_3$ | H | H | Cyclo-propyl | $CF_3$ | Fp. 63-64°C |
| 2.647 | 2-$CF_3$ | H | H | 2-Thienyl | $CF_3$ | Fp. 97-98°C |
| 2.648 | 2-Br | H | H | $C_4H_9$(n) | $CF_3$ | Fp. 65-66°C |
| 2.649 | 2-Br | H | H | $C_3H_7$(i) | $CF_3$ | $n_D^{25}$: 1,5583 |
| 2.650 | 2-Br | H | H | $CH_3$ | $CHF_2$ | |
| 2.651 | 2-Br | H | H | $CH_3$ | $CF_2Cl$ | |
| 2.652 | 2-Br | H | H | Cyclo-propyl | $CF_2Cl$ | |
| 2.653 | 2-Br | H | H | $C_2H_5$ | $CHF_2$ | |
| 2.654 | 2-Cl | 6-$CH_3$ | H | $CH_3$ | $CH_3$ | Fp. 141-142°C |
| 2.655 | 2-Cl | 6-Cl | H | $CH_3$ | $CHF_2$ | Fp. 99-101°C |
| 2.656 | 2-Cl | 6-Cl | H | $C_2H_5$ | $CHF_2$ | |
| 2.657 | 2-Cl | 5-Cl | H | $C_5H_{11}$(n) | $CF_3$ | Fp. 56-57°C |
| 2.658 | 2-$COOCH_3$ | H | H | $CH_3$ | $CF_3$ | Fp. 143-145°C |
| 2.659 | 2-$COOCH_3$ | H | H | $C_2H_5$ | $CF_3$ | |
| 2.660 | 2-$COOCH_3$ | H | H | $C_3H_7$(i) | $CF_3$ | |
| 2.661 | 2-$COOCH_3$ | H | H | Cyclo-propyl | $CF_3$ | |
| 2.662 | 2-$COOCH_3$ | H | H | $CH_3$ | $CF_2Cl$ | |
| 2.663 | 2-$COOCH_3$ | H | H | $CH_3$ | $CHF_2$ | |
| 2.664 | 2-$COOC_2H_5$ | H | H | $CH_3$ | $CF_3$ | |
| 2.665 | 2-$COOC_3H_7$(n) | H | H | $CH_3$ | $CF_3$ | |
| 2.666 | 2-$COOC_3H_7$(i) | H | H | $CH_3$ | $CF_3$ | |
| 2.667 | 2-$COOC_4H_9$(n) | H | H | $CH_3$ | $CF_3$ | |
| 2.668 | 2-$OCH_3$ | 5-F | H | $CH_3$ | $CF_3$ | Fp. 89-93°C |
| 2.669 | 2-$OCH_3$ | 5-F | H | $CH_3$ | $CHF_2$ | |
| 2.670 | 2-$OCH_3$ | 5-F | H | $C_2H_5$ | $CHF_2$ | |
| 2.671 | 2-$OCH_3$ | 5-F | H | $C_3H_7$(i) | $CF_3$ | |
| 2.672 | 2-$OCH_3$ | 5-F | H | Cyclo-propyl | $CF_3$ | |
| 2.673 | 2-$OCH_3$ | 5-F | H | Cyclo-propyl | $CF_3Cl$ | |

Tabelle II (Fortsetzung)

| Verb.Nr. | $R^1$ | $R^2$ | $R^3$ | $R^4$ | $R^5$ | physikalische Daten |
|---|---|---|---|---|---|---|
| 2.674 | 2-$OCHF_2$ | 5-F | H | $CH_3$ | $CF_3$ | |
| 2.675 | 2-$OCHF_2$ | 5-F | H | $CH_3$ | $CHF_2$ | |
| 2.676 | 2-$OCHF_2$ | 5-F | H | $CH_3$ | $CF_2Cl$ | |
| 2.677 | 2-$COOCH_3$ | 6-$CH_3$ | H | $CH_3$ | $CF_3$ | Fp. 90-91°C |
| 2.678 | 2-$COOCH_3$ | 6-$CH_3$ | H | $CH_3$ | $CHF_2$ | |
| 2.679 | 2-$COOCH_3$ | 6-$CH_3$ | H | $C_3H_7$(i) | $CF_3$ | |
| 2.680 | 2-$COOCH_3$ | 6-$CH_3$ | H | Cyclo-propyl | $CF_3$ | |
| 2.681 | 2-$COOCH_3$ | 6-Cl | H | $CH_3$ | $CF_3$ | Fp. 86-87°C |
| 2.682 | 2-$COOCH_3$ | 6-Cl | H | $CH_3$ | $CHF_2$ | |
| 2.683 | 2-Br | H | H | 2-Furyl | $CF_3$ | Fp. 108-109°C |
| 2.684 | 2-Cl | 6-Cl | 3-$CH_3$ | $CH_3$ | Cl | Fp. 158-164°C |
| 2.685 | 2-$CF_3$ | H | H | $C_2H_5$ | $CHCl_2$ | $n_D^{25}$: 1,5462 |
| 2.686 | 2-Cl | 5-Cl | H | Cl | $CF_3$ | Fp. 66-67°C |
| 2.687 | 2-$CONH_2$ | H | H | $CH_3$ | $CF_3$ | Fp. 187-190°C |
| 2.688 | 2-Cl | 6-Cl | H | $CH_3$<br>$CHCH_2CH_3$ | $CF_3$ | Fp. 91-92°C |
| 2.689 | 2-F | H | H | $C_2H_5$ | $CF_3$ | $n_D^{25}$: 1,5332 |
| 2.690 | 2-F | H | H | $CH_2OCH_3$ | $CF_3$ | Fp. 48-50°C |
| 2.691 | 2-F | H | H | $CH_2OCH_3$ | $CF_2Cl$ | $n_D^{25}$: 1,5525 |
| 2.692 | 2-$OCH_3$ | 6-Cl | H | $CH_3$ | $CF_3$ | Fp. 113-114°C |
| 2.693 | 2-$OCH_3$ | 6-Cl | H | $C_2H_5$ | $CF_3$ | Fp. 68-70°C |
| 2.694 | 2-$OCH_3$ | 6-Cl | H | $C_3H_7$(i) | $CF_3$ | Fp. 80-81°C |
| 2.695 | 2-$CH_3$ | 6-$CH_3$ | H | $C_2H_5$ | $CF_3$ | Fp. 88-89°C |
| 2.696 | 2-$CH_3$ | 6-$CH_3$ | H | $C_3H_7$(i) | $CF_3$ | Fp. 64-65°C |
| 2.697 | 2-F | 6-F | H | $C_4H_9$(i) | $CF_3$ | Fp. 97-99°C |
| 2.698 | 2-F | 6-F | H | 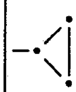 | $CF_3$ | Fp. 111-113°C |
| 2.699 | 2-F | 6-F | H | $CH_2OCH_3$ | $CF_3$ | Fp. 62-65°C |
| 2.700 | 2-F | 6-F | H | $C_2H_5$ | $CHCl_2$ | Fp. 123-125°C |
| 2.701 | 2-F | 6-F | H | 2-Furyl | $CF_3$ | Fp. 122-123°C |
| 2.702 | 2-$CH_3$ | 5-F | H | $C_2H_5$ | $CHCl_2$ | Oel |
| 2.703 | 2-Cl | 6-$CH_3$ | H | $CH_2OCH_3$ | $CF_3$ | Fp. 95-96°C |
| 2.704 | 2-Cl | 6-$CH_3$ | H | $CH_2OCH_3$ | $CF_2Cl$ | Fp. 103-105°C |
| 2.705 | 2-Cl | 6-$CH_3$ | H | $C_3H_7$(i) | $CF_2Cl$ | Fp. 60-61°C |
| 2.706 | 2-Cl | 6-$CH_3$ | H | Cl | $CF_3$ | Fp. 102°C |
| 2.707 | 2-Cl | 6-$CH_3$ | H | $CH_2OCH_3$ | $CHF_2$ | $n_D^{25}$: 1,5375 |
| 2.708 | 2-F | H | H | $CH_2OCH_3$ | $CHF_2$ | Fp. 44-45°C |
| 2.709 | 2-$OCH_3$ | 6-$CH_3$ | H | $CH_3$ | $CF_3$ | Fp. 94-95°C |
| 2.710 | 2-Cl | 6-Cl | H | Cl | $CF_3$ | Fp. 124-125°C |
| 2.711 | 2-$SCHF_2$ | H | H | $CH_3$ | $CF_3$ | Fp. 78-79°C |
| 2.712 | 2-$OCHF_2$ | 6-$CH_3$ | H | $CH_3$ | $CF_3$ | $n_D^{23}$: 1,5050 |
| 2.713 | 2-$OCHF_2$ | 6-$CH_3$ | H | $C_2H_5$ | $CF_3$ | $n_D^{23}$: 1,4910 |
| 2.714 | 2-F | 6-Cl | H | 2-Furyl | $CF_3$ | Fp. 119-120°C |
| 2.715 | 2-$CH_3$ | 6-F | H | $C_2H_5$ | $CHCl_2$ | |

H. 3. Verbindungen der Formel III

H. 3.1. N-Chlorcarbonyl-N-(4,6-dimethyl-pyrimidin-2-yl)-2,5-dichloranilin

5,0 g (0.018 Mol) N-(4,6-Dimethyl-pyrimidin-2-yl)-2,5-dichloranilin werden in 100 ml Toluol gelöst und auf Rückfluss erhitzt. Dann wird 4 Std. lang ein leichter Phosgenstrom eingeleitet. Das überschüssige Phosgen wird mit Stickstoff ausgeblasen, die Toluollösung kalt mit Wasser gewaschen, mit Magnesiumsulfat getrocknet und dann eingedampft. Man isoliert 5,8 g (95.1 %) der Titelverbindung der Formel

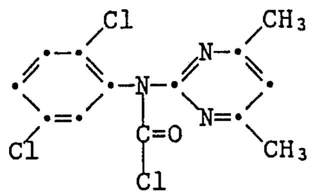

als Kristalle vom Smp. 121-123°C.

Biologische Beispiele

Beispiel B1: Pre-emergente Herbizid-Wirkung

Im Gewächshaus wird unmittelbar nach der Einsaat der Versuchspflanzen in Saatschalen die Erdoberfläche mit einer wässrigen Spritzbrühe entsprechend einer Aufwandmenge von 4 kg Wirksubstanz/Hektar behandelt. Die Saatschalen werden im Gewächshaus bei 22 - 25°C und 50 - 70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung.

In diesem Versuch zeigen unter anderem die folgenden Verbindungen der Formel I gute bis sehr gute Herbizidwirkung gegen Setaria italica und Stellaria media: Verb. No. 1.009, 1.016, 1.020, 1.021, 1.023, 1.032, 1.037, 1.038, 1.042, 1.050, 1.052, 1.055, 1.056, 1.064, 1.065, 1.096, 1.109, 1.113, 1.132, 1.141, 1.144, 1.170, 1.219, 1.221, 1.258, 1.271, 1.332, 1.346, 1.380, 1.392, 1.459, 1.501, 1.505, 1.527, 1.528, 1.540, 1.543, 1.553, 1.638, 1.691, 1.703 und 1.712.

Beispiel B2: Selektive Herbizidwirkung im Vorauflauf

Unmittelbar nach der Einsaat der Samen im Blumentöpfe von 12-15 cm Durchmesser wird die Oberfläche mit einer wässrigen Spritzbrühe, entsprechend einer Aufwandmenge von 1000 und 500 [g] AS/[ha], behandelt.

Die Töpfe werden im Gewächshaus bei einer Temperatur von 22-25°C und 50-70 % relativer Luftfeuchtigkeit belassen.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

Die Ergebnisse dieses Versuchs sind in der nachstehenden Tabelle III zusammengefasst. Dabei bedeutet T eine gute bis sehr gute Toleranz (bei der Kulturpflanze) und H eine gute bis sehr gute Herbizidwirkung (beim Unkraut).

Tabelle III

| Testpflanze | Verbindung No. 1.020 | 1.055 | 1.056 | 1.141 | 1.219 | 1.528 | 1.540 |
|---|---|---|---|---|---|---|---|
| Gerste | T | | T | T | T | T | T |
| Weizen | T | | T | | T | T | T |
| Mais | T | T | T | T | T | T | T |
| Soja | T | T | T | T | T | T | T |
| Baumwolle | T | T | T | T | T | T | T |
| Sonnenblume | T | T | T | T | T | T | T |
| Raps | | | | | | | T |
| Lolium perenne | | H | H | | | | H |
| Alopecurus Myos. | | H | | | | | H |
| Digitaria sang. | H | H | | H | H | H | H |
| Echinochloa crus galli | H | H | | H | | H | H |
| Sorghum halep. | | | | H | H | H | H |
| Chenopodium Sp. | H | H | H | | H | H | H |
| Solanum nigrum | | | | H | H | | H |
| Stellaria | H | H | H | H | | H | H |
| Viola tricolor | | | | H | H | H | H |
| Galuim aparine | H | H | | | | | H |
| Veronica Sp. | H | H | H | H | H | H | H |

Beispiel B3: Herbizidwirkung für Wasserreis (verpflanzt)

Wasserunkräuter werden in Plastikgefässen (425 $cm^2$ Oberfläche, 5,0 1 Volumen) ausgesät. Zusätzlich wird Reis im Dreiblattstadium verpflanzt (verpflanzter Reis). Nach dem Verpflanzen wird bis zur Erdoberfläche mit Wasser aufgefüllt. 3 Tage nach der Saat wird der Wasserspiegel bis leicht über die Erdoberfläche erhöht (3-5 mm). Die Applikation der Prüfsubstanz erfolgt 3 Tage nach dem Verpflanzen in einer Aufwandmenge von 500 und 1000 [g/ha] durch Injektion einer wässrigen Emulsion in das Wasser (das Applikationsvolumen entspricht dabei 1400 l/ha). Die Pflanzgefässe werden dann im Gewächshaus unter optimalen Wachstumsbedingungen für die Einsaat aufgestellt, d.h. bei 25°-30°C und hoher Luftfeuchtigkeit.

Die Versuche werden drei Wochen nach Applikation in einem neunstufigen Bonitierungsschema im Vergleich zur unbehandelten Kontrolle ausgewertet.

Boniturnoten von 1 bis 4 (insbesondere von 1 bis 3) weisen auf eine gute bis sehr gute Herbizidwirkung hin. Boniturnoten von 6 bis 9 (insbesondere 7 bis 9) weisen auf eine gute Toleranz (insbesondere bei dem Reis) hin.

In diesem Test zeigen die Verbindungen 1.009, 1.096 und 1.141 sehr gute Herbizidwirkung gegen Echinochola crus galli, bei gleichzeitig sehr guter Toleranz durch den Reis. Darüber hinaus zeigt Verbindung 1.141 noch sehr gute Herbizidwirkung gegen Monocharia.

Beispiel B4: Wuchshemmung bei Getreide

Die Pflanzen (z.B. Sommergerste der Sorte Iban) werden in 15 cm-Kunststofftöpfen mit steriler Landerde angesät und in der Klimakammer bei einer Tagestemperatur von 10-15°C und einer Nachttemperatur von 5-10°C angezogen. Die Beleuchtungsdauer ist 13.5 Stunden pro Tag bei einer Intensität von ca. 25000 Lux.

Ca. 34 Tage nach der Saat und dem Ausdünnen auf 4 Pflanzen/Topf erfolgt die Applikation mit 0,3 bis 3 kg Wirkstoff/ha, in der Regel 25%-ig formuliert und in wässeriger Spritzbrühe. Die Wasseraufwandmenge ist ca. 500 l/ha. Nach der Applikation werden die Pflanzen im Gewächshaus bei einer Tagestemperatur von mindestens 10°C aufgestellt. Die Beleuchtungsdauer ist mindestens 13,5 Stunden/Tag.

Ca. 28 Tage nach der Behandlung findet die Auswertung statt. Hierbei wird die Höhe des Neuzuwachses dargestellt.

Die geprüften Verbindungen der Formel I bewirken eine Reduzierung des Neuzuwachses im Vergleich zur unbehandelten Kontrolle.

Beispiel B5: Wuchshemmung bei Gräsern mit Klee

Eine Mischung von Gräsern (z.B. Poa, Festuca, Lolium, Bromus, Cynosurus) und Klee (Trifolium pratense/repens) wird in 15 cm-Kunststofftöpfen mit steriler Landerde angesät und im Gewächshaus bei einer Tagestemperatur von 21° C und einer Nachttemperatur von 17° C angezogen. Die Beleuchtungsdauer ist 13,5 Stunden/Tag bei einer Lichtintensität von mindestens 7000 Lux. Nach dem Auflauf werden die Pflanzen wöchentlich auf ca. 6 cm Höhe zurückgeschnitten. Ca. 42 Tage nach der Saat und 1 Tag nach dem letzten Schnitt erfolgt die Applikation mit 0,3 bis 3 kg Wirkstoff/ha, in der Regel 25%-ig formuliert und in wässriger Spritzbrühe. Die Wasseraufwandmenge ist ca. 500 l/ha. Ca. 3 Wochen nach der Behandlung findet die Auswertung statt. Hierbei wird die Höhe des Neuzuwachses gemessen.

Die geprüften Verbindungen der Formel I bewirken eine Reduktion des Neuzuwachses im Vergleich zu unbehandelten Kontrolle.

Formulierungsbeispiele

Beispiel F1: Formulierungsbeispiele für Wirkstoffe der Formel I
(% = Gewichtsprozent)

a) Emulsionskonzentrate

| | a) | b) | c) |
|---|---|---|---|
| Wirkstoff gemäss Herstellungsbeispiel 1 | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenol-polyäthylenglykoläther (30 mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

b) Lösungen

| | a) | b) | c) |
|---|---|---|---|
| Wirkstoff gem. Herstellungsbeispiel 1 | 80 % | 10 % | 5 % |
| Aethylenglykol-monomethyläther | 20 % | - | - |
| Polyäthylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

c) Granulate

| | a) | b) |
|---|---|---|
| Wirkstoff gemäss Herstellungsbeispiel 1 | 5 % | 10 % |
| Kaolin | 94 % | - |
| Hochdisperse Kieselsäure | 1 % | |
| Attapulgit | - | 90 % |

Eine Wirkstofflösung wird auf Träger ausgesprüht und das Lösungsmittel anschliessend im Vakuum abgedampft.

d) Stäubemittel

| | a) | b) |
|---|---|---|
| Wirkstoff gemäss Herstellungsbeispiel 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertiges Stäubemittel

e) Spritzpulver

| | a) | b) |
|---|---|---|
| Wirkstoff gemäss Herstellungsbeispiel 1 | 20 % | 60 % |
| Na-Ligninsulfonat | 5 % | 5 % |
| Na-Laurylsulfat | - | 6 % |
| Octylphenol-polyäthylenglykoläther (7-8 Mol Ae) | - | 2 % |
| Hochdisperse Kieselsäure | 5 % | 27 % |
| Kaolin | 70 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

f) Extruder Granulat

| | |
|---|---|
| Wirkstoff gemäss Herstellungsbeispiel 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

g) Umhüllungs-Granulat

| | |
|---|---|
| Wirkstoff gemäss Herstellungsbeispiel 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

| | |
|---|---|
| Wirkstoff gemäss Herstellungsbeispiel 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | ad 100 % |

Der Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Patentansprüche**

1. Harnstoffe der Formel I

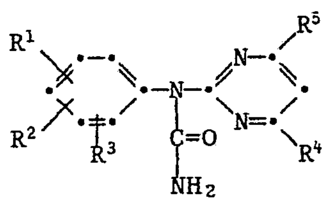

(I),

worin
$R^1$, $R^2$, und $R^3$ unabhängig voneinander Wasserstoff; Halogen; $C_1$-$C_4$-Alkyl; $C_1$-$C_4$-Alkoxy; $C_1$-$C_4$-Halogenalkyl; $C_1$-$C_4$-Halogenalkoxy; $C_1$-$C_4$-Alkyl-$S(O)_n$-; Nitro; Cyano; $C_1$-$C_4$-Alkoxycarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-$S(O)_n$-; $C_1$-$C_4$-Alkylcarbonyl; oder -PO[O-($C_1$-$C_4$)-Alkyl]$_2$;
$R^4$ $C_1$-$C_6$-Alkyl; $C_1$-$C_4$-Alkoxy; $C_1$-$C_4$-Alkyl-$S(O)_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituierten oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;
$R^5$ Wasserstoff; $C_1$-$C_3$-Alkyl; $C_1$-$C_3$-Halogenalkyl; Halogen; oder $C_1$-$C_3$-Halogenalkoxy; und
n 0, 1 oder 2
bedeutet, mit der Massgabe, dass, wenn einer der Reste $R^1$, $R^2$ oder $R^3$ Nitro bedeutet, dieser Substituent nicht in 2- oder 6-Stellung des Phenylringes gebunden sein darf, sowie Salze und Additionsverbindungen der Verbindungen der Formel I mit Säuren, Basen und Komplexbildnern.

2. Harnstoffe gemäss Anspruch 1, worin
$R^1$ Halogen; Cyano; $C_1$-$C_3$-Alkoxy; $C_1$-$C_2$-Halogenalkoxy; Methyl-$S(O)_n$-; $C_1$-$C_3$-Alkyl; $C_1$-$C_2$-Halogenalkyl; $C_1$-$C_4$-Alkoxycarbonyl; Carbamoyl; Difluormethylthio oder -PO[O-($C_1$-$C_2$)Alkyl]$_2$;
$R^2$ Wasserstoff; Fluor; Chlor; Brom; Cyano; Nitro; $C_1$-$C_3$-Alkyl; $C_1$-$C_2$-Halogenalkyl; oder $C_1$-$C_3$-Alkoxycarbonyl;
$R^3$ Wasserstoff; Chlor; Fluor; oder $C_1$-$C_3$-Alkyl;
$R^4$ $C_1$-$C_5$-Alkyl; $C_1$-$C_4$-Alkoxy; $C_1$-$C_4$-Alkylthio; Cyclopropyl; Phenyl; Furan-2-yl; Thiophen-2-yl; Cyano; $C_1$-$C_3$-Halogenalkoxy; $C_1$-$C_2$-Alkoxy-$C_1$-$C_2$-alkyl; $C_1$-$C_2$-Alkoxy-$C_1$-$C_2$-alkoxy; Methylsulfinyl; Methylsulfonyl; oder $C_2$-$C_3$-Halogenalkenyl;
$R^5$ $C_1$-$C_3$-Alkyl; Fluor; Chlor; Brom; $C_1$-$C_3$-Halogenalkyl; oder $C_1$-$C_3$-Halogenalkoxy; und
n 0, 1 oder 2
bedeutet.

3. Harnstoffe gemäss Anspruch 1 oder 2, worin
$R^1$ Halogen; Methyl; Trifluormethyl; Trifluormethoxy; Difluormethoxy; $C_1$-$C_3$-Alkoxy; Methylthio; Methylsulfinyl; Methylsulfonyl; Cyano; $C_1$-$C_4$-Alkoxycarbonyl; Carbamoyl; Difluormethylthio; oder

$-PO(O-C_2H_5)_2$;
$R^2$ Wasserstoff; Fluor; Chlor; Brom; Nitro; Ethyl; Methyl; Trifluormethyl; Methoxy; oder $C_1$-$C_3$-Alkoxycarbonyl;
$R^3$ Wasserstoff; Chlor; oder Methyl;
$R^4$ $C_1$-$C_5$-Alkyl; $C_1$-$C_4$-Alkoxy; $C_1$-$C_4$-Alkylthio; Cyclopropyl; Phenyl; Furan-2-yl; Thiophen-2-yl; Cyano; 1,1,2,2-Tetrafluorethoxy; 2-Chlorethoxy; Methoxymethyl; 2-Methoxy-ethoxy; Methylsulfinyl; Methylsulfonyl; oder 2,2-Dichlorvinyl;
$R^5$ $C_1$-$C_3$-Alkyl; Fluor; Chlor; Brom; Difluormethoxy; Trifluormethyl; Pentafluorethyl; Chlordifluormethyl; Difluormethyl; Dichlormethyl; Chlorfluormethyl; 1,1-Dichlor-2,2,2-trifluorethyl; 1,1-Dichlorethyl; oder Heptafluorpropyl;
bedeutet.

4. Harnstoffe gemäss Anspruch 1, worin
$R^1$ in Position 2 des Phenylringes gebundenes Fluor, Chlor, Brom, Trifluormethyl, Difluormethoxy, Trifluormethoxy, Cyano oder Methoxy,
$R^2$ in Position 3, 5 oder 6 des Phenylringes gebundenes Fluor oder Chlor,
$R^4$ Methyl, Furan-2-yl oder Cyclopropyl, und
$R^5$ Chlor, Methyl, Trifluormethyl oder Chlordifluormethyl, oder Chlordifluormethyl
bedeutet.

5. Harnstoffe gemäss Anspruch 1, worin
$R^1$ in Position 3 des Phenylringes gebundenes Chlor,
$R^2$ in Position 5 des Phenylringes gebundenes Chlor,
$R^4$ Methyl, Furan-2-yl oder Cyclopropyl, und
$R^5$ Chlor, Methyl Trifluormethyl oder Chlordifluormethyl bedeutet.

6. N-(2-Bromphenyl)-N-(4-chlor-6-methyl-pyrimidin-2-yl)-harnstoff,
N-(4-Methyl-6-trifluormethyl-pyrimidin-2-yl)-N-(2-trifluormethylphenyl)-harnstoff,
N-(2,3-Dichlorphenyl)-N-(4-methyl-6-trifluormethyl-pyrimidin-2-yl)-harnstoff,
N-(2,5-Dichlorphenyl)-N-(4,6-dimethyl-pyrimidin-2-yl)-harnstoff,
N-(4-Chlor-6-methyl-pyrimidin-2-yl)-N-(2,5-dichlorphenyl)-harnstoff,
N-(2,5-Dichlorphenyl)-N-(4-methyl-6-trifluormethyl-pyrimidin-2-yl)-harnstoff,
N-[4-(Chlordifluormethyl)-6-methyl-pyrimidin-2-yl]-N-(2,5-dichlorphenyl)-harnstoff,
N-(4-Chlor-6-methyl-pyrimidin-2-yl)-N-(2,6-dichlorphenyl)-harnstoff,
N-(2,6-Dichlorphenyl)-N-(4-methyl-6-trifluormethyl-pyrimidin-2-yl)-harnstoff,
N-(4-Cyclopropyl-6-trifluormethyl-pyrimidin-2-yl)-N-(2,6-dichlorphenyl)-harnstoff,
N-(3,5-Dichlorphenyl)-N-(4-methyl-6-trifluormethyl-pyrimidin-2-yl)-harnstoff,
N-(5-Chlor-2-methyl-phenyl)-N-(4-chlor-6-methyl-pyrimidin-2-yl)-harnstoff,
N-(5-Chlor-2-methoxyphenyl)-N-(4-methyl-6-trifluormethyl-pyrimidin-2-yl)-harnstoff,
N-(2-Jodphenyl)-N-(4-methyl-6-trifluormethylpyrimidin-2-yl)-harnstoff,
N-(2-Chlor-6-methylphenyl)-N-(4-chlor-6-methyl-pyrimidin-2-yl)-harnstoff,
N-(3-Chlor-2-methoxyphenyl)-N-(4-methyl-6-trifluormethylpyrimidin-2-yl)-harnstoff,
N-(2,6-Difluorphenyl)-N-(4-methyl-6-trifluormethylpyrimidin-2-yl)-harnstoff,
N-(2,5-Difluorphenyl)-N-(4-methyl-6-trifluormethyl-pyrimidin-2-yl)-harnstoff,
N-(2-Cyanophenyl)-N-(4-methyl-6-trifluormethyl-pyrimidin-2-yl)-harnstoff,
N-(2-Bromphenyl)-N-(4-cyclopropyl-6-trifluormethyl-pyrimidin-2-yl)-harnstoff,
N-(4-Isopropyl-6-trifluormethyl-pyrimidin-2-yl)-N-2-(trifluoromethylphenyl)-harnstoff,
N-(2-Fluorphenyl)-N-(4-methyl-6-trifluormethyl-pyrimidin-2-yl)-harnstoff,
N-(2-Fluorphenyl)-N-(4-isopropyl-6-trifluormethyl-pyrimidin-2-yl)-harnstoff,
N-(2,5-Dichlorphenyl)-N-(4-ethyl-6-trifluormethyl-pyrimidin-2-yl)-harnstoff,
N-(2,6-Dichlorphenyl)-N-(4-difluormethyl-6-methyl-pyrimidin-2-yl)-harnstoff,
N-(2,6-Dichlorphenyl)-N-(4-methylthio-6-trifluormethyl-pyrimidin-2-yl)-harnstoff,
N-(2,6-Dichlor-3-methyl)-N-(4-methyl-6-trifluor-methyl-pyrimidin-2-yl)-harnstoff,
N-(2,6-Dichlor-3-methylphenyl)-N-(4-isopropyl-6-trifluormethyl-pyrimidin-2-yl)-harnstoff,
N-(2-Chlor-6-trifluormethyl-phenyl)-N-(4-methyl-6-trifluormethyl-pyrimidin-2-yl)-harnstoff,
N-(2-Chlor-6-methyl-phenyl)-N-(4-methyl-6-trifluormethyl-pyrimidin-2-yl)-harnstoff,
N-(2-Methyl-6-trifluormethyl-phenyl)-N-(4-methyl-6-trifluormethyl-pyrimidin-2-yl)-harnstoff,
N-(5-Chlor-2-difluormethoxy-phenyl)-N-(4-methyl-6-trifluormethyl-pyrimidin-2-yl)-harnstoff,
N-(2-Methylthio-phenyl)-N-(4-methyl-6-trifluormethyl-pyrimidin-2-yl)-harnstoff,
N-(4-Cyclopropyl-6-trifluormethyl-pyrimidin-2-yl)-N-(2,5-difluorphenyl)-harnstoff,
N-(2,6-Difluorphenyl)-N-(4-ethyl-6-trifluormethyl-pyrimidin-2-yl)-harnstoff,
N-(6-Chlor-2-fluor-phenyl)-N-(4-methyl-6-trifluormethyl-pyrimidin-2-yl)-harnstoff,
N-(6-Chlor-2-fluor-phenyl)-N-(4-cyclopropyl-6-trifluormethyl-pyrimidin-2-yl)-harnstoff,
N-(2-Difluormethoxyphenyl)-N-(4-methyl-6-trifluormethyl-pyrimidin-2-yl)-harnstoff,
N-(6-Chlor-2-methoxycarbonyl-phenyl)-N-(4-methyl-6-trifluormethyl-pyrimidin-2-yl)-harnstoff,
N-(2-Bromphenyl)-N-[4-(furan-2-yl)-6-trifluormethyl-pyrimidin-2-yl]-harnstoff,
N-(2-Chlor-6-methyl-phenyl)-N-(4-methoxymethyl-6-trifluormethyl-pyrimidin-2-yl)-harnstoff,
oder

N-(2-Difluormethoxy-6-methyl-phenyl)-N-(4-methyl-6-trifluormethyl-pyrimidin-2-yl)-harnstoff, als Verbindung der Formel I gemäss Anspruch 1.

7. Verfahren zur Herstellung von Harnstoffen gemäss einem der Ansprüche 1 bis 6, dadurch gekennzeichnet, dass man.

a) ein Anilin der Formel II mit Phosgen zu einem Carbaminchlorid der Formel III umsetzt und dieses in einer zweiten Stufe mit $NH_3$ zu

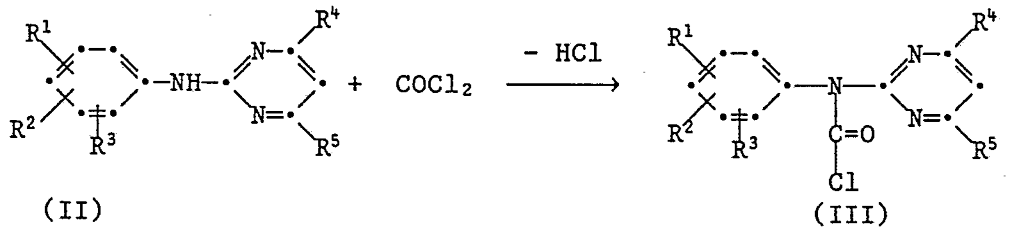


(II) (III)

$$III + NH_3 \xrightarrow{-\ HCl} I$$

einem Harnstoff der Formel I reagieren lässt oder

b) ein Anilin der Formel II mit Halogensulfonylisocyanat IX zu einem Halogensulfonylharnstoff der Formel IV umsetzt

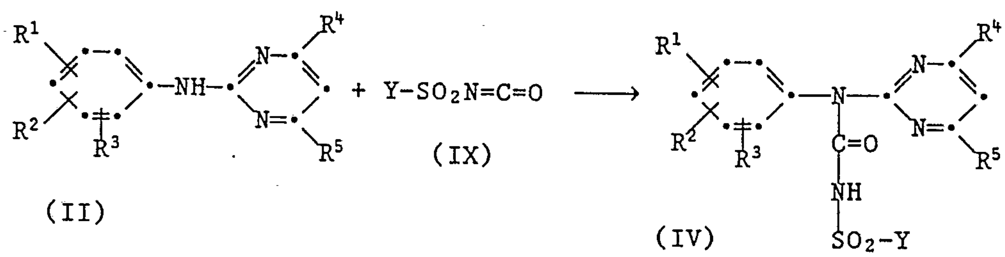


(II) (IX) (IV)

$$IV + 2H_2O \xrightarrow[-\ SO_4H_2]{-\ HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor, steht.

8. Aniline der Formel II

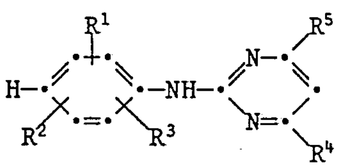

II

worin

$R^1$ Halogen; Cyano; $C_1$-$C_4$-Alkoxy; $C_1$-$C_4$-Halogenalkoxy; $C_1$-$C_4$-Alkyl-$S(O)_n$-; $C_1$-$C_4$-Alkyl; $C_1$-$C_4$-Halogenalkyl; $C_1$-$C_4$-Alkoxycarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-$S(O)_n$-; oder -PO[O-($C_1$-$C_4$)-Alkyl]$_2$;

$R^2$ Wasserstoff; Halogen; Cyano; Nitro; $C_1$-$C_4$-Alkyl; $C_1$-$C_4$-Alkoxy; $C_1$-$C_4$-Halogenalkyl; $C_1$-$C_4$-Alkoxycarbonyl; oder $C_1$-$C_4$-Alkylcarbonyl;

$R^3$ Wasserstoff; Halogen; oder $C_1$-$C_4$-Alkyl;

$R^4$ $C_1$-$C_6$-Alkyl; $C_1$-$C_4$-Alkoxy; $C_1$-$C_4$-Alkyl-$S(O)_n$-; $C_1$-$C_4$-Halogenalkyl; $C_1$-$C_4$-Halogenalkoxy; unsubstituiertes oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituiertes oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;

$R^5$ Wasserstoff; $C_1$-$C_3$-Alkyl; $C_1$-$C_3$-Halogenalkyl; Halogen; oder $C_1$-$C_3$-Halogenalkoxy; und

n 0, 1 oder 2

bedeutet, mit der Massgabe, dass a) wenn einer der Reste $R^2$ oder $R^3$ Nitro bedeutet, dieser Substituent nicht in 2- oder 6-Stellung des Phenylringes gebunden sein darf und, wenn die Reste $R^4$ und $R^5$ für Methyl stehen $R^1$ nicht für Chlor, Methoxy, Ethoxy, Fluor, Jod, Methyl oder Brom steht und dass ausserdem folgende Einzelverbindungen nicht mit umfasst sind: N-[(4,6-Bis-trifluormethyl)-pyrimidin-2-yl]-2,6-dichloranilin und N-(4-Chlor-6-methylpyrimidin-2-yl)-3-chloranilin.

9. Carbamoylchloride der Formel III

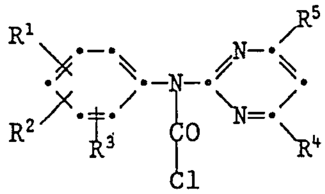

(III),

worin

$R^1$, $R^2$, und $R^3$ unabhängig voneinander Wasserstoff; Halogen; $C_1$-$C_4$-Alkyl; $C_1$-$C_4$-Alkoxy; $C_1$-$C_4$-Halogenalkyl; $C_1$-$C_4$-Halogenalkoxy; $C_1$-$C_4$-Alkyl-S(O)$_n$-; Nitro; Cyano; $C_1$-$C_4$-Alkoxycarbonyl; $C_1$-$C_4$-Alkylcarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-S(O)$_n$-; oder -PO[O-($C_1$-$C_4$)-Alkyl]$_2$;

$R^4$ $C_1$-$C_6$-Alkyl; $C_1$-$C_4$-Alkoxy; $C_1$-$C_4$-Alkyl-S(O)$_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituierten oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;

$R^5$ Wasserstoff; $C_1$-$C_3$-Alkyl; $C_1$-$C_3$-Halogenalkyl; Halogen; oder $C_1$-$C_3$-Halogenalkoxy; und

n 0, 1 oder 2

bedeutet, mit der Massgabe, dass, wenn einer der Reste $R^1$, $R^2$ oder $R^3$ Nitro bedeutet, dieser Substituent nicht in 2- oder 6-Stellung des Phenylringes gebunden sein darf.

10. Halogensulfonylharnstoffe der Formel IV

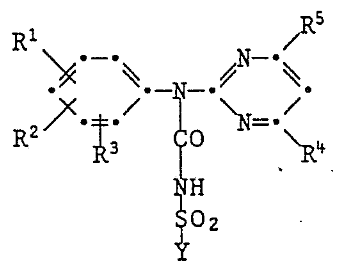

(IV),

worin

Y Halogen; $C_1$-$C_4$-Alkoxy; oder Phenoxy;

$R^1$, $R^2$, und $R^3$ unabhängig voneinander Wasserstoff; Halogen; $C_1$-$C_4$-Alkyl; $C_1$-$C_4$-Alkoxy; $C_1$-$C_4$-Halogenalkyl; $C_1$-$C_4$-Halogenalkoxy; $C_1$-$C_4$-Alkyl-S(O)$_n$-; Nitro; Cyano; $C_1$-$C_4$-Alkoxycarbonyl; $C_1$-$C_4$-Alkylcarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-S(O)$_n$-; oder -PO[O-($C_1$-$C_4$)-Alkyl]$_2$;

$R^4$ $C_1$-$C_6$-Alkyl; $C_1$-$C_4$-Alkoxy; $C_1$-$C_4$-Alkyl-S(O)$_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituierten oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;

$R^5$ Wasserstoff; $C_1$-$C_3$-Alkyl; $C_1$-$C_3$-Halogenalkyl; Halogen; oder $C_1$-$C_3$-Halogenalkoxy; und

n 0, 1 oder 2

bedeutet, mit der Massgabe, dass, wenn einer der Reste $R^1$, $R^2$ oder $R^3$ Nitro bedeutet, dieser Substituent nicht in 2- oder 6-Stellung des Phenylringes gebunden sein darf.

11. Verfahren zur Herstellung von Anilinen der Formel II gemäss Anspruch 8 durch

a) Umsetzung von Guanidinen der Formel V mit 1,3-Dicarbonylverbindungen der Formel VI

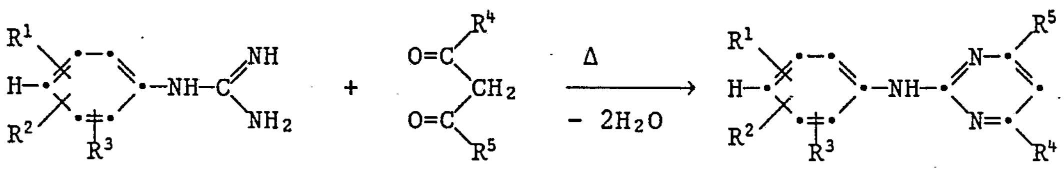


(V) (VI) (II)

wobei die Kondensationsreaktion gewünschtenfalls in Gegenwart wasserbindender Mittel durchgeführt wird oder

b) Umsetzung eines Anilins der Formel VII mit einem Pyrimidin der Formel VIII unter Baseneinwirkung

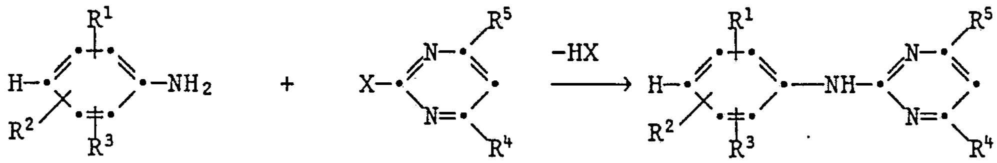


VII VIII II

worin die Reste $R^1$ bis $R^5$ wie zuvor definiert sind und X für eine nucleofuge Gruppe, wie Halogen, $C_1$-$C_4$-Alkylsulfonyl oder Phenylsulfonyl, steht.

12. Verfahren zur Herstellung von Carbamoylchloriden der Formel III gemäss Anspruch 9, dadurch gekennzeichnet, dass man ein Anilin der Formel II

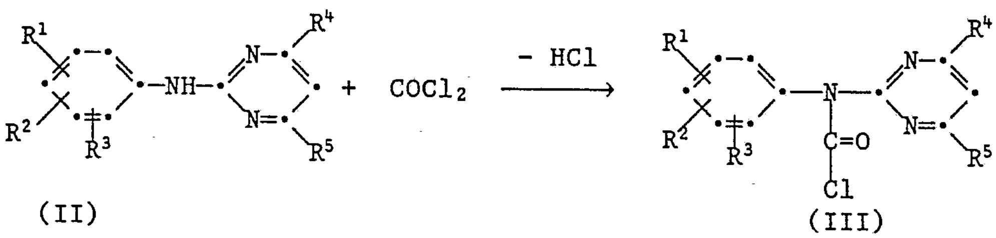


(II) (III)

worin die Reste $R^1$ bis $R^5$ wie unter Formel II definiert sind, mit Phosgen umsetzt.

13. Verfahren zur Herstellung eines Halogensulfonylharnstoffs der Formel IV gemäss Anspruch 10, dadurch gekennzeichnet, dass man ein Anilin der Formel II

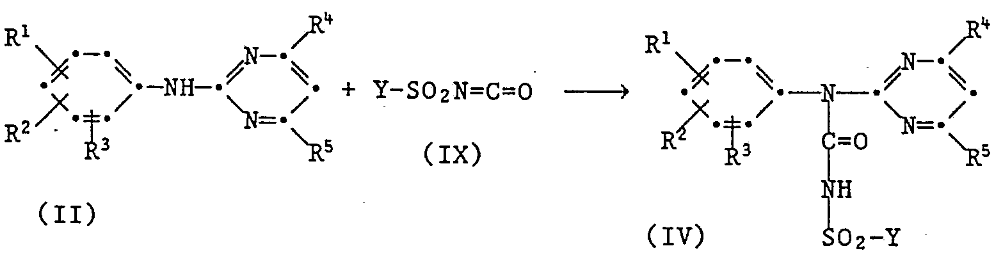


(II) (IX) (IV)

worin die Reste $R^1$ bis $R^5$ wie unter Formel IV definiert sind, mit einem Halogensulfonylisocyanat IX, worin Y Halogen bedeutet, umsetzt.

14. Herbizides Mittel, enthaltend als Wirksubstanz eine Verbindung der Formel I, gemäss einem der Ansprüche 1 bis 6, neben weiteren Hilfs-und/oder Trägerstoffen.

15. Pflanzenwuchsregulatorisches Mittel, enthaltend als Wirksubstanz eine Verbindung der Formel I, gemäss einem der Ansprüche 1 bis 6, neben weiteren Hilfs- und/oder Trägerstoffen.

16. Verfahren zur Bekämpfung unerwünschten Pflanzenwuchses, dadurch gekennzeichnet, dass man eine herbizid wirksame Menge einer Verbindung, gemäss einem der Ansprüche 1 bis 6, oder eines Mittels gemäss Anspruch 14 auf die zu bekämpfende Pflanze oder deren Lebensraum einwirken lässt.

17. Verfahren zur Beeinflussung des Pflanzenwuchses, dadurch gekennzeichnet, dass man eine pflanzenwuchsregulatorisch wirksame Menge einer Verbindung, gemäss einem der Ansprüche 1 bis 6 oder eines Mittels, gemäss Anspruch 15 auf die Pflanze oder deren Lebensraum einwirken lässt.

**Patentansprüche für folgenden Vertragsstaat: ES**

1. Verfahren zur Herstellung von Harnstoffen der Formel I

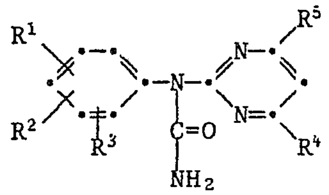

(I),

worin

$R^1$, $R^2$, und $R^3$ unabhängig voneinander Wasserstoff; Halogen; $C_1$-$C_4$-Alkyl; $C_1$-$C_4$-Alkoxy; $C_1$-$C_4$-Halogenalkyl; $C_1$-$C_4$-Halogenalkoxy; $C_1$-$C_4$-Alkyl-S(O)$_n$-; Nitro; Cyano; $C_1$-$C_4$-Alkoxycarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-S(O)$_n$-; Alkylcarbonyl; oder -PO[O-($C_1$-$C_4$)-Alkyl]$_2$;

$R^4$ $C_1$-$C_6$-Alkyl; $C_1$-$C_4$-Alkoxy; $C_1$-$C_4$-Alkyl-S(O)$_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituierten oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;

$R^5$ Wasserstoff; $C_1$-$C_3$-Alkyl; $C_1$-$C_3$-Halogenalkyl; Halogen; oder $C_1$-$C_3$-Halogenalkoxy; und

n 0, 1 oder 2

bedeutet, mit der Massgabe, dass, wenn einer der Reste $R^1$, $R^2$ oder $R^3$ Nitro bedeutet, dieser Substituent nicht in 2- oder 6-Stellung des Phenylringes gebunden sein darf, sowie Salze und Additionsverbindungen der Verbindungen der Formel I mit Säuren, Basen und Komplexbildnern, dadurch gekennzeichnet, dass man

a) ein Anilin der Formel II mit Phosgen zu einem Carbaminchlorid der Formel III umsetzt und dieses in einer zweiten Stufe mit $NH_3$ zu

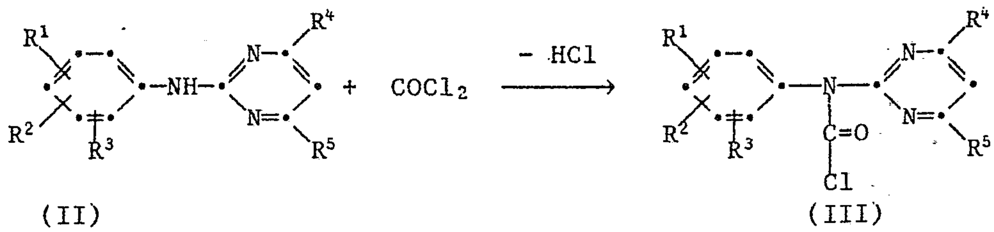


(II) (III)

$$III + NH_3 \xrightarrow{-\ HCl} I$$

einem Harnstoff der Formel I reagieren lässt oder

b) ein Anilin der Formel II mit Halogensulfonylisocyanat IX zu einem Halogensulfonylharnstoff der Formel IV umsetzt

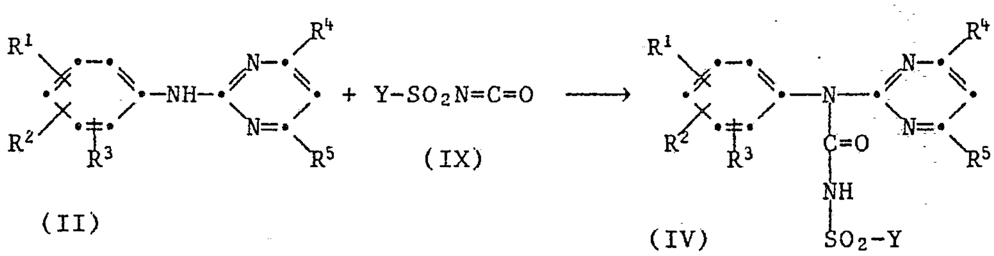


(II) (IX) (IV)

$$IV + 2H_2O \xrightarrow[-\ SO_4H_2]{-\ HY} I$$

und diesen in einer zweiten Stufe oder direkt zu einer Verbindung der Formel I hydrolisiert, wobei Y für eine unter den Reaktionsbedingungen abspaltbare Gruppe wie Halogen, vorzugsweise Chlor, steht und gewünschtenfalls die so erhaltene Verbindung der Formel I mit einer Säure, einer Base oder einem Komplexbildner zu einem Salz oder einem Komplex umsetzt.

2. Verfahren nach Anspruch 1 zur Herstellung von Harnstoffen der Formel I worin
$R^1$ Halogen; Cyano; $C_1$-$C_3$-Alkoxy; $C_1$-$C_2$-Halogenalkoxy; Methyl-S(O)$_n$-; $C_1$-$C_3$-Alkyl; $C_1$-$C_2$-Halogenalkyl; $C_1$-$C_4$-Alkoxycarbonyl; Carbamoyl; Difluormethylthio; oder -PO[O-($C_1$-$C_2$)Alkyl]$_2$;
$R^2$ Wasserstoff; Fluor; Chlor; Brom; Cyano; Nitro; $C_1$-$C_3$-Alkyl; $C_1$-$C_2$-Halogenalkyl; oder $C_1$-$C_3$-Alkoxycarbonyl;
$R^3$ Wasserstoff; Chlor; Fluor; oder $C_1$-$C_3$-Alkyl;
$R^4$ $C_1$-$C_5$-Alkyl; $C_1$-$C_4$-Alkoxy; $C_1$-$C_4$-Alkylthio; Cyclopropyl; Phenyl; Furan-2-yl; Thiophen-2-yl; Cyano; $C_1$-$C_3$-Halogenalkoxy; $C_1$-$C_2$-Alkoxy-$C_1$-$C_2$-alkyl; $C_1$-$C_2$-Alkoxy-$C_1$-$C_2$-alkoxy; Methylsulfinyl; Methylsulfonyl; oder $C_2$-$C_3$-Halogenalkenyl;
$R^5$ $C_1$-$C_3$-Alkyl; Fluor; Chlor; Brom; $C_1$-$C_3$-Halogenalkyl; oder $C_1$-$C_3$-Halogenalkoxy; und
n 0, 1 oder 2
bedeutet.

3. Verfahren nach Anspruch 1 zur Herstellung von Harnstoffen der Formel I, worin
$R^1$ Halogen; Methyl; Trifluormethyl; Trifluormethoxy; Difluormethoxy; $C_1$-$C_3$-Alkoxy; Methylthio; Methylsulfinyl; Methylsulfonyl; Cyano; $C_1$-$C_4$-Alkoxycarbonyl; Carbamoyl; Difluormethylthio; oder -PO(O-$C_2H_5$)$_2$;
$R^2$ Wasserstoff; Fluor; Chlor; Brom; Nitro; Ethyl; Methyl; Trifluormethyl; Methoxy; oder $C_1$-$C_3$-Alkoxycarbonyl;
$R^3$ Wasserstoff; Chlor; oder Methyl;
$R^4$ $C_1$-$C_5$-Alkyl; $C_1$-$C_4$-Alkoxy; $C_1$-$C_4$-Alkylthio; Cyclopropyl; Phenyl; Furan-2-yl; Thiophen-2-yl; Cyano; 1,1,2,2-Tetrafluorethoxy; 2-Chlorethoxy; Methoxymethyl; 2-Methoxy-ethoxy; Methylsulfinyl; Methylsulfonyl; oder 2,2-Dichlorvinyl;
$R^5$ $C_1$-$C_3$-Alkyl; Fluor; Chlor; Brom; Difluormethoxy; Trifluormethyl; Pentafluorethyl; Chlordifluormethyl; Difluormethyl; Dichlormethyl; Chlorfluormethyl; 1,1-Dichlor-2,2,2-trifluorethyl; 1,1-Dichlorethyl; oder Heptafluorpropyl;
bedeutet.

4. Verfahren nach Anspruch 1 zur Herstellung von Harnstoffen der Formel I, worin
$R^1$ in Position 2 des Phenylringes gebundenes Fluor, Chlor, Brom, Trifluormethyl, Difluormethoxy, Trifluormethoxy, Cyano oder Methoxy,
$R^2$ in Position 3, 5 oder 6 des Phenylringes gebundenes Fluor oder Chlor,
$R^4$ Methyl, Furan-2-yl oder Cyclopropyl, und
$R^5$ Chlor, Methyl, Trifluormethyl oder Chlordifluormethyl,
bedeutet.

5. Verfahren nach Anspruch 1 zur Herstellung von Harnstoffen der Formel I, worin
$R^1$ in Position 3 des Phenylringes gebundenes Chlor,
$R^2$ in Position 5 des Phenylringes gebundenes Chlor,
$R^4$ Methyl, Furan-2-yl oder Cyclopropyl, und
$R^5$ Chlor, Methyl Trifluormethyl oder Chlordifluormethyl bedeutet.

6. Verfahren nach Anspruch 1 zur Herstellung von N-(2-Bromphenyl)-N-(4-chlor-6-methyl-pyrimidin-2-yl)-harnstoff,
N-(4-Methyl-6-trifluormethyl-pyrimidin-2-yl)-N-(2-trifluormethylphenyl)-harnstoff,
N-(2,3-Dichlorphenyl)-N-(4-methyl-6-trifluormethyl-pyrimidin-2-yl)-harnstoff,
N-(2,5-Dichlorphenyl)-N-(4,6-dimethyl-pyrimidin-2-yl)-harnstoff,
N-(4-Chlor-6-methyl-pyrimidin-2-yl)-N-(2,5-dichlorphenyl)-harnstoff,
N-(2,5-Dichlorphenyl)-N-(4-methyl-6-trifluormethyl-pyrimidin-2-yl)-harnstoff,
N-[4-(Chlordifluormethyl)-6-methyl-pyrimidin-2-yl]-N-(2,5-dichlorphenyl)-harnstoff,
N-(4-Chlor-6-methyl-pyrimidin-2-yl)-N-(2,6-dichlorphenyl)-harnstoff,
N-(2,6-Dichlorphenyl)-N-(4-methyl-6-trifluormethyl-pyrimidin-2-yl)-harnstoff,
N-(4-Cyclopropyl-6-trifluormethyl-pyrimidin-2-yl)-N-(2,6-dichlorphenyl)-harnstoff,
N-(3,5-Dichlorphenyl)-N-(4-methyl-6-trifluormethyl-pyrimidin-2-yl)-harnstoff,
N-(5-Chlor-2-methyl-phenyl)-N-(4-chlor-6-methyl-pyrimidin-2-yl)-harnstoff,
N-(5-Chlor-2-methoxyphenyl)-N-(4-methyl-6-trifluormethyl-pyrimidin-2-yl)-harnstoff,
N-(2-Jodphenyl)-N-(4-methyl-6-trifluormethylpyrimidin-2-yl)-harnstoff,
N-(2-Chlor-6-methylphenyl)-N-(4-chlor-6-methyl-pyrimidin-2-yl)-harnstoff,
N-(3-Chlor-2-methoxyphenyl)-N-(4-methyl-6-trifluormethylpyrimidin-2-yl)-harnstoff,
N-(2,6-Difluorphenyl)-N-(4-methyl-6-trifluormethylpyrimidin-2-yl)-harnstoff,
N-(2,5-Difluorphenyl)-N-(4-methyl-6-trifluormethyl-pyrimidin-2-yl)-harnstoff,
N-(2-Cyanophenyl)-N-(4-methyl-6-trifluormethyl-pyrimidin-2-yl)-harnstoff,
N-(2-Bromphenyl)-N-(4-cyclopropyl-6-trifluormethyl-pyrimidin-2-yl)-harnstoff,
N-(4-Isopropyl-6-trifluormethyl-pyrimidin-2-yl)-N-2-(trifluormethylphenyl)-harnstoff,
N-(2-Fluorphenyl)-N-(4-methyl-6-trifluormethyl-pyrimidin-2-yl)-harnstoff,
N-(2-Fluorphenyl)-N-(4-isopropyl-6-trifluormethyl-pyrimidin-2-yl)-harnstoff,
N-(2,5-Dichlorphenyl)-N-(4-ethyl-6-trifluormethyl-pyrimidin-2-yl)-harnstoff,
N-(2,6-Dichlorphenyl)-N-(4-difluormethyl-6-methyl-pyrimidin-2-yl)-harnstoff,
N-(2,6-Dichlorphenyl)-N-(4-methylthio-6-trifluormethyl-pyrimidin-2-yl)-harnstoff,

N-(2,6-Dichlor-3-methyl)-N-(4-methyl-6-trifluor-methyl-pyrimidin-2-yl)-harnstoff,
N-(2,6-Dichlor-3-methylphenyl)-N-(4-isopropyl-6-trifluormethyl-pyrimidin-2-yl)-harnstoff,
N-(2-Chlor-6-trifluormethyl-phenyl)-N-(4-methyl-6-trifluormethyl-pyrimidin-2-yl)-harnstoff,
N-(2-Chlor-6-methyl-phenyl)-N-(4-methyl-6-trifluormethyl-pyrimidin-2-yl)-harnstoff,
N-(2-Methyl-6-trifluormethyl-phenyl)-N-(4-methyl-6-trifluormethyl-pyrimidin-2-yl)-harnstoff,
N-(5-Chlor-2-difluormethoxy-phenyl)-N-(4-methyl-6-trifluormethyl-pyrimidin-2-yl)-harnstoff,
N-(2-Methylthio-phenyl)-N-(4-methyl-6-trifluormethyl-pyrimidin-2-yl)-harnstoff,
N-(4-Cyclopropyl-6-trifluormethyl-pyrimidin-2-yl)-N-(2,5-difluorphenyl)-harnstoff,
N-(2,6-Difluorphenyl)-N-(4-ethyl-6-trifluormethyl-pyrimidin-2-yl)-harnstoff,
N-(6-Chlor-2-fluor-phenyl)-N-(4-methyl-6-trifluormethyl-pyrimidin-2-yl)-harnstoff,
N-(6-Chlor-2-fluor-phenyl)-N-(4-cyclopropyl-6-trifluormethyl-pyrimidin-2-yl)-harnstoff,
N-(2-Difluormethoxyphenyl)-N-(4-methyl-6-trifluormethyl-pyrimidin-2-yl)-harnstoff,
N-(6-Chlor-2-methoxycarbonyl-phenyl)-N-(4-methyl-6-trifluormethyl-pyrimidin-2-yl)-harnstoff,
N-(2-Bromphenyl)-N-[4-(furan-2-yl)-6-trifluormethyl-pyrimidin-2-yl]-harnstoff,
N-(2-Chlor-6-methyl-phenyl)-N-(4-methoxymethyl-6-trifluormethyl-pyrimidin-2-yl)-harnstoff,
oder
N-(2-Difluormethoxy-6-methyl-phenyl)-N-(4-methyl-6-trifluormethyl-pyrimidin-2-yl)-harnstoff.

7. Verfahren zur Herstellung von Anilinen der Formel II

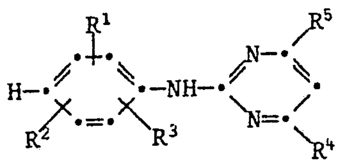

II

worin

$R^1$ Halogen; Cyano; $C_1$-$C_4$-Alkoxy; $C_1$-$C_4$-Halogenalkoxy; $C_1$-$C_4$-Alkyl-$S(O)_n$-; $C_1$-$C_4$-Alkyl; $C_1$-$C_4$-Halogenalkyl; $C_1$-$C_4$-Alkoxycarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-$S(O)_n$-; oder -PO[O-($C_1$-$C_4$)-Alkyl]$_2$;

$R^2$ Wasserstoff; Halogen; Cyano; Nitro; $C_1$-$C_4$-Alkyl; $C_1$-$C_4$-Alkoxy; $C_1$-$C_4$-Halogenalkyl; $C_1$-$C_4$-Alkoxycarbonyl; oder $C_1$-$C_4$-Alkylcarbonyl;

$R^3$ Wasserstoff; Halogen; oder $C_1$-$C_4$-Alkyl;

$R^4$ $C_1$-$C_6$-Alkyl; $C_1$-$C_4$-Alkoxy; $C_1$-$C_4$-Alkyl-$S(O)_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituiertes oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;

$R^5$ Wasserstoff; $C_1$-$C_3$-Alkyl; $C_1$-$C_3$-Halogenalkyl; Halogen; oder $C_1$-$C_3$-Halogenalkoxy; und

n 0, 1 oder 2

bedeutet, mit der Massgabe, dass a) wenn einer der Reste $R^2$ oder $R^3$ Nitro bedeutet, dieser Substituent nicht in 2- oder 6-Stellung des Phenylringes gebunden sein darf und, wenn die Reste $R^4$ und $R^5$ für Methyl stehen $R^1$ nicht für Chlor, Methoxy, Ethoxy, Fluor, Jod, Methyl oder Brom steht und dass ausserdem folgende Einzelverbindungen nicht mit umfasst sind: N-[(4,6-Bis-trifluormethyl)-pyrimidin-2-yl]-2,6-dichloranilin und N-(4-Chlor-6-methylpyrimidin-2yl)-3-chloranilin, gekennzeichnet durch

a) Umsetzung von Guanidinen der Formel V mit 1,3-Dicarbonylverbindungen der Formel VI

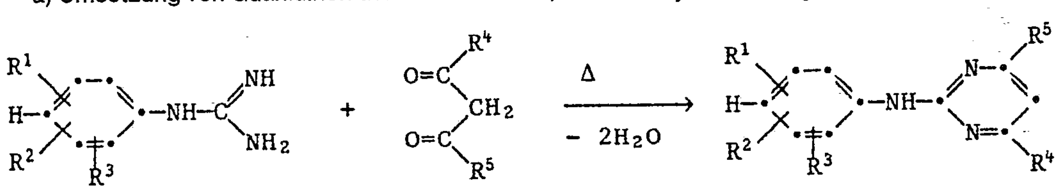


(V) (VI) (II)

wobei die Kondensationsreaktion gewünschtenfalls in Gegenwart wasserbindender Mittel durchgeführt wird oder

b) Umsetzung eines Anilins der Formel VII mit einem Pyrimidin der Formel VIII unter Baseneinwirkung

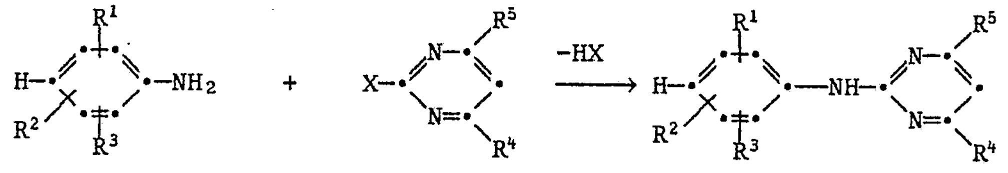


VII VIII II

worin die Reste $R^1$ bis $R^5$ zuvor definiert sind und X für eine nucleofuge Gruppe, wie Halogen, $C_1$-$C_4$-Alkylsulfonyl oder Phenylsulfonyl, steht.

8. Verfahren zur Herstellung von Carbamoylchloriden der Formel III

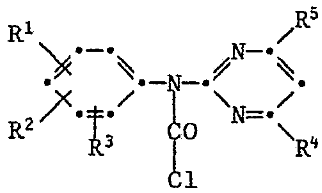
(III),

worin
$R^1$, $R^2$, und $R^3$ unabhängig voneinander Wasserstoff; Halogen; $C_1$-$C_4$-Alkyl; $C_1$-$C_4$-Alkoxy; $C_1$-$C_4$-Halogenalkyl; $C_1$-$C_4$-Halogenalkoxy; $C_1$-$C_4$-Alkyl-$S(O)_n$-; Nitro; Cyano; $C_1$-$C_4$-Alkoxycarbonyl; $C_1$-$C_4$-Alkylcarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-$S(O)_n$-; oder -$PO[O$-$(C_1$-$C_4)$-$Alkyl]_2$;
$R^4$ $C_1$-$C_6$-Alkyl; $C_1$-$C_4$-Alkoxy; $C_1$-$C_4$-Alkyl-$S(O)_n$-; $C_1$-$C_4$-Halogenalkyl; $C_1$-$C_4$-Halogenalkoxy; unsubstituiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituierten oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;
$R^5$ Wasserstoff; $C_1$-$C_3$-Alkyl; $C_1$-$C_3$-Halogenalkyl; Halogen; oder $C_1$-$C_3$-Halogenalkoxy; und
n 0, 1 oder 2
bedeutet, mit der Massgabe, dass, wenn einer der Reste $R^1$, $R^2$ oder $R^3$ Nitro bedeutet, dieser Substituent nicht in 2- oder 6-Stellung des Phenylringes gebunden sein darf, dadurch gekennzeichnet, dass man ein Anilin der Formel II

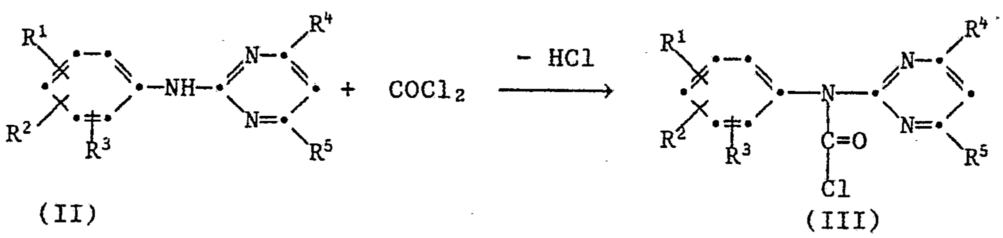


(II) (III)

worin die Reste $R^1$ bis $R^5$ wie unter Formel II definiert sind, mit Phosgen umsetzt.

9. Verfahren zur Herstellung von Halogensulfonylharnstoffen der Formel IV

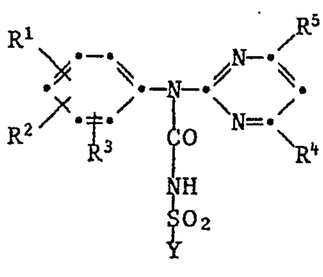
(IV),

worin
Y Halogen; $C_1$-$C_4$-Alkoxy; oder Phenoxy;
$R^1$, $R^2$, und $R^3$ unabhängig voneinander Wasserstoff; Halogen; $C_1$-$C_4$-Alkyl; $C_1$-$C_4$-Alkoxy; $C_1$-$C_4$-Halogenalkyl; $C_1$-$C_4$-Halogenalkoxy; $C_1$-$C_4$-Alkyl-$S(O)_n$-; Nitro; Cyano; $C_1$-$C_4$-Alkoxycarbonyl; $C_1$-$C_4$-Alkylcarbonyl; di-($C_1$-$C_4$-Alkylamino)carbonyl; mono-($C_1$-$C_4$-Alkylamino)carbonyl; Carbamoyl; $C_1$-$C_4$-Halogenalkyl-$S(O)_n$-; oder -$PO[O$-$(C_1$-$C_4)$-$Alkyl]_2$;
$R^4$ $C_1$-$C_6$-Alkyl; $C_1$-$C_4$-Alkoxy; $C_1$-$C_4$-Alkyl-$S(O)_n$-; $C_1$-$C_4$-Halogenalkyl; $C_1$-$C_4$-Halogenalkoxy; unsubsti-

tuiertes, oder bis zu dreifach gleich oder verschieden durch Halogen, $C_1$-$C_4$-Alkyl, $C_1$-$C_4$-Halogenalkyl oder $C_1$-$C_4$-Alkoxy substituiertes Phenyl; 2-Furanyl; 2-Thienyl; 3-Thienyl; unsubstituierten oder bis zu dreifach gleich oder verschieden durch $C_1$-$C_4$-Alkyl substituiertes $C_3$-$C_6$-Cycloalkyl; Cyano; $C_2$-$C_4$-Halogenalkenyl; $C_1$-$C_4$-Alkoxy-$C_1$-$C_4$-alkyl; $C_1$-$C_4$-Alkoxy-$C_1$-$C_4$-alkoxy; oder Halogen-$C_1$-$C_4$-alkylthio;
$R^5$ Wasserstoff; $C_1$-$C_3$-Alkyl; $C_1$-$C_3$-Halogenalkyl; Halogen; oder $C_1$-$C_3$-Halogenalkoxy; und
n 0, 1 oder 2
bedeutet, mit der Massgabe, dass, wenn einer der Reste $R^1$, $R^2$ oder $R^3$ Nitro bedeutet, dieser Substituent nicht in 2- oder 6-Stellung des Phenylringes gebunden sein darf, dadurch gekennzeichnet, dass man ein Anilin der Formel II

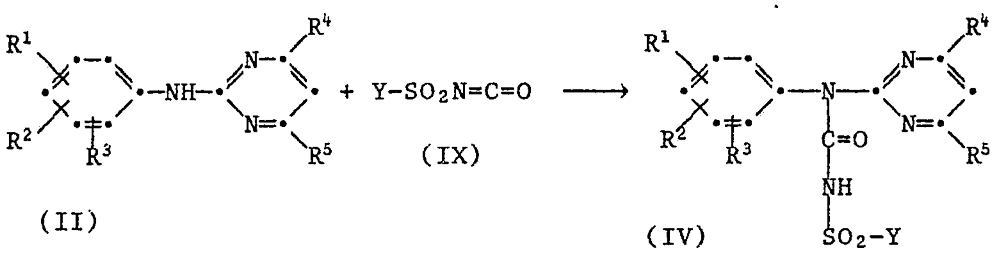

worin die Reste $R^1$ bis $R^5$ wie unter Formel IV definiert sind, mit einem Halogensulfonylisocyanat IX, worin Y Halogen bedeutet, umsetzt.

10. Verfahren zur Bekämpfung unerwünschten Pflanzenwuchses, dadurch gekennzeichnet, dass man eine herbizid wirksame Menge einer Verbindung der Formel I, wie sie in einem der Ansprüche 1 bis 6 definiert ist, auf die zu bekämpfende Pflanze oder deren Lebensraum einwirken lässt.

11. Verfahren zur Beeinflussung des Pflanzenwuchses, dadurch gekennzeichnet, dass man eine pflanzenwuchsregulatorisch wirksame Menge einer Verbindung der Formel I, wie sie in einem der Ansprüche 1 bis 6 definiert ist, auf die Pflanze oder durch Lebensraum einwirken lässt.

12. Verfahren zur Herstellung einer herbiziden oder pflanzenwuchsregulatorisch wirksamen Mittels, dadurch gekennzeichnet, dass man eine Verbindung der Formel I, wie sie in einem der Ansprüche 1 bis 6 definiert ist, mit geeigneten agrochemischen Hilfs- und/oder Trägerstoffen mischt.